# EUROPEAN PATENT SPECIFICATION

(11) **EP 2 535 403 B1**
(45) Date of publication and mention of the grant of the patent: **19.06.2019**
(21) Application number: 12166007.0
(22) Date of filing: 02.11.2006
(51) Int. Cl.: C12N 5/074

(54) **Very small embryonic-like (VSEL) stem cells and methods of isolating and using the same**
VSEL (Very Small Embryonic-Like)-Stammzellen und Verfahren zur Isolierung und Verwendung davon
Très petites cellules souches de type embryoniques (VSEL) et procédés d'isolation et d'utilisation correspondant

(30) Priority: 08.12.2005 US 748685 P
(43) Date of publication of application: 19.12.2012
(62) Divisional of application: 06827358.0
(73) Proprietor: UNIVERSITY OF LOUISVILLE RESEARCH FOUNDATION, INC., Louisville, KY 40208 (US)
(72) Inventor: Ratajczak, Mariusz, Louiseville, KY 40245 (US); Kucia, Magdalena, Louisville, KY 40202 (US); Ratajczak, Janina, Louiseville, KY 40245 (US)
(74) Representative: Pohlman, Sandra M.

(56) References cited:
- WO-A2-01/11011
- WO-A2-2004/043990
- WO-A2-2005/042723
- US-A1- 2005 181 502
- KUCIA M ET AL: "Cells enriched in markers of neural tissue-committed stem cells reside in the bone marrow and are mobilized into the peripheral blood following stroke", LEUKEMIA, MACMILLAN PRESS LTD, US, vol. 20, no. 1, 3 November 2005 (2005-11-03), pages 18-28, XP002604061, ISSN: 0887-6924, DOI: 10.1038/SJ.LEU.2404011 [retrieved on 2005-11-03]
- KUCIA M ET AL: "Bone marrow as a home of heterogenous populations of nonhematopoietic stem cells", LEUKEMIA (BASINGSTOKE), vol. 19, no. 7, July 2005 (2005-07), pages 1118-1127, XP002559661, ISSN: 0887-6924
- KUCIA MAGDA ET AL: "Cells expressing early cardiac markers reside in the bone marrow and are mobilized into the peripheral blood after myocardial infarction", CIRCULATION RESEARCH, GRUNE AND STRATTON, BALTIMORE, US, vol. 95, no. 12, 10 December 2004 (2004-12-10), pages 1191-1199, XP002592556, ISSN: 0009-7330, DOI: 10.1161/01.RES.0000150856.47324.5B [retrieved on 2004-11-18]
- KUCIA M ET AL: "Are bone marrow stem cells plastic or heterogenous-That is the question", EXPERIMENTAL HEMATOLOGY, NEW YORK, NY, US, vol. 33, no. 6, 1 June 2005 (2005-06-01), pages 613-623, XP004899127, ISSN: 0301-472X
- KUCIA M ET AL: "Bone marrow as a source of circulating CXCR4(+) tissue-committed stem cells", BIOLOGY OF THE CELL, ELSEVIER, PARIS, FR, vol. 97, no. 2, 1 February 2005 (2005-02-01), pages 133-146, XP002409123, ISSN: 0248-4900
- PROCKOP D J: "Marrow stromal cells as stem cells for nonhematopoietic tissues", SCIENCE 19970404 US, vol. 276, no. 5309, 4 April 1997 (1997-04-04), pages 71-74, XP002559662, ISSN: 0036-8075
- D'IPPOLITO GIANLUCA ET AL: "Marrow-isolated adult multilineage inducible (MIAMI) cells, a unique population of postnatal young and old human cells with extensive expansion and differentiation potential", JOURNAL OF CELL SCIENCE, vol. 117, no. 14, 15 June 2004 (2004-06-15), pages 2971-2981, XP002559663, ISSN: 0021-9533
- JIANG Y ET AL: "Pluripotency of mesenchymal stem cells derived from adult marrow", NATURE 20020704 GB, vol. 418, no. 6893, 4 July 2002 (2002-07-04), pages 41-49, XP002559664, ISSN: 0028-0836
- KOGLER GESINE ET AL: "A new human somatic stem cell from placental cord blood with intrinsic pluripotent differentiation potential", JOURNAL OF EXPERIMENTAL MEDICINE, vol. 200, no. 2, 19 July 2004 (2004-07-19) , pages 123-135, XP002559665, ISSN: 0022-1007
- KUCIA M ET AL: "Identification of very small embryonic like (VSEL) stem cells in bone marrow", CELL AND TISSUE RESEARCH, SPRINGER, BERLIN, DE, vol. 331, no. 1, 9 September 2007 (2007-09-09), pages 125-134, XP019564036, ISSN: 1432-0878
- DAVID MASON ET AL: "CD antigens 2001", EUROPEAN JOURNAL OF IMMUNOLOGY, vol. 31, no. 10, 1 October 2001 (2001-10-01), pages 2841-2847, XP055125173, ISSN: 0014-2980, DOI: 10.1002/1521-4141(2001010)31:10<2841::AID- IMMU2841>3.0.CO;2-Y
- DAWN BUDDHADEB ET AL: "Transplantation of bone marrow-derived very small embryonic-like stem cells attenuates left ventricular dysfunction and remodeling after myocardial infarction", STEM CELLS, ALPHAMED PRESS, DAYTON, OH, US, vol. 26, no. 6, 1 June 2008 (2008-06-01), pages 1646-1655, XP002604064, ISSN: 1066-5099, DOI: 10.1634/STEMCELLS.2007-0715 [retrieved on 2008-04-17]
- Tatsuya Mimura ET AL: "Treatment of Rabbit Bullous Keratopathy with Precursors Derived from Cultured Human Corneal Endothelium", Investigative Opthalmology & Visual Science, vol. 46, no. 10, 1 October 2005 (2005-10-01), pages 3637-3644, XP055241734, US ISSN: 1552-5783, DOI: 10.1167/iovs.05-0462
- D M SHIN ET AL: "Novel epigenetic mechanisms that control pluripotency and quiescence of adult bone marrow-derived Oct4+ very small embryonic-like stem cells", LEUKEMIA, vol. 23, no. 11, 30 July 2009 (2009-07-30) , pages 2042-2051, XP055027724, ISSN: 0887-6924, DOI: 10.1038/leu.2009.153

## Description

### CROSS REFERENCE TO RELATED APPLICATIONS

This application claims the benefit of U.S. Provisional Patent Application Serial No. 60/748,685, filed December 8, 2005.

### TECHNICAL FIELD

The presently disclosed subject matter relates, in general, to the identification, isolation, and use of a population of stem cells isolated from bone marrow, umbilical cord blood, and/or other sources and that are referred to herein as very small embryonic-like (VSEL) stem cells. More particularly, the presently disclosed subject matter relates to isolating said VSEL stem cells and employing the same, optionally after *in vitro* manipulation, to treat tissue and/or organ damage in a subject in need thereof.

### BACKGROUND

The use of stem cells and stem cell derivatives has gained increased interest in medical research, particularly in the area of providing reagents for treating tissue damage either as a result of genetic defects, injuries, and/or disease processes. Ideally, cells that are capable of differentiating into the affected cell types could be transplanted into a subject in need thereof, where they would interact with the organ microenvironment and supply the necessary cell types to repair the injury.

Considerable effort has been expended to isolate stem cells from a number of different tissues for use in regenerative medicine. For example, U.S. Patent No. 5,750,397 to Tsukamoto et al. discloses the isolation and growth of human hematopoietic stem cells that are reported to be capable of differentiating into lymphoid, erythroid, and myelomonocytic lineages. U.S. Patent No. 5,736,396 to Bruder et al. discloses methods for lineage-directed differentiation of isolated human mesenchymal stem cells under the influence of appropriate growth and/or differentiation factors. The derived cells can then be introduced into a host for mesenchymal tissue regeneration or repair.

One area of intense interest relates to the use of embryonic stem (ES) cells, which have been shown in mice to have the potential to differentiate into all the different cell types of the animal. Mouse ES cells are derived from cells of the inner cell mass of early mouse embryos at the blastocyst stage, and other pluripotent and/or totipotent cells have been isolated from germinal tissue (e.g., primordial germ cells; PGCs). The ability of these pluripotent and/or totipotent stem cells to proliferate *in vitro* in an undifferentiated state, retain a normal karyotype, and retain the potential to differentiate to derivatives of all three embryonic germ layers (endoderm, mesoderm, and ectoderm) makes these cells attractive as potential sources of cells for use in regenerative therapies in post-natal subjects.

The development of human ES (hES) cells has not been as successful as the advances that have been made with mouse ES cells. Thomson *et al.* reported pluripotent stem cells from lower primates (U.S. Patent No. 5,843,780; Thomson et al. (1995) 92 Proc Natl Acad Sci U S A 7844-7848), and from humans (Thomson et al. (1998) 282 Science 1145-1147). Gearhart *et al.* generated human embryonic germ (hEG) cell lines from fetal gonadal tissue (Shamblott et al. (1998) 95 Proc Natl Acad Sci U S A 13726-13731; and U.S. Patent No. 6,090,622). Both hES and hEG cells have the desirable characteristics of pluripotent stem cells in that they are capable of being propagated *in vitro* without differentiating, they generally maintain a normal karyotype, and they remain capable of differentiating into a number of different cell types. Clonally derived human embryonic stem cell lines maintain pluripotency and proliferative potential for prolonged periods in culture (Amit et al. (2000) 227 Dev Biol 271-278).

One significant challenge to the use of ES cells or other pluripotent cells for regenerative therapy in a subject is to control the growth and differentiation of the cells into the particular cell type required for treatment of a subject. There have been several reports of the effect of growth factors on the differentiation of ES cells. For example, Schuldiner *et al.* report the effects of eight growth factors on the differentiation of cells into different cell types from hES cells (see Schuldiner et al. (2000) 97 Proc Natl Acad Sci U S A 11307-11312). As disclosed therein, after initiating differentiation through embryoid body-like formation, the cells were cultured in the presence of bFGF, TGF*β*1, activin-A, BMP-4, HGF, EGF, *β*NGF, or retinoic acid. Each growth factor had a unique effect on the differentiation pathway, but none of the growth factors directed differentiation exclusively to one cell type.

Ideally, it would be beneficial to be able to isolate and purify stem and/or precursor cells from a subject that could be purified and/or manipulated *in vitro* before being reintroduced into the subject for treatment purposes. The use of a subject's own cells would obviate the need to employ adjunct immunosuppressive therapy, thereby maintaining the competency of the subject's immune system. However, the current strategies for isolating ES cell lines, particularly hES cell lines, preclude isolating the cells from a subject and reintroducing them into the same subject.

Thus, the search for other stem cell types from adult animals continues. For example, mesenchymal stem cells (MSCs) are one such cell type. MSCs have been shown to have the potential to differentiate into several lineages including bone (Haynesworth et al. (1992) 13 Bone 81-88), cartilage (Mackay et al. (1998) 4 Tissue Eng 415-28; Yoo et al. (1998) 80 J Bone Joint Surg Am 1745-57), adipose tissue (Pittenger et al. (2000) 251 Curr Top Microbiol Immunol 3-11), tendon (Young et al. (1998) 16 J Orthop Res 406-13), muscle, and stroma (Caplan et al. (2001) 7 Trends Mol Med 259-64).

Another population of cells, multipotent adult progenitor cells (MAPCs), has also been purified from bone marrow (BM; Reyes et al. (2001) 98 Blood 2615-2625; Reyes & Verfaillie (2001) 938 Ann NY Acad Sci 231-235). These cells have been shown to be capable of expansion *in vitro* for more than 100 population doublings without telomere shortening or the development of karyotypic abnormalities. MAPCs have also been shown to be able to differentiate under defined culture conditions into various mesenchymal cell types (e.g., osteoblasts, chondroblasts, adipocytes, and skeletal myoblasts), endothelium, neuroectoderm cells, and more recently, into hepatocytes (Schwartz et al. (2000) 109 J Clin Invest 1291-1302).

Additionally, hematopoietic stem cells (HSCs) have been reported to be able to differentiate into numerous cell types. BM hematopoietic stem cells have been reported to be able to 'transdifferentiate' into cells that express early heart (Orlic et al. (2003) 7 Pediatr Transplant 86-88; Makino et al. (1999) 103 J Clin Invest 697-705), skeletal muscle (Labarge & Blau (2002) 111 Cell 589-601; Corti et al. (2002) 277 Exp Cell Res 74-85), neural (Sanchez-Ramos (2002) 69 Neurosci Res 880-893), liver (Petersen et al. (1999) 284 Science 1168-1170), or pancreatic cell (Ianus et al. (2003) 111 J Clin Invest 843-850; Lee & Stoffel (2003) 111 J Clin Invest 799-801) markers. *In vivo* experiments in humans also demonstrated that transplantation of CD34⁺ peripheral blood (PB) stem cells led to the appearance of donor-derived hepatocytes (Korbling et al. (2002) 346 N Engl J Med 738-746), epithelial cells (Korbling et al. (2002) 346 N Engl J Med 738-746), and neurons (Hao et al. (2003) 12 J Hematother Stem Cell Res 23-32). Additionally, human BM-derived cells have been shown to contribute to the regeneration of infarcted myocardium (Stamm et al. (2003) 361 Lancet 45-46).

These reports have been interpreted as evidence for the existence of the phenomenon of transdifferentiation or plasticity of adult stem cells. However, the concept of transdifferentiation of adult tissue-specific stem cells is currently a topic of extensive disagreement within the scientific and medical communities (*see e.g*., Lemischka (2002) 30 Exp Hematol 848-852; Holden & Vogel (2002) 296 Science 2126-2129). Studies attempting to reproduce results suggesting transdifferentiation with neural stem cells have been unsuccessful (Castro et al. (2002) 297 Science 1299). It has also been shown that the hematopoietic stem/progenitor cells (HSPC) found in muscle tissue originate in the BM (McKinney-Freeman et al. (2002) 99 Proc Natl Acad Sci U S A 1341-1346; Geiger et al. 100 Blood 721-723; Kawada & Ogawa (2001) 98 Blood 2008-2013). Additionally, studies with chimeric animals involving the transplantation of single HPCs into lethally irradiated mice demonstrated that transdifferentiation and/or plasticity of circulating HPSC and/or their progeny, if it occurs at all, is an extremely rare event (Wagers et al. (2002) 297 Science 2256-2259).

Thus, there continues to be a need for new approaches to generate populations of transplantable cells suitable for a variety of applications, including but not limited to treating injury and/or disease of various organs and/or tissues.

### SUMMARY

This Summary lists several embodiments of the presently disclosed subject matter, and in many cases lists variations and permutations of these embodiments. This Summary is merely exemplary of the numerous and varied embodiments. Mention of one or more representative features of a given embodiment is likewise exemplary. Such an embodiment can typically exist with or without the feature(s) mentioned; likewise, those features can be applied to other embodiments of the presently disclosed subject matter, whether listed in this Summary or not. To avoid excessive repetition, this Summary does not list or suggest all possible combinations of such features. The present invention provides a composition comprising isolated CD45⁻ stem cells comprising VSEL stem cells for use in treating a myocardial infarction in a subject, wherein the VSEL stem cells comprise Sca-1⁺/lin⁻/CD45⁻ and express at least one of SSEA-1, Oct-4, Rex-1, and Nanog, or comprise CD34⁺/lin⁻/CD45⁻ and express at least one of SSEA-4, Oct-4, Rex-1, and Nanog. Further, the presently disclosed subject matter provides methods for forming an embryoid body-like sphere from a population of very small embryonic-like (VSEL) stem cells or derivatives thereof. In some embodiments, the methods can comprise (a) providing a population of CD45⁻ cells comprising VSEL stem cells or derivatives thereof; and (b) culturing the VSEL stem cells or derivatives thereof in a medium comprising one or more factors that induce embryoid body-like sphere formation of the VSEL stem cells or derivatives thereof for a time sufficient for an embryoid body-like sphere to form. In some embodiments, the VSEL stem cells or derivatives thereof comprise CD34⁺/lin⁻/CD45⁻ or Sca-1⁺/lin⁻/CD45⁻ very small embryonic-like (VSEL) stem cells. In some embodiments, the VSEL stem cells are about 3-4 µm in diameter, express at least one of SSEA-1, Oct-4, Rev-1, and Nanog, posses large nuclei surrounded by a narrow rim of cytoplasm, and have open-type chromatin (euchromatin). In some embodiments, the population of CD45⁻ cells comprising VSEL stem cells or derivatives thereof is isolated from a human or from a mouse. In some embodiments, the population of CD45⁻ cells comprising VSEL stem cells or derivatives thereof is isolated from a source in the human or the mouse selected from the group consisting of bone marrow, peripheral blood, spleen, cord blood, and combinations thereof. In some embodiments, the one or more growth factors that induce embryoid body-like sphere formation of the VSEL stem cells or derivatives thereof comprise epidermal growth factor (EGF), fibroblast growth factor-2, and combinations thereof. In some embodiments, the one or more factors are provided to the VSEL stem cells or derivatives thereof by co-culturing the VSEL stem cells or derivatives thereof with C2C12 cells.

In some embodiments, the population of CD45⁻ cells comprising VSEL stem cells can be isolated by a method comprising the steps of (a) providing an initial population of cells suspected of comprising CD45⁻ stem cells; (b) contacting the initial population of cells with a first antibody that is specific for CD45 and a second antibody that is specific for CD34 or Sca-1 under conditions sufficient to allow binding of each antibody to its target, if present, on each cell of the initial population of cells; (c) selecting a first subpopulation of cells that are CD34⁺ or Sca-1⁺, and are also CD45⁻; (d) contacting the first subpopulation of cells with one or more antibodies that are specific for one or more cell surface markers selected from the group consisting of CD45R/B220, Gr-1, TCRα*β*, TCRγδ, CD11b, and Ter-119 under conditions sufficient to allow binding of each antibody to its target, if present, on each cell of the population of cells; (e) removing from the first subpopulation of cells those cells that bind to at least one of the antibodies of step (d); and (f) collecting a second subpopulation of cells that are either CD34⁺/lin⁻/CD45⁻ or Sca-1⁺/lin⁻/CD45⁻, whereby a subpopulation of CD45⁻ stem cells is isolated. In some embodiments, each antibody comprises a detectable label. In some embodiments, the detectable label comprises a fluorescent label or a moiety that can be detected by a reagent comprising a fluorescent label. In some embodiments, the separating comprises FACS sorting. In some embodiments, the presently disclosed methods further comprise isolating those cells that are c-met⁺, c-kit⁺, and/or LIF-R⁺. In some embodiments, the presently disclosed methods further comprise isolating those cells that express one or more genes selected from the group consisting of SSEA-1, Oct-4, Rev-1, and Nanog. In some embodiments, the population of cells comprises a bone marrow sample, a cord blood sample, or a peripheral blood sample.

In some embodiments of the presently disclosed subject matter, the population of cells is isolated from peripheral blood of a subject subsequent to treating the subject with an amount of a mobilizing agent sufficient to mobilize the CD45⁻ stem cells comprising VSEL stem cells from bone marrow into the peripheral blood of the subject. In some embodiments, the mobilizing agent comprises at least one of granulocyte-colony stimulating factor (G-CSF) and a CXCR4 antagonist. In some embodiments, the CXCR4 antagonist is a T140 peptide. In some embodiments, the subject is a mouse.

In some embodiments, the presently disclosed methods further comprise contacting the subpopulation of stem cells with an antibody that binds to CXCR4 and isolating from the subpopulation of stem cells those cells that are CXCR4⁺.

In some embodiments, the presently disclosed methods further comprise isolating those cells that are CXCR4⁺ and/or AC133⁺.

In some embodiments, the presently disclosed methods further comprise selecting those cells that are HLA-DR⁻, MHC class I⁻, CD90⁻, CD29⁻, CD105⁻, or combinations thereof.

The presently disclosed subject matter also provides embryoid body-like spheres comprising a plurality of very small embryonic-like (VSEL) stem cells.

The presently disclosed subject matter also provides cell cultures comprising embryoid body-like spheres as disclosed herein. In some embodiments, the embryoid body-like spheres disclosed herein are provided in a medium comprising one or more factors that induce embryoid body-like sphere formation of the VSEL stem cells or derivatives thereof.

The presently disclosed subject matter also provides methods for differentiating a very small embryonic-like (VSEL) stem cell into a cell type of interest. In some embodiments, the method comprise (a) providing an embryoid body-like sphere comprising VSEL stem cells or derivatives thereof; and (b) culturing the embryoid body-like sphere in a culture medium comprising a differentiation-inducing amount of one or more factors that induce differentiation of the VSEL stem cells or derivatives thereof into the cell type of interest until the cell type of interest appears in the culture.

In some embodiments, the cell type of interest is a cardiomyocyte. In some embodiments, the culturing is for at least about 15 days. In some embodiments, the culture medium comprises a combination of basic fibroblast growth factor, vascular endothelial growth factor, and transforming growth factor *β*1 in an amount sufficient to cause a subset of the embryoid body-like sphere cells to differentiate into cardiomyocytes. In some embodiments, the cardiomyocyte expresses a marker selected from the group consisting of Nsx2.5/Csx and GATA-4.

In some embodiments of the presently disclosed methods, the embryoid body-like sphere is prepared by (a) providing a population of CD45⁻ cells comprising VSEL stem cells; and (b) culturing the VSEL stem cells in a culture medium comprising one or more factors that induce embryoid body-like sphere formation of the VSEL cells for a time sufficient for an embryoid body-like sphere to appear.

The presently disclosed subject matter also provides formulations comprising the differentiated very small embryonic-like (VSEL) stem cells disclosed herein in a pharmaceutically acceptable carrier or excipient. In some embodiments, the pharmaceutically acceptable carrier or excipient is acceptable for use in humans.

The presently disclosed subject matter also provides methods for treating an injury to a tissue in a subject wherein the injury is a myocardial infraction. In some embodiments, the methods comprise administering to the subject a composition comprising a plurality of isolated CD45⁻ stem cells comprising VSEL stem cells in a pharmaceutically acceptable carrier, in an amount and via a route sufficient to allow at least a fraction of the population of CD45⁻ stem cells to engraft the tissue and differentiate therein, whereby the injury is treated. In some embodiments, the subject is a mammal. In some embodiments, the mammal is selected from the group consisting of a human and a mouse. In some embodiments, the isolated CD45⁻ stem cells comprising VSEL stem cells were isolated from a source selected from the group consisting of bone marrow, peripheral blood, spleen, cord blood, and combinations thereof.

In some embodiments, the presently disclosed methods further comprise differentiating the isolated CD45⁻ stem cells to produce a pre-determined cell type prior to administering the composition to the subject. In some embodiments, the pre-determined cell type is a cardiomyocyte.

The presently disclosed subject matter also provides methods for purifying a very small embryonic-like (VSEL) stem cell for a cell type of interest from a population of CD45⁻ stem cells. In some embodiments, the methods comprise (a) providing a population of CD45⁻ stem cells comprising VSEL stem cells; (b) identifying a subpopulation of the CD45⁻ stem cells that express a marker of VSEL stem cells; and (c) purifying the subpopulation. In some embodiments, the population and the subpopulation are both CD34⁺/CXCR4⁺/lin⁻ or Sca-1⁺/lin⁻ in addition to being CD45⁻. In some embodiments, the population of CD45⁻ stem cells comprising VSEL stem cells was isolated from a source selected from the group consisting of bone marrow, peripheral blood, spleen, cord blood, and combinations thereof. In some embodiments, the cell type of interest is selected from the group consisting of a skeletal muscle cell, an intestinal epithelium cell, a pancreas cell, an endothelial cell, an epidermis cell, a melanocyte, a neuronal cell, a myocardial cell, a chondrocyte, an adipocyte, a liver cell, a pancreas cell, an endothelial cell, an epithelial cell, a retinal pigment cell, and an endodermal cell.

In some embodiments, the cell type of interest is a myocardial cell and the marker is selected from the group consisting of Nkx2.5/Csx, GATA-4, and MEF2C. In some embodiments, the cell type of interest is an endothelial cell and the marker is selected from the group consisting of VEGFR2, VE-cadherin, von Willebrand factor, and TIE2. In some embodiments, the cell type of interest is a skeletal muscle cell and the marker is selected from the group consisting of Myf5, MyoD, and myogenin. In some embodiments, the cell type of interest is a liver cell and the marker is selected from the group consisting of *α*-fetoprotein and CK19. In some embodiments, the cell type of interest is a neural cell and the marker is selected from the group consisting of *β* III tubulin, Olig1, Olig2, GFAP, and nestin. In some embodiments, the cell type of interest is a pancreas cell and the marker is selected from the group consisting of Nkx 6.1 and Pdx 1. In some embodiments, the cell type of interest is a melanocyte and the marker is selected from the group consisting of DCT, TYR, and TRP.

The presently disclosed subject matter also provides methods for isolating a subpopulation of CD45⁻ stem cells comprising VSEL stem cells from umbilical cord blood or a fraction thereof. In some embodiments, the methods comprise (a) contacting the umbilical cord blood or the fraction thereof with a first antibody that is specific for CD45 and a second antibody that is specific for CD34 or Sca-1 under conditions sufficient to allow binding of each antibody to its target, if present, on each cell of the population of cells; (b) selecting a first subpopulation of cells that are CD34⁺ or Sca-1⁺, and are also CD45⁻; (c) contacting the first subpopulation of cells with one or more antibodies that are specific for one or more cell surface markers selected from the group consisting of CD45R/B220, Gr-1, TCRα*β*, TCRγδ, CD11b, and Ter-119 under conditions sufficient to allow binding of each antibody to its target, if present, on each cell of the population- of cells; (d) removing from the first subpopulation of cells those cells that bind to at least one of the antibodies of step (d); and (e) collecting a second subpopulation of cells that are either CD34⁺/lin⁻/CD45⁻ or Sca-1⁺/lin⁻/CD45⁻, whereby a subpopulation of CD45⁻ stem cells comprising VSEL stem cells is isolated. In some embodiments, the presently disclosed methods further comprise incubating the umbilical cord blood or the fraction thereof or any of the subpopulations in a hypotonic solution for a time sufficient to lyse essentially all erythocytes that might be present. In some embodiments, the presently disclosed methods further comprise isolating those cells that are positive for at least one of CXCR4, c-met, c-kit, or LIF-R.

Accordingly, it is an object of the presently disclosed subject matter to provide new populations of stem cells, and methods of preparing and using the same. This object and other objects are achieved in whole or in part by the presently disclosed subject matter.

An object of the presently disclosed subject matter having been stated above, other objects will become evident as the description proceeds, when taken in connection with the Examples and Figures as described hereinbelow.

### BRIEF DESCRIPTION OF THE DRAWINGS

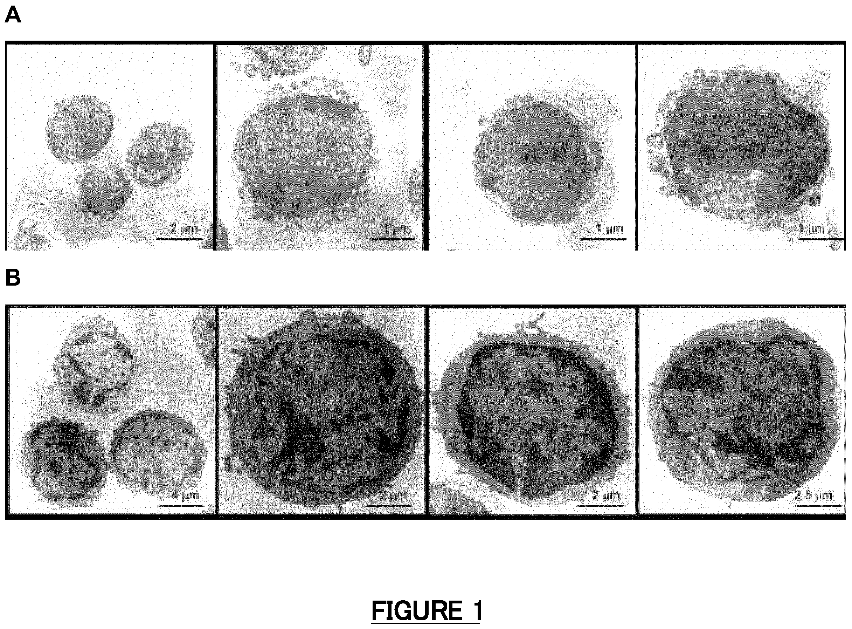

Figures 1A and 1B depict transmission electron microscopy (TEM) images of Sca-1⁺/lin⁻/CD45⁻ and Sca-1⁺/lin⁻/CD45⁺ cells.
Figure 1A shows that Sca-1⁺/lin⁻/CD45⁻ cells are small and measure 3-4 µm in diameter. They possess a relatively large nucleus surrounded by a narrow rim of cytoplasm. At the ultrastructural level, the narrow rim of cytoplasm possesses a few mitochondria, scattered ribosomes, small profiles of endoplasmic reticulum, and a few vesicles. The nucleus is contained within a nuclear envelope with nuclear pores. Chromatin is loosely packed and consists of euchromatin. Figure 1B shows that in contrast, Sca-1⁺/lin⁻/CD45⁺ cells display heterogeneous morphology and are larger. They measure on average 8-10 µm in diameter and possess scattered chromatin and prominent nucleoli.

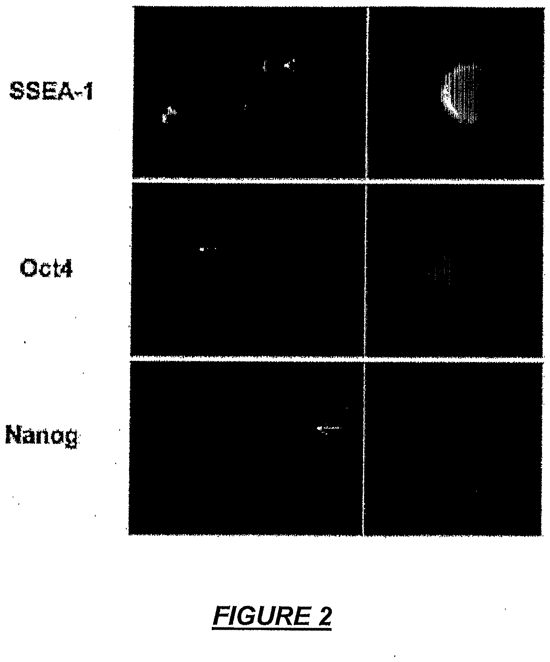

Figure 2 depict fluorescence micrographic images depicting the results of expression studies on Sca-1⁺/lin⁻/CD45⁻ cells and showing that Sca-1⁺/lin⁻ /CD45⁻ cells are SSEA-1⁺ and express Oct-4 and Nanog. As shown on the left panels, Sca-1⁺/lin⁻/CD45⁻ cells isolated by FACS were evaluated for expression of SSEA-1, Oct-4, and Nanog. All images were taken under Plan Apo 60XA/1.40 oil objective (Nikon, Japan). The right panels show 10x enlarged images of representative cells (arrows) performed in ADOBE® PHOTOSHOP® CS software (Adobe System Incorporated, San Jose, California, United States of America). Negative staining controls are not shown. Staining was performed on cells isolated from four independent sorts. Representative data are shown.

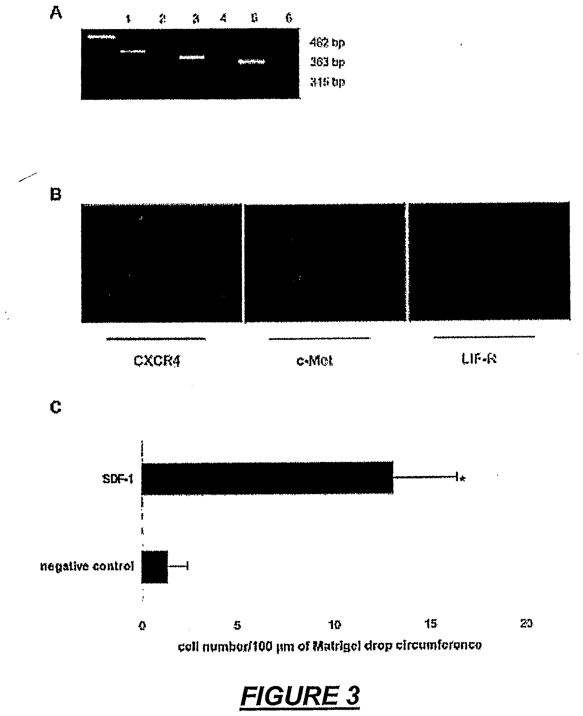

Figures 3A-3C depict the results of expression studies of Sca-1⁺/lin⁻/CD45⁻ for CXCR4, c-met, and LIF-R.
Figure 3A depicts a photograph of a gel on which RT-PCR products have been separated and stained with ethidium bromide, and depict the results of expression of mRNA for CXCR4 (lane 1), c-Met (lane 3) and LIF-R (lane 5) in Sca-1⁺/lin⁻/CD45⁻ is depicted. RT-PCR was run for 30 cycles. Negative RT-PCR reactions (DNA instead of cDNA: lanes 2,4 and 6). Representative result from three independent sorts is shown. Figure 3B depicts fluorescence micrographic images of Sca-1⁺/lin⁻/CD45⁻ cells isolated by FACS and evaluated for expression of CXCR4, c-MET and LIF-R by immunohistochemical staining (the images were taken under Plan Apo 60XA/1.40 oil objective (Nikon, Japan)). Negative staining controls are not shown. Representative result from four independent experiments is shown. Figure 3C is a bar graph depicting the results of chemoattraction studies of Sca-1⁺/lin⁻/CD45⁻ cells by MATRlGEL® drop containing SDF-1 or not (negative control). The number of chemoattracted Sca-1⁺/lin⁻/CD45⁻ cells is shown per 100 µm of MATRIGEL® drop circumference. The data are pooled together from three independent experiments. *p < 0.00001 as compared to control MATRIGEL®.

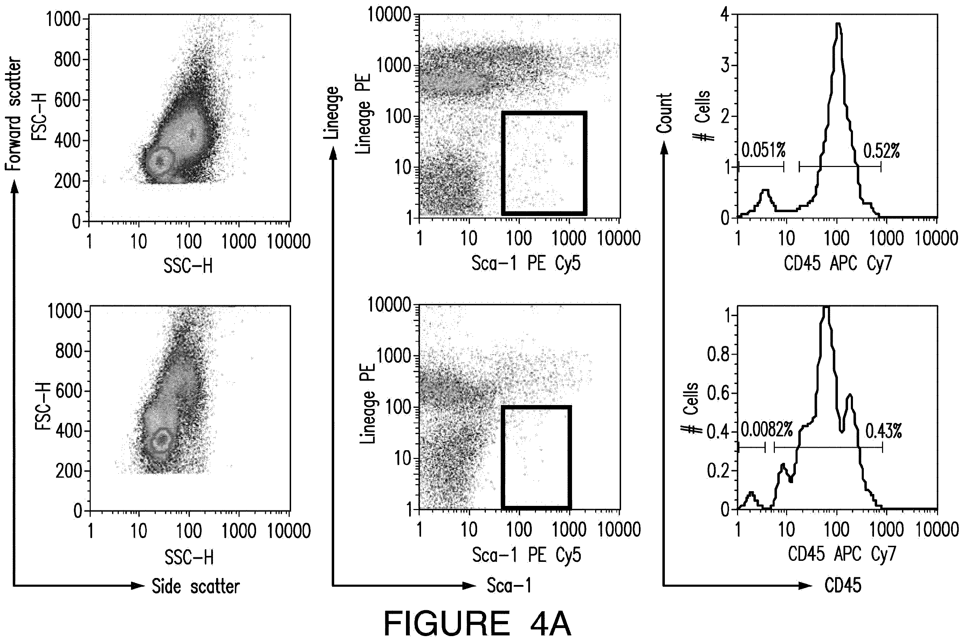

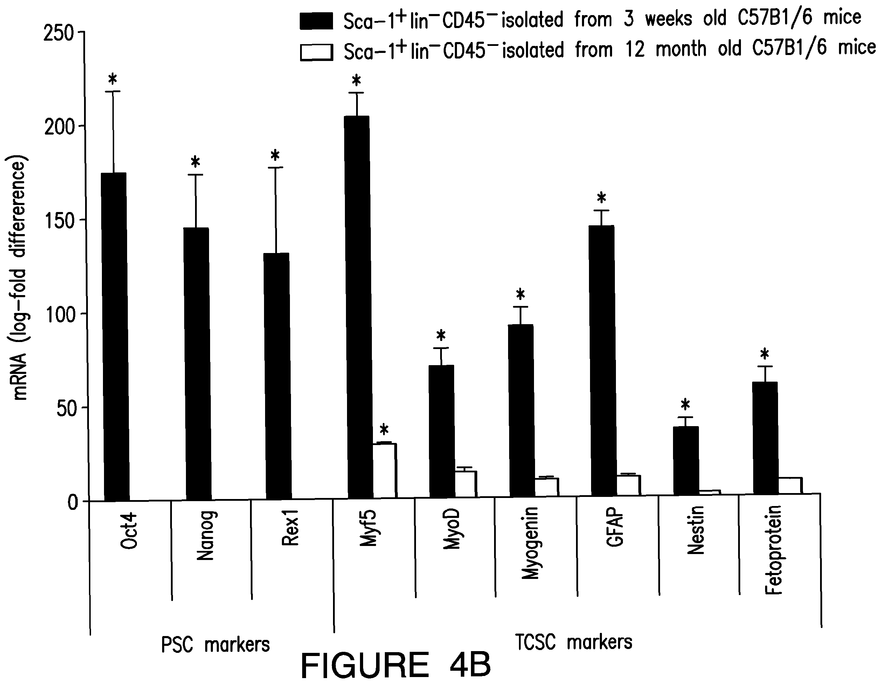

Figures 4A and 4B depict the results of FACS sorting Sca-1⁺/lin⁻/CD45⁻ cells isolated from animals of different ages.
Figure 4A are FACS dot-plots of cells sorted from BMMNC derived from 3 week old (upper panel) and 1 year old mice (lower panel). The left panels depict dot-plots of murine BMMNCs. Cells from the lymphoid gate that were Sca-1⁺/lin⁻ (middle panels) were sorted by FACS for CD45 expression (right panels). Three independent sorting experiments were performed (the BM of 8 mice was pooled for each sort). Representative sorts are shown. Figure 4B is a bar graph depicting expression of mRNA for PSC and VSEL stem cell markers in Sca-1⁺/lin⁻/CD45⁻ cells isolated by FACS from 3 week old and 1 year old mice was compared by RQ-PCR between the same number of sorted cells. Four independent sorting experiments were performed (the BM of 8 mice was pooled for each sort). Data are mean ± SD. *p < 0.01 vs. cells from old animals.

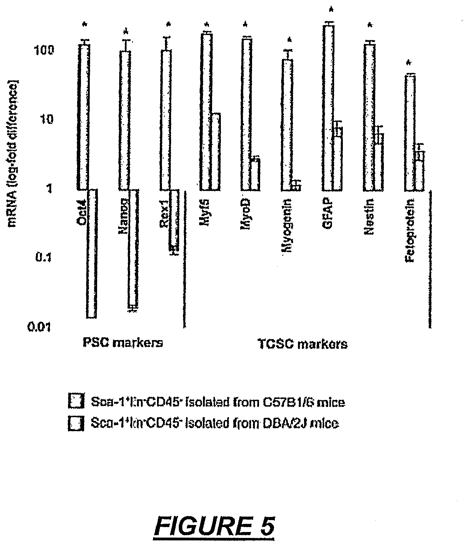

Figure 5 is a bar graph depicting the results of comparing cell numbers from a mouse strain with a relatively long lifespan (C57BL/6) to that of a mouse strain with a relatively short lifespan (DBA/2J). The Figure shows the reduced number of Sca-1⁺/lin⁻/CD45⁻ cells in the DBA/2J mice as compared to the C57BI/6 mice. The expression of mRNA for PSC and VSEL stem cell markers in Sca-1⁺/lin⁻/CD45⁻ cells isolated by FACS from three week old DBA/2J and C57BI6 mice was compared by RQ-PCR between the same number of sorted cells. Three independent sorting experiments were performed (the BM of 6 mice was pooled for each sort). Data are mean ± SD. *p < 0.01 vs. cells from old DBA/2J mice.

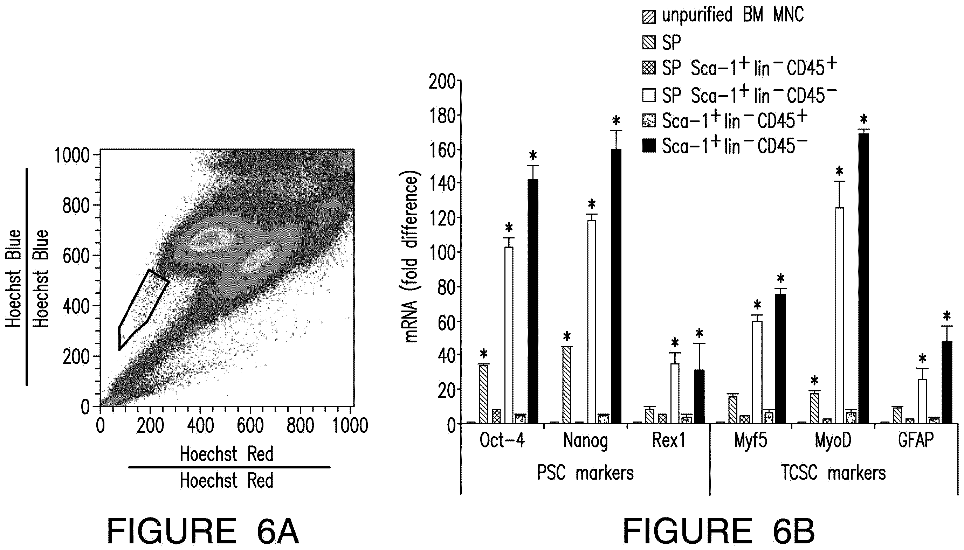

Figures 6A and 6B depict the sorting of a side population (SP) of bone marrow mononuclear cells (BMMNC).
Figure 6A is a dot-plot depicting FACS sorting of the SP of BMMNC. Figure 6B is a bar graph depicting the expression of mRNA for PSC and VSEL stem cell markers in BMMNC, SP, SP Sca-1⁺/lin⁻/CD45⁻, SP Sca-1⁺/lin⁻/CD45⁺, Sca-1⁺/lin⁻/CD45⁻ and Sca-1⁺/lin⁻/CD45⁺ cells isolated by FACS from 3 week old mice was compared by RQ-PCR between the same number of sorted cells. Three independent sorting experiments were performed (the BM of 8 mice was pooled for each sort). Data are presented as mean ± SD. *p < 0.01 vs. cells from old animals.

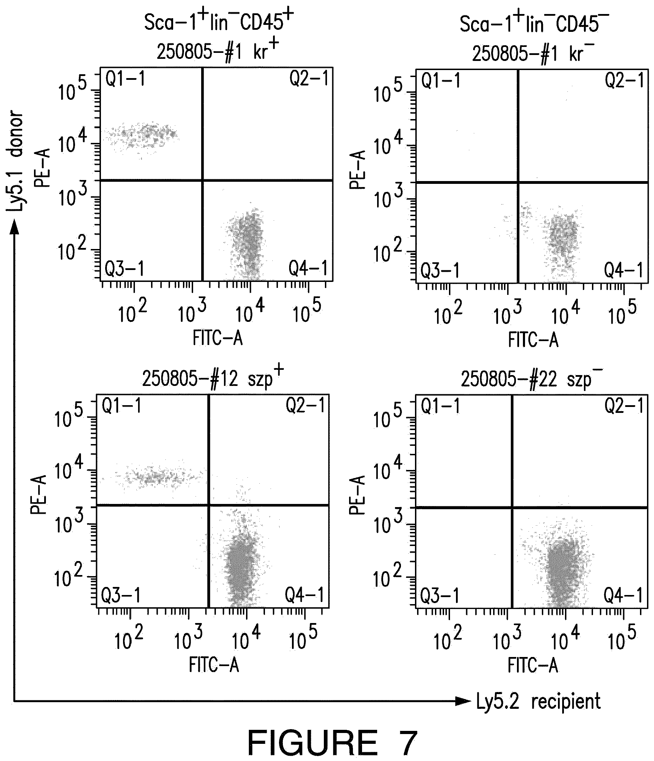

Figure 7 is four dot-plots depicting the results of transplantation of various subpopulations of cells and the contribution of these cells to long-term hematopoiesis. Sca-1⁺/lin⁻/CD45⁻ cells do not contribute to long-term hematopoiesis. Ly 5.2 mice were transplanted with 10⁴ Sca-1⁺/lin⁻/CD45⁺ or 2 x 10⁴ Sca-1⁺/lin⁻/CD45⁻ cells from Ly5.1 mice along with 10⁶ BMMNC Ly5.2 cells into Ly5.2 recipient mice and evaluated 8 months after transplantation for the presence of Ly5.1 cells by FACS. The upper panels depict analysis of MNC from the peripheral blood. The lower panels depict analysis of MNC from the bone marrow. Representative results are shown.

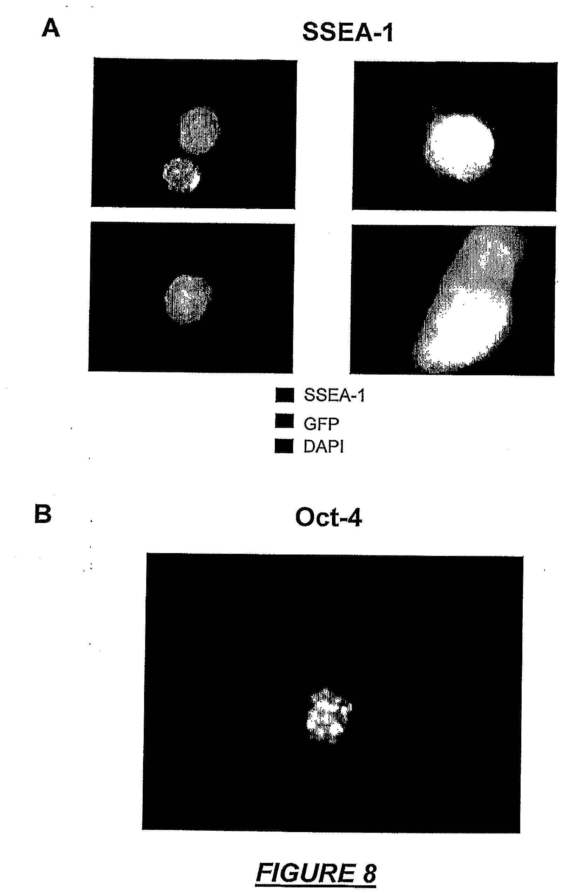

Figures 8A and 8B depict fluorescence micrographic images depicting staining of ES-like spheres of Sca-1⁺/lin⁻/CD45⁻ BM cells with antibodies specific for SSEA-1 (Figure 8A; 4 panels) or Oct-4 (Figure 8B).

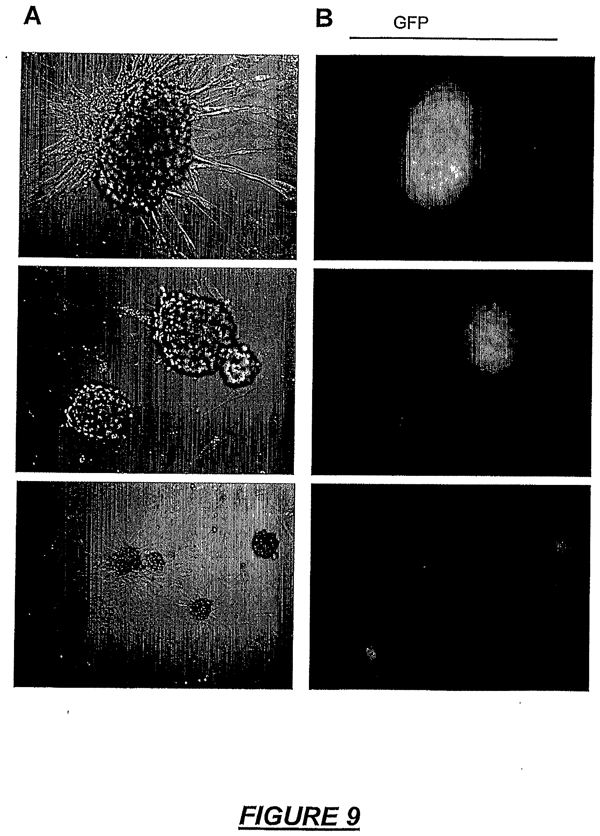

Figures 9A and 9B depict the formation of embryoid body-like spheres of GFP⁺ Sca-1⁺/lin⁻/CD45⁻ BM cells on C2C12 cells.
Figure 9A depicts a micrographic image of an embryoid body (EB)-like spheres after co-culture of Sca-1⁺/lin⁻/CD45⁻ BM cells with C2C12 cells under the conditions described in EXAMPLE 20. Figure 9B is a fluorescence micrographic image depicting the expression of green fluorescent protein (GFP) in the Sca-1⁺/lin⁻/CD45⁻ cells, indicating that these embryoid bodies are derived from purified Sca-1⁺/lin⁻/CD45⁻ BM cells isolated from green immunofluorescence positive (GFP+) mice (C57BL/6-Tg(ACTB-EGFP)1Osb/J mice purchased from The Jackson Laboratory, Bar Harbor, Maine, United States of America) and not the C2C12 cells.
Figure 10 is a series of dot-plots of propidium iodide-stained cells isolated from murine lymph nodes, HSCs (hematopoietic stem cells; Sca-1⁺/lin⁻/CD45⁺) or VSEL stem cells (Sca-1⁺/lin⁻/CD45⁻).
Figures 11A-11G are a series of dot-plots of FACS analysis of murine bone marrow cells.

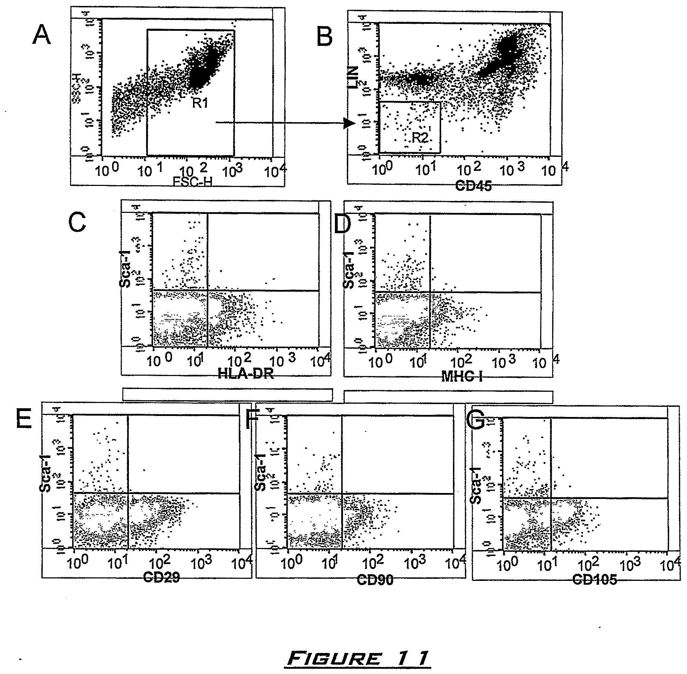

Figure 11A is a dot-plot of murine bone marrow MNC after hypotonic lysis. Figure 11B is a dot-plot showing staining of cells from R1 gate for lineage markers expression and CD45 antigen. In this Figure, R2 indicates lineage minus and CD45 negative BM MNC. Cells from R1 and R2 were analyzed for expression of Sca-1 and co-expression of HLA-DR (see Figure 11C), MHC class I (see Figure 11D), CD29 (see Figure 11E), CD90 (see Figure 11F), and CD105 (see Figure 11G) antigens.

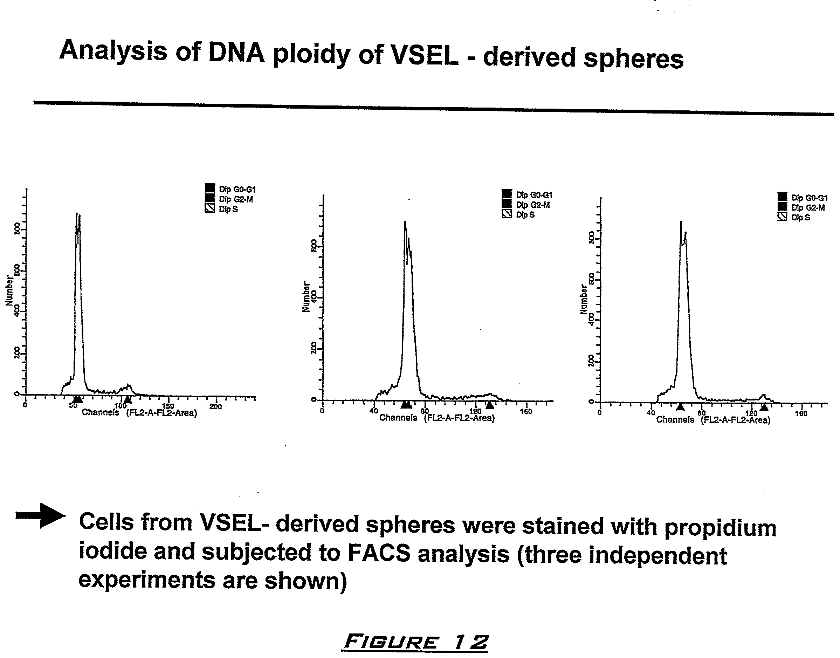

Figure 12 is a series of dot-plots of propidium iodide-stained cells from VSEL stem cell-derived spheres (VSEL-DS). Three independent representative examples are shown.

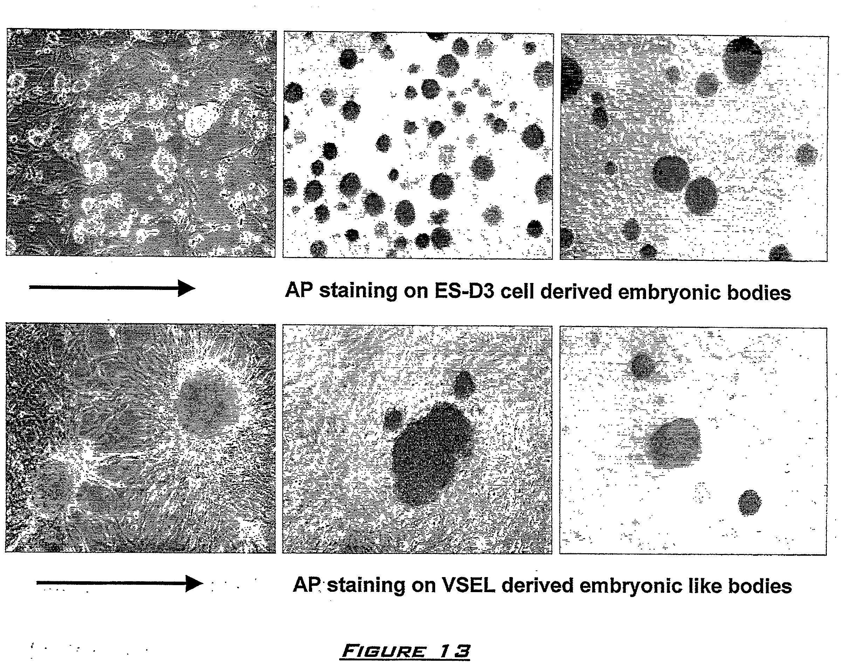

Figure 13 depicts photographs of alpkaline phosphatase (AP) staining on embryoid bodies formed from D3 embryonic stem cells (ED-D3; top panel) and on embryoid body-like spheres formed from VSEL stem cells (bottom panel).

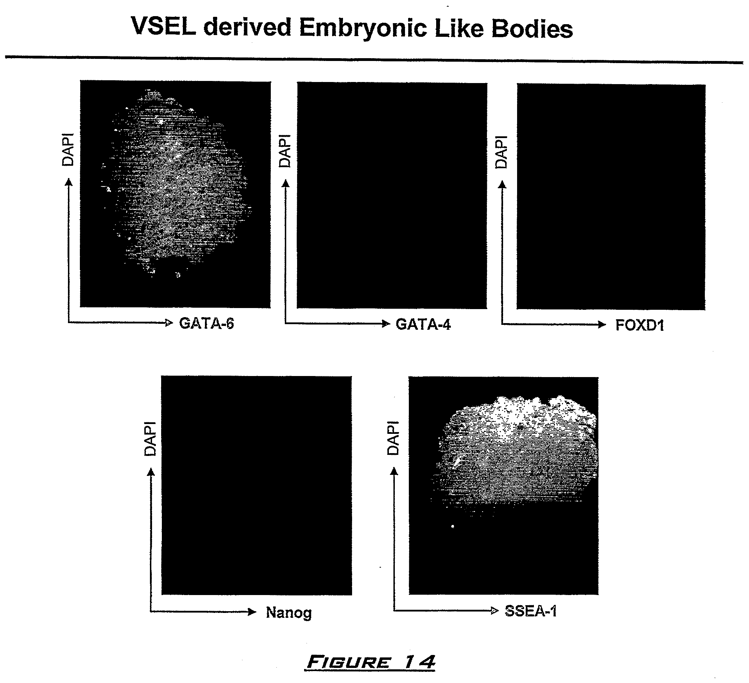

Figure 14 depicts fluorescence micrographic images demonstrating that VSEL stem cell- derived embryonic body-like spheres express early embryonic developmental markers such as SSEA-1, GATA-6, GATA-4, FOXD1, and Nanog.

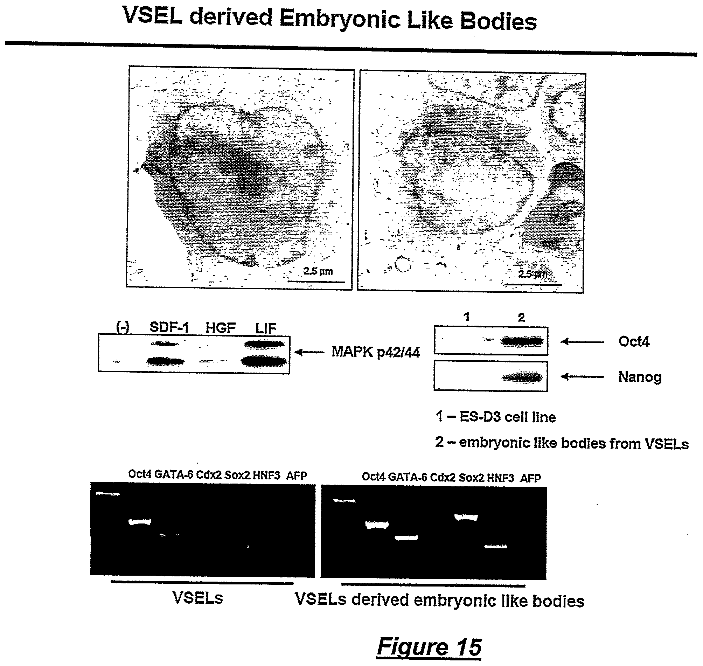

Figure 15 depicts transmission electron microscopy of the cells that were present in the VSEL stem cell-derived embryoid body-like spheres showing that these cells were larger in size than the original VSEL stem cells from which they were derived (Figure 15, upper panel), but still possessed very primitive nuclei containing euchromatin. The middle panel of Figure 15 depicts the results of studies of phosphorylation of MAPKp42/44 after stimulation of cells isolated from VSEL stem cell-derived embryoid body-like spheres with SDF-1, HGF/SF, and LIF, indicating that the corresponding receptors (CXCR4, c-met, and LIF-R, respectively) are expressed on the surfaces of these cells. And finally, the lower panel of Figure 15 depicts the results of RT-PCR analysis of cells isolated from consecutive passages of cells from VSEL stem cell-derived embryoid body-like spheres, which revealed an increase in expression of mRNA for genes regulating gastrulation of embryonic bodies such as GATA-6, Cdx2, Sox2, HNF3, and AFP.

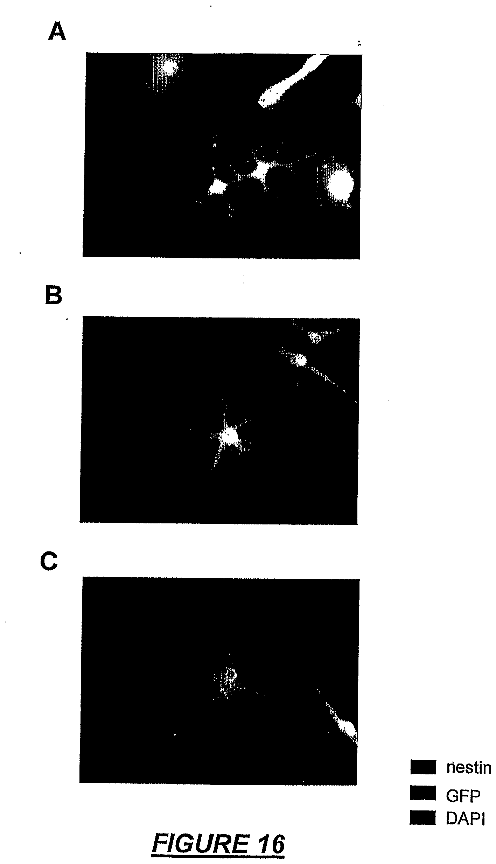

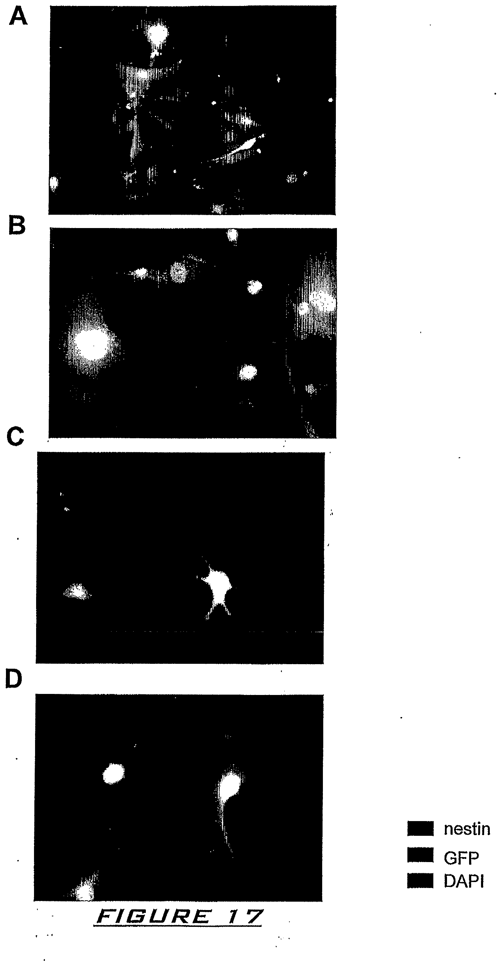

Figures 16A-16C and 17A-17D depict fluorescence micrographic images depicting the differentiation of ES like spheres into oligodendrocytes (Figures 16A-16C) or neurons (Figures 17A-17D). Cells were stained with antibodies directed to nestin, which were detecting using an Alexa Fluor 594-labeled goat anti-mouse IgG secondary antibody, which imparts a red fluorescence. GFP present in the cells was detected with an anti-green fluorescent protein Alexa Fluor 488 conjugate (green fluorescence), and nuclei were stained with DAPI (blue fluorescence).

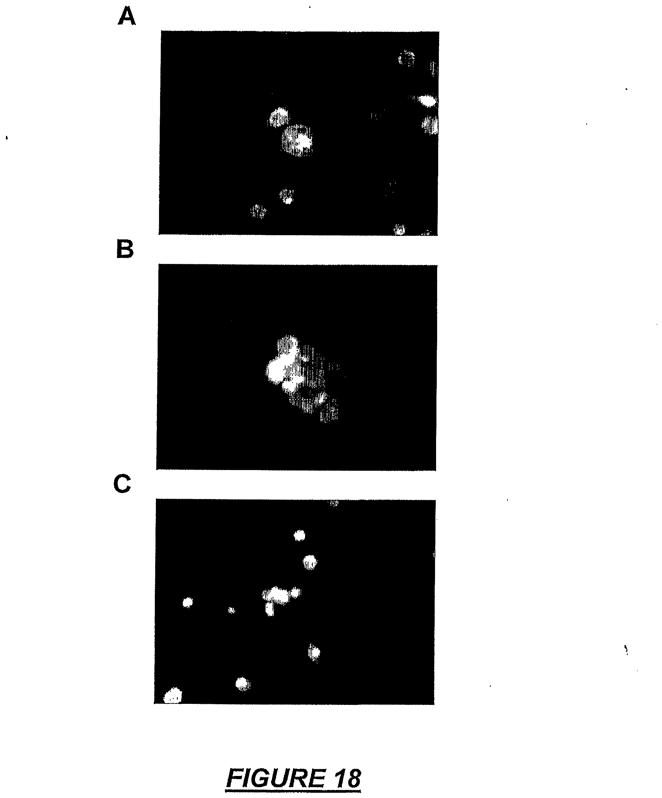

Figures 18A-18C depict fluorescence micrographic images depicting the differentiation of ES-like spheres into endodermal cells expressing a marker for pancreatic cells (C-peptide).

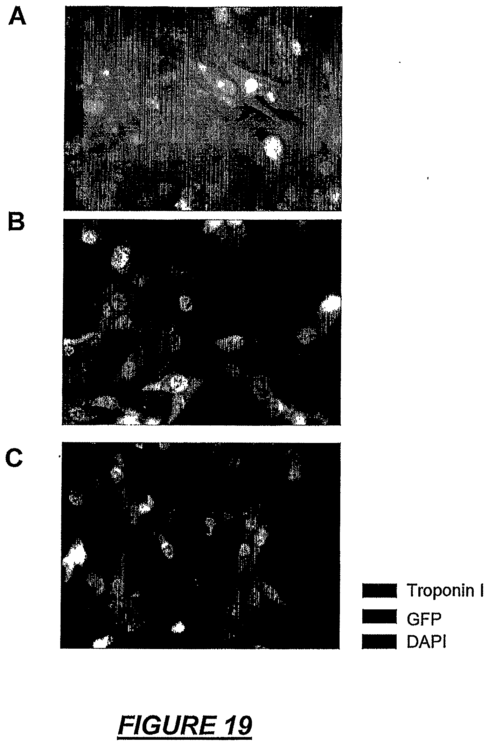

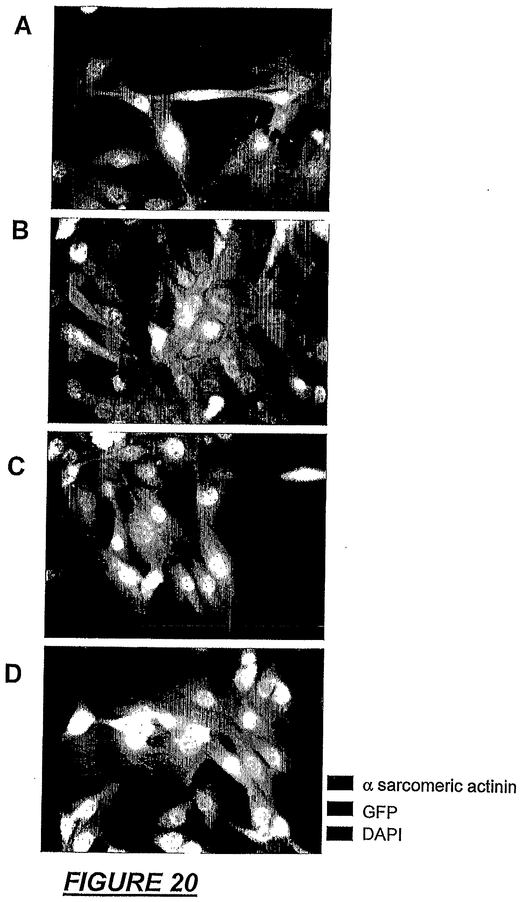

Figures 19A-19C and 20A-20D depict fluorescence micrographic images depicting the differentiation of ES-like spheres into cardiomyocytes. These cells express green fluorescent protein (GFP), indicating that the cardiomyocytes are derived from embryoid bodies formed by GFP⁺ Sca-1⁺/lin⁻/CD45⁻ BM cells. Cells were stained with antibodies directed to troponin I or α sarcomeric actinin (Figures 19 and 20, respectively), which were detecting using an Alexa Fluor 594-conjugated secondary antibody, which imparts a red fluorescence. GFP present in the cells was detected with an anti-green fluorescent protein Alexa Fluor 488 conjugate (green fluorescence), and nuclei were stained with DAPI (blue fluorescence).

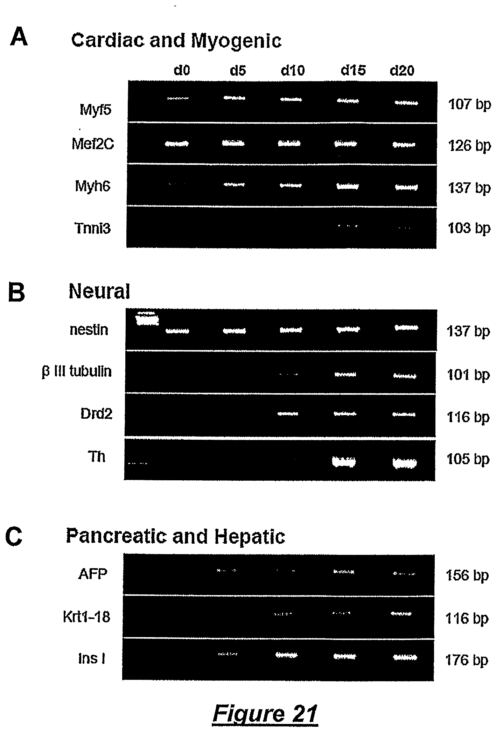

Figures 21A-21C depict the results of RT-PCR on cells from single VSEL stem cell-derived spheres, which indicated that these cells can differentiate into cardiomyocytes (mesoderm; see Figure 21A), neural cells and olgodendrocytes (ectoderm; seeFigure 21B), and pancreatic or hepatic cells (endoderm; see Figure 21C).

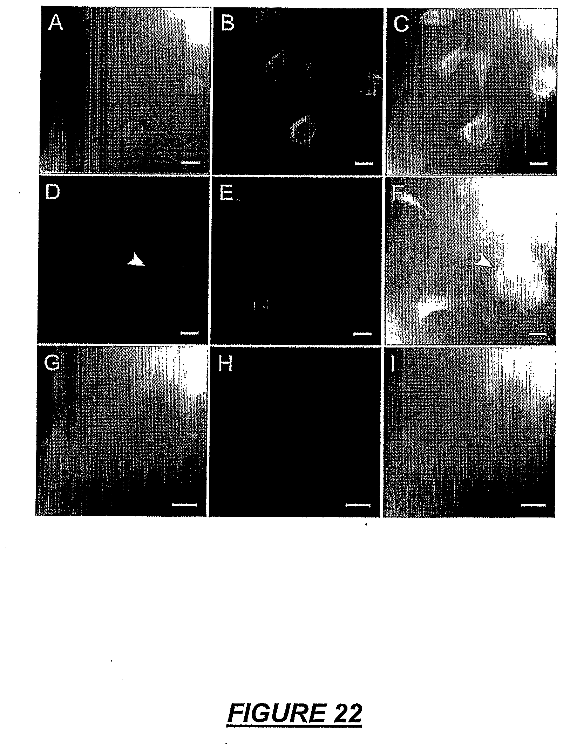

Figures 22A-22I depict immunofluorescent and transmission confocal microscopic images documenting the expression of cardiac-specific antigens in cultured cells.
Figures 22A-22C and 22D-22F depict images of culture plates in which Sca-1⁺/lin⁻/CD45⁻ BMMNCs were grown. Numerous cells in plates with Sca-1⁺/lin⁻/CD45⁻ cells were positive for cardiac-specific myosin heavy chain (Figures 22B, 22C, 22E, and 22F; green fluorescence). Many of these cardiac-specific myosin heavy chain-positive cells were also positive for cardiac troponin I (Figures 22D and 22F [arrowheads]; red fluorescence). Figures 22G-22I are images of culture plates in which Sca-1⁺/lin⁻/CD45⁺ cells were grown. These cells were largely negative for the expression of the aforementioned cardiac-specific antigens (see Figure 22H). Nuclei are identified in each of Figures 22A-22I by DAPI staining (blue fluorescence). Scale bar = 20 µm.

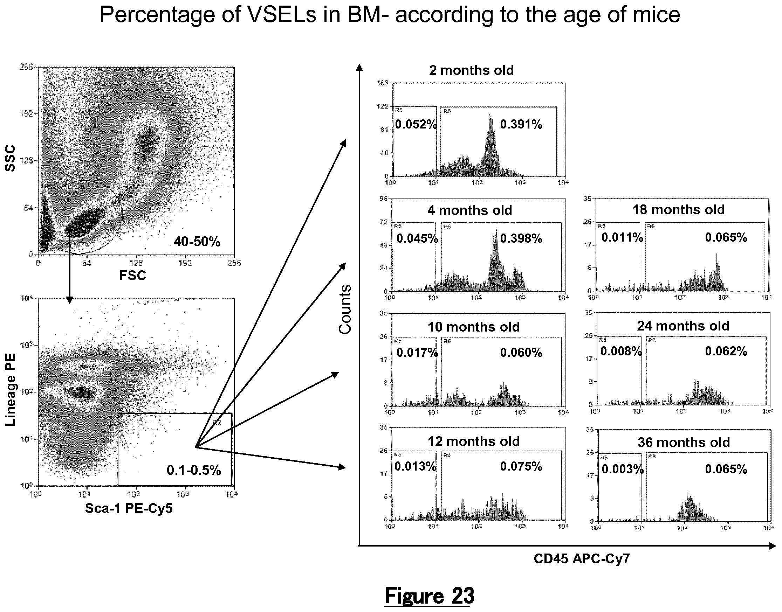

Figure 23 depicts the results of FACS sorting of Sca-1⁺/lin⁻/CD45⁻ cells showing that the yield of these cells that could be sorted decreased with age of the donor animal.

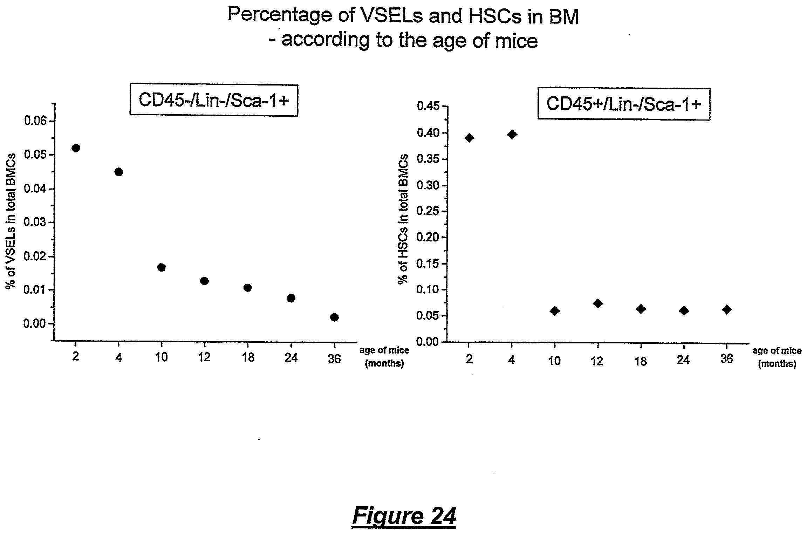

Figure 24 is two graphs depicting the percentages of VSEL stem cells (left panel) and HSCs (right panel) present in the bone marrow of mice as a function of age.

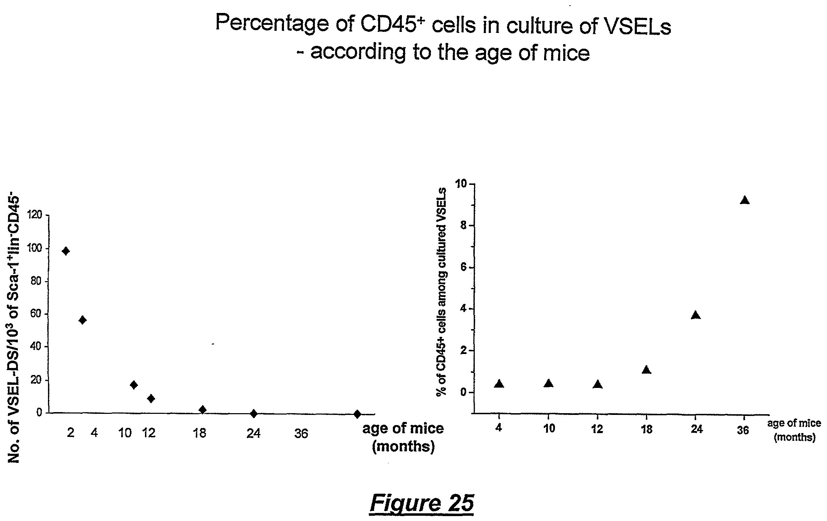

Figure 25 is two graphs depicting the decline in the ability of VSEL stem cells isolated from older mice to form embryoid body-like spheres (left panel) and the increased percentage of CD45⁺ cells in cultures of VSEL stem cells according to the age of the mice from which the cells were isolated (right panel).

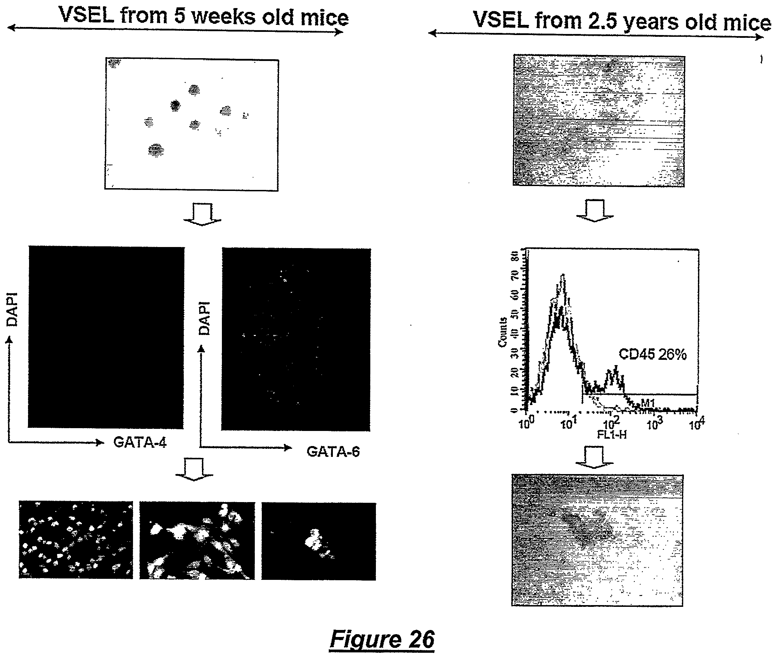

Figure 26 different expression patters for VSEL stem cells isolated from 5 week old mice (left panel) versus 2.5 year old mice (right panel). In the left panel are shown immunofluorescent and transmission confocal microscopic images documenting the expression of different hematopoietic antigens in cultured cells from 5 week old mice. In the right panel is shown that in VSEL stem cells isolated from 2.5 year old mice, CD45 is expressed and the cells were able to grow hematopoietic colonies in secondary cultures in methylcellulose cultures.

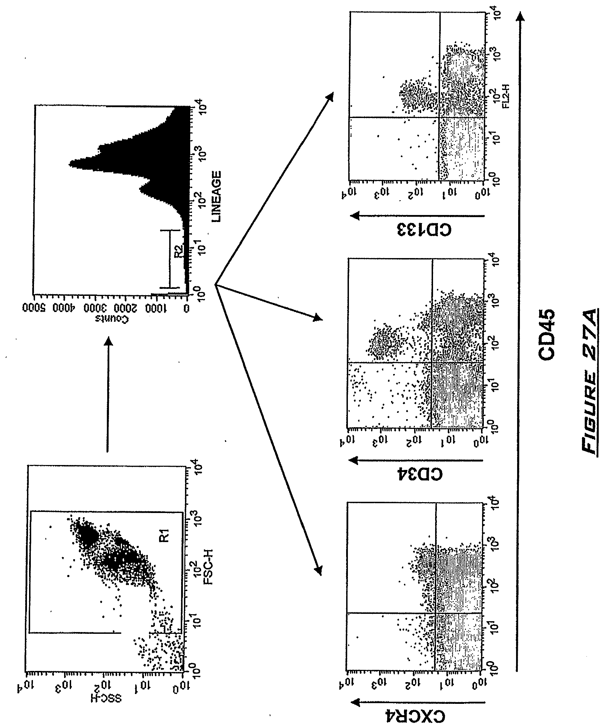

Figures 27A-27B depict the results of FACS sorting of human cord blood.

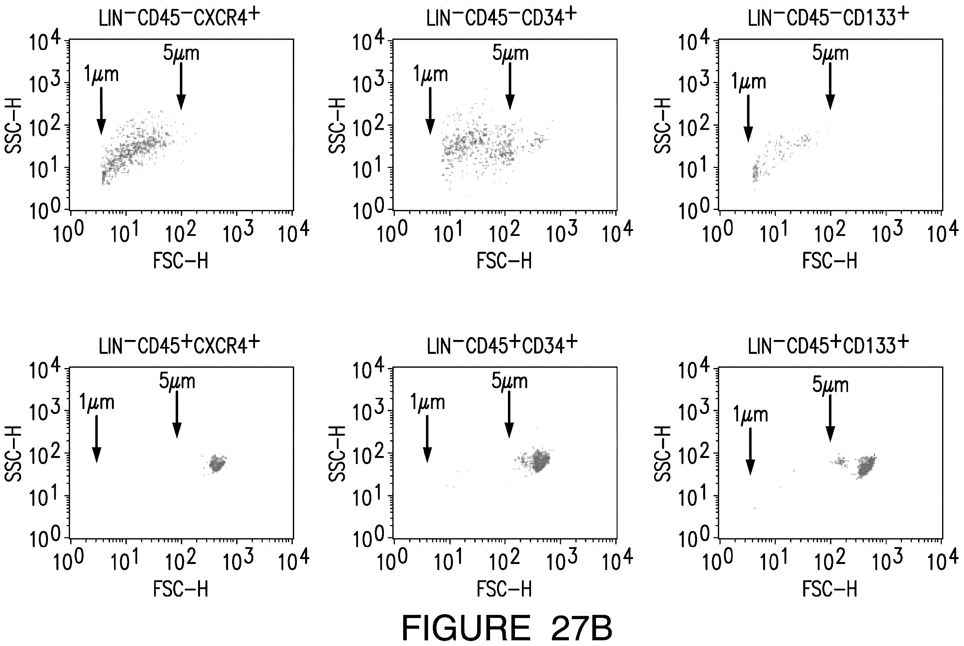

Figure 27A shows that human CB contained a population of lin⁻/CD45⁻ MNC that express CXCR4 (0.037 ± 0.02%, n = 9), CD34 (0.118 ± 0.028%, n = 5), and CD133 (0.018 ± 0.008%, n = 5). Figure 27B shows that these CXCR4⁺/CD133⁺/CD34⁺/lin⁻/CD45⁻ cells are very small (about 3-5 µm; Figure 27B, upper panel), whereas CB-derived lin⁻/CD45⁺ hematopoietic cells are larger (> 6 µm; Figure 27B, lower panel).

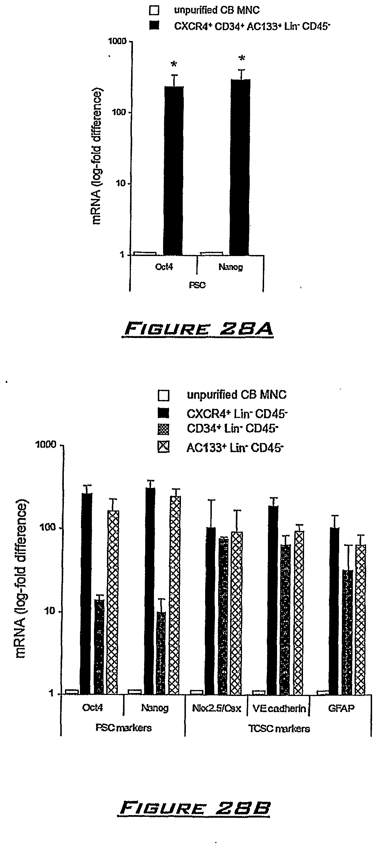

Figures 28A-28C depict the results of gene expression studies on sorted cells from human cord blood.

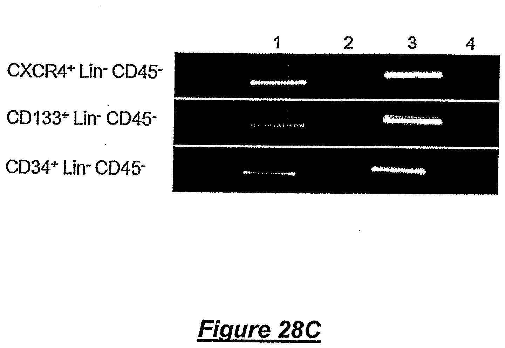

Figures 28A and 28B are bar graphs showing that CB-derived CXCR4⁺/CD133⁺/CD34⁺/lin⁻/CD45⁻ cells sorted by FACS, as well as CXCR4⁺/lin⁻/CD45⁻, CD34⁺/lin⁻/CD45⁻, and CD133⁺/lin⁻/CD45⁻/ cells are highly enriched for mRNA for transcriptions factors expressed by pluripotent embryonic cells such as Oct-4 and Nanog. Figure 28C shows the results of RT-PCR that confirm the FACS analysis.

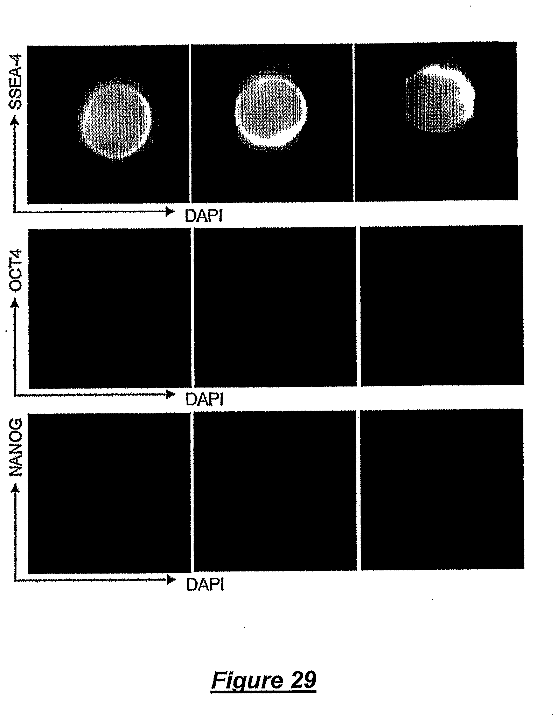

Figure 29 depicts the results of immunofluorescence staining of CB-VSEL stem cells showing that highly purified CB-derived CXCR4⁺/lin⁻/CD45⁻ cells expressed SSEA-4 on their surface and Oct-4 and Nanog transcription factors in nuclei.

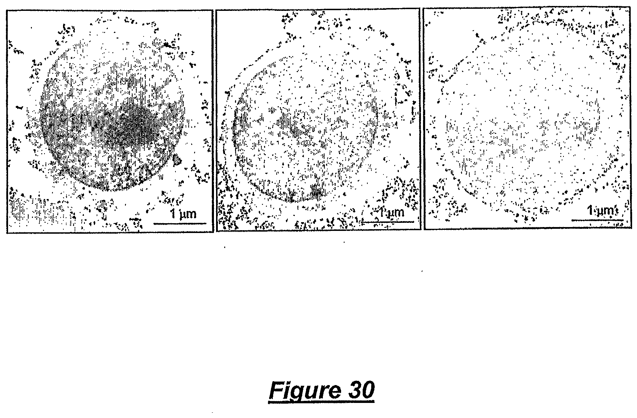

Figure 30 depicts photomicroscopic images of three different CB-VSEL stem cells demonstrating that these cells were very small -3-5 µm and contained relatively large nuclei and a narrow rim of cytoplasm with numerous mitochondria. DNA in the nuclei of these cells contained open-type euchromatin that is characteristic for pluripotent embryonic stem cells.

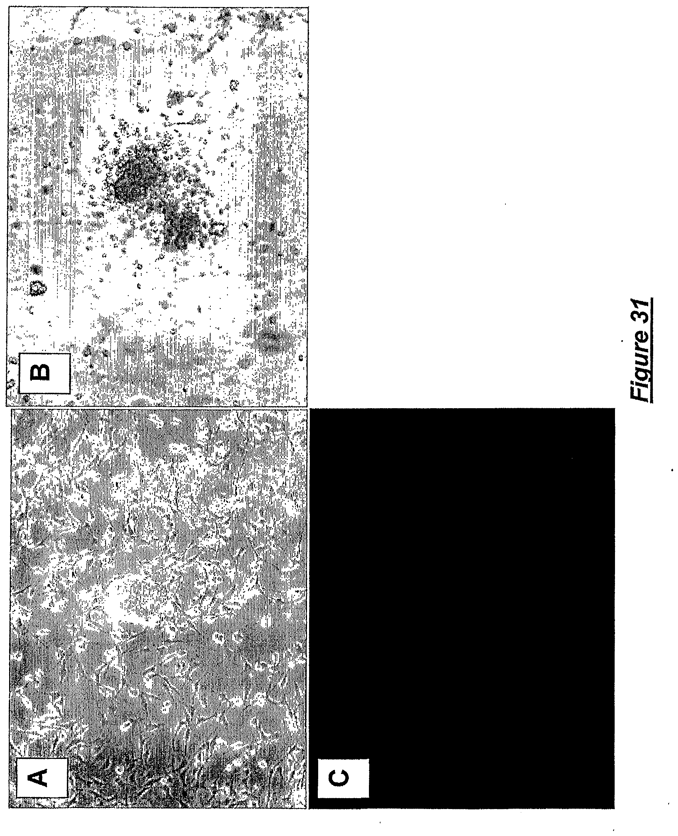

Figures 31A-31C depict photomicroscopic images showing that VSEL stem cell-DS derived from GFP⁺ mice can form small secondary spheres if plated in methylcellulose cultures supplemented with IL-3 + GM-CSF (Figures 31A and 31B). The single cell suspension prepared from these secondary spheres recovered by methylcellulose solubilization from the primary methylcellulose cultures, if plated again in methylcellulose cultures (Figure 31B) or plasma clot (Figure 31C) and stimulated by IL-3 and GM-CSF formed hematopoietic colonies. Evidence that these were hematopoietic colonies was obtained by FACS analysis of CD45 expression of cells derived from solubilized colonies growing in methylcellulose or by immunofluorescence staining cells from colonies growing in plasma clot cultures for CD45.

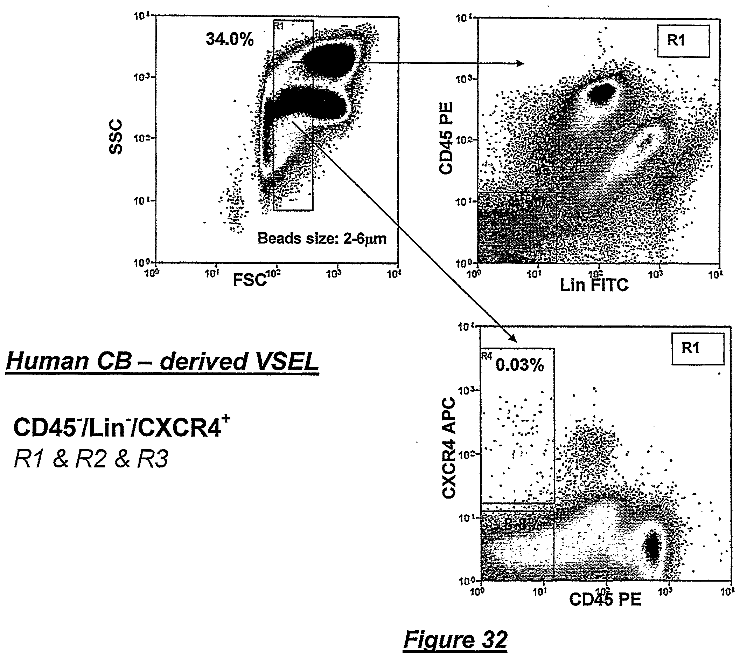

Figure 32 is an outline of a FACS-based strategy for isolating VSEL stem cells from human cord blood.

### BRIEF DESCRIPTION OF THE SEQUENCE LISTING

SEQ ID NOs: 1-64 are the nucleotide sequences of 32 primer pairs that can be used to amplify various murine nucleic acid sequences as summarized in Table 1.

**Table 1**

| Sequences of Murine Primers Employed for Real Time RT-PCR | |
|---|---|
| Gene (GENBANK® Accession No.) | Sequences (presented in 5' to 3' order) |
| *β*2 microglobulin (NM_009735) | CATACGCCTGCAGAGTTAAGCA (SEQ ID NO: 1) |
| | GATCACATGTCTCGATCCCAGTAG (SEQ ID NO: 2) |
| Oct4 (X52437) | ACCTTCAGGAGATATGCAAATCG (SEQ ID NO: 3) |
| | TTCTCAATGCTAGTTCGCTTTCTCT (SEQ ID NO: 4) |
| Nanog (AY278951) | CGTTCCCAGAATTCGATGCTT (SEQ ID NO: 5) |
| | TTTTCAGAAATCCCTTCCCTCG (SEQ ID NO: 6) |
| Rex1 (M28382) | AGATGGCTTCCCTGACGGATA (SEQ ID NO: 7) |
| | CCTCCAAGCTTTCGAAGGATTT (SEQ ID NO: 8) |
| Dppa3 (NM_139218) | GCAGTCTACGGAACCGCATT (SEQ ID NO: 9) |
| | TTGAACTTCCCTCCGGATTTT (SEQ ID NO: 10) |
| Rif1 (NM_175238) | GAGCTGGATTCTTTTGGATCAGTAA (SEQ ID NO: 11) |
| | GCCAAAGGTGACCAGACACA (SEQ ID NO: 12) |
| GFAP (X02801) | GGAGCTCAATGACCGCTTTG (SEQ ID NO: 13) |
| | TCCAGGAAGCGAACCTTCTC (SEQ ID NO: 14) |
| Nestin (NM_016701) | CCCTGATGATCCATCCTCCTT (SEQ ID NO: 15) |
| | CTGGAATATGCTAGAAACTCTAGACTCACT (SEQ ID NO: 16) |
| *β* III tubulin (NM_023279) | TCCGTTCGCTCAGGTCCTT (SEQ ID NO: 17) |
| | CCCAGACTGACCGAAAACGA (SEQ ID NO: 18) |
| Olig1 (NM_016968) | ACGTCGTAGCGCAGGCTTAT (SEQ ID NO: 19) |
| | CGCCCAACTCCGCTTACTT (SEQ ID NO: 20) |
| Olig2 (NM_016967) | GGGAGGCGCCATTGTACA (SEQ ID NO: 21) |
| | GTGCAGGCAGGAAGTTCCA (SEQ ID NO: 22) |
| Myf5 (NM_008656) | CTAGGAGGGCGTCCTTCATG (SEQ ID NO: 23) |
| | CACGTATTCTGCCCAGCTTTT (SEQ ID NO: 24) |
| MyoD (NM_010866) | GGACAGCCGGTGTGCATT (SEQ ID NO: 25) |
| | CACTCCGGAACCCCAACAG (SEQ ID NO: 26) |
| Myogenin (X15784) | GGAGAAGCGCAGGCTCAAG (SEQ ID NO: 27) |
| | TTGAGCAGGGTGCTCCTCTT (SEQ ID NO: 28) |
| Nsx2.5/Csx (AF091351) | CGGATGTGGCTCGTTTGC (SEQ ID NO: 29) |
| | TTGGGACCCTCCCGAGAT (SEQ ID NO: 30) |
| GATA-4 (U85046) | TCCAGTGCTGTCTGCTCTAAGC (SEQ ID NO: 31) |
| | TGGCCTGCGATGTCTGAGT (SEQ ID NO: 32) |
| α-fetoprotein (NM_007423) | ACCCGCTTCCCTCATCCT (SEQ ID NO: 33) |
| | AAACTCATTTCGTGCAATGCTT (SEQ ID NO: 34) |
| CK19 (M28698) | CATGCGAAGCCAATATGAGGT (SEQ ID NO: 35) |
| | TCAGCATCCTTCCGGTTCTG (SEQ ID NO: 36) |
| Nkx2-3 (NM_008699) | GGAGCCAAAAAAGCTGTCAGTT (SEQ ID NO: 37) |
| | CGTCCTCGCTCGTCCTACA (SEQ ID NO: 38) |
| Tcf4 (NM_013685) | ACCCTTGCACTCACTGCAAAG (SEQ ID NO: 39) |
| | GGAGAACATGAATCGCATCGT (SEQ ID NO: 40) |
| Nkx6.1 (NM_144955) | GCCTGTACCCCCCATCAAG (SEQ ID NO: 41) |
| | ACGTGGGTCTGGTGTGTTTTC (SEQ ID NO: 42) |
| Pdx1 (NM_008814) | CGGCTGAGCAAGCTAAGGTT (SEQ ID NO: 43) |
| | GGAAGAAGCGCTCTCTTTGAAA (SEQ ID NO: 44) |
| VE-cadherin (X83930) | TTCAAGCTGCCAGAAAACCA (SEQ ID NO: 45) |
| | GAGCCTTGTCAGGGTCTTTGG (SEQ ID NO: 46) |
| Krt2-5 (NM_027011) | CCCTCTGAACCTGCAAATCG (SEQ ID NO: 47) |
| | TGATCTGCTCCCTCTCCTCAGT (SEQ ID NO: 48) |
| Krt2-6a (NM_008476) | AGGAACCATGTCTACCAAAACCA (SEQ ID NO: 49) |
| | CTGGCTGAGCTGGCACTGT (SEQ ID NO: 50) |
| BNC (NM_007562) | CATGCACCCCTTTGAGAACCT (SEQ ID NO: 51) |
| | ATGTACTGTTCAGGCAGCGACC (SEQ ID NO: 52) |
| DCT (NM_010024) | CAGTTTCCCCGAGCTTGCAT (SEQ ID NO: 53) |
| | AGAGGCGGGCAGCATTC (SEQ ID NO: 54) |
| TYR (NM_011661) | CGAGCCTGTGCCTCCTCTAA (SEQ ID NO: 55) |
| | GACTCCCATCACCCATCCAT (SEQ ID NO: 56) |
| TYRP1 (NM_031202) | CCTAGCTCAGTTCTCTGGACATGA (SEQ ID NO: 57) |
| | GCAGGCCTCTAAGATACGAGAATT (SEQ ID NO: 58) |
| CXCR4 (BC031665) | GACGGACAAGTACCGGCTGC (SEQ ID NO: 59) |
| | GACAGCTTAGAGATGATGAT (SEQ ID NO: 60) |
| Met receptor (NM_008591) | CGCGTCGACTTATTCATGG (SEQ ID NO: 61) |
| | CACACATTGATTGTGGCACC (SEQ ID NO: 62) |
| LIF-R (NM_013584) | GAGCATCCTTTGCTATCGGAAGC (SEQ ID NO: 63) |
| | CGTTATTTCCTCCTCGATGATGG (SEQ ID NO: 64) |

SEQ ID NOs: 65-80 are the nucleotide sequences of 8 primer pairs that can be used to amplify various human nucleic acid sequences as summarized in Table 2.

**Table 2**

| Sequences of Human Primers Employed for Real Time RT-PCR | |
|---|---|
| Gene (GENBANK® Accession No.) | Sequences (presented in 5' to 3' order) |
| Oct4 (DQ486513) | TTGCCAAGCTCCTGAAGCA (SEQ ID NO: 65) |
| | CGTTTGGCTGAATACCTTCCC (SEQ ID NO: 66) |
| Nanog (NM_024865) | CCCAAAGCTTGCCTTGCTTT (SEQ ID NO: 67) |
| | AGACAGTCTCCGTGTGAGGCAT (SEQ ID NO: 68) |
| Oct4 (DQ486513) | GATGTGGTCCGAGTGTGGTTCT (SEQ ID NO: 69) |
| | TGTGCATAGTCGCTGCTTGAT (SEQ ID NO: 70) |
| Nanog (NM_024865) | GCAGAAGGCCTCAGCACCTA (SEQ ID NO: 71) |
| | AGGTTCCCAGTCGGGTTCA (SEQ ID NO: 72) |
| Nkx2.5/Csx (NM_004387) | CCCCTGGATTTTGCATTCAC (SEQ ID NO: 73) |
| | CGTGCGCAAGAACAAACG (SEQ ID NO: 74) |
| VE-cadherin (AF240635) | CCGACAGTTGTAGGCCCTGTT (SEQ ID NO: 75) |
| | GGCATCTTCGGGTTGATCCT (SEQ ID NO: 76) |
| GFAP (NM_002055) | GTGGGCAGGTGGGAGCTTGATTCT (SEQ ID NO: 77) |
| | CTGGGGCGGCCTGGTATGACA (SEQ ID NO: 78) |
| *β*2 microglobulin (NM_004048) | AATGCGGCATCTTCAAACCT (SEQ ID NO: 79) |
| | TGACTTTGTCACAGCCCAAGATA (SEQ ID NO: 80) |

### DETAILED DESCRIPTION

The present subject matter will be now be described more fully hereinafter with reference to the accompanying Examples, in which representative embodiments of the presently disclosed subject matter are shown. The presently disclosed subject matter can, however, be embodied in different forms and should not be construed as limited to the embodiments set forth herein. Rather, these embodiments are provided so that this disclosure will be thorough and complete, and will fully convey the scope of the presently disclosed subject matter to those skilled in the art.

Throughout the specification and claims, a given chemical formula or name shall encompass all optical isomers and stereoisomers as well as racemic mixtures where such isomers and mixtures exist.

### I. General Considerations

The concept that hematopoietic stem cells (HSC) isolated from relatively easily accessible sources such as bone marrow (BM), mobilized peripheral blood (mPB), or cord blood (CB) could be subsequently employed as precursors for other stem cells necessary for regeneration of various solid organs (*e.g*., heart, brain, liver or pancreas) created excitement in the scientific community. It had been postulated that HSC possess germlayer-unrestricted plasticity and can transdifferentiate into stem cells from all non-hematopoietic lineages. Unfortunately, the first promising reports showing a robust contribution of "HSC" to regeneration of different tissues were not reproduced by other investigators.

In response to this, the scientific community became polarized in its view on stem cell plasticity. Several alternative explanations of previously reported data have been proposed. The first concept that was rapidly accepted was explaining stem cell plasticity through the phenomenon of cell fusion. Data were presented that donor-derived HSC and/or monocytes might fuse with differentiated cells present in recipient tissues, leading to the creation of fused cells that have a double number of chromosomes in their nuclei and express cell surface and cytoplasmic markers that are derived from both "parental" cells.

Another explanation of stem cell plasticity is based on the appearance of epigenetic changes in cells exposed to external stimuli (*e.g*., organ damage, non-physiological culture conditions, and/or other stresses). Both cell fusion and epigenetic changes, however, are extremely rare, randomly occurring events that would not appear to fully account for the previously published positive "trans-dedifferentiation" data. Furthermore, fusion was excluded as a major contributor to the observed donor derived chimerism in several recently published studies.

The concept that BM might contain heterogeneous populations of stem cells was surprisingly not appreciated as a part of the discussion concerning stem cell plasticity. Disclosed herein is direct evidence that BM stem cells are heterogeneous and expected to be pluripotent. BM has been shown to contain endothelial-, bone-, skeletal muscle-, cardiac-, hepatic-, and neural-tissue committed stem cells.

However, these potential candidate cells had not been characterized well at the single cell level. As disclosed herein, murine bone marrow (BM) contains a population of rare (∼0.02% of BMMNC) Sca-1⁺/lin⁻/CD45⁻ cells that express markers of non-hematopoietic stem cells. More importantly, these rare cells were able to differentiate into cardiomyocytes, pancreatic cells, and grow neurospheres in *in vitro* cultures. These Sca-1⁺/lin⁻/CD45⁻ cells have the morphology of, and express several markers of, undifferentiated embryonic-like stem cells.

Disclosed herein is the identification and purification from murine bone marrow (BM) of a subpopulation of rare CD34⁺/lin⁻/CD45⁻ (human) or Sca-1⁺/lin⁻/CD45⁻ (mouse) cells, referred to herein as "very small embryonic-like (VSEL) stem cells". In addition to being Sca-1⁺/lin⁻/CD45⁻ or CD34⁺/lin⁻/CD45⁻, VSEL stem cells express markers of pluripotent stem cells (PSC) such as SSEA-1, Oct-4, Nanog, and Rex-1. The direct electron microscopic analysis revealed that VSEL stem cells are small (about 3-4 µm), possess large nuclei surrounded by a narrow rim of cytoplasm, and contain open-type chromatin (euchromatin) that is typical of embryonic stem cells. The number of VSEL stem cells is highest in BM from young (∼1 month-old) mice, and decreases with age. It is also significantly diminished in short living DBA/2J mice as compared to long living C57BL/6 animals. VSEL stem cells respond strongly to SDF-1, HGF/SF, and LIF *in vitro,* and express CXCR4, c-met, and LIF-R. This population of VSEL stem cells expressing pluripotent- and tissue committed stem cells markers can be a source of pluripotent stem cells for tissue and/or organ regeneration.

### II. Definitions

All technical and scientific terms used herein, unless otherwise defined below, are intended to have the same meaning as commonly understood by one of ordinary skill in the art. References to techniques employed herein are intended to refer to the techniques as commonly understood in the art, including variations on those techniques or substitutions of equivalent techniques that would be apparent to one of skill in the art. While the following terms are believed to be well understood by one of ordinary skill in the art, the following definitions are set forth to facilitate explanation of the presently disclosed subject matter.

Following long-standing patent law convention, the terms "a", "an", and "the" mean "one or more" when used in this application, including the claims. Thus, the phrase "a stem cell" refers to one or more stem cells, unless the context clearly indicates otherwise.

The terms "target tissue" and "target organ" as used herein refer to an intended site for accumulation of VSEL stem cells and/or an *in vitro* differentiated VSEL stem cell derivative following administration to a subject. For example, in some embodiments the methods of the presently disclosed subject matter involve a target tissue or a target organ that has been damaged, for example by ischemia or other injury.

The term "control tissue" as used herein refers to a site suspected to substantially lack accumulation of an administered cell. For example, in accordance with the methods of the presently disclosed subject matter, a tissue or organ that has not been injured or damaged is a representative control tissue, as is a tissue or organ other than the intended target tissue. For example, if the injury to be treated is a myocardial infarction, the intended target tissue would be the heart, and essentially all other tissues and organs in the subject can be considered control tissues.

The terms "targeting" and "homing", as used herein to describe the *in vivo* activity of a cell (for example, a VSEL stem cells and/or an *in vitro* differentiated VSEL stem cell derivative thereof) following administration to a subject, and refer to the preferential movement and/or accumulation of the cell in a target tissue as compared to a control tissue.

The terms "selective targeting" and "selective homing" as used herein refer to a preferential localization of a cell (for example, a VSEL stem cells and/or an *in vitro* differentiated VSEL stem cell derivative thereof) that results in an accumulation of the administered VSEL stem cells and/or an *in vitro* differentiated VSEL stem cell derivative thereof in a target tissue that is in some embodiments about 2-fold greater than accumulation of the administered VSEL stem cells and/or an *in vitro* differentiated VSEL stem cell derivative thereof in a control tissue, in some embodiments accumulation of the administered VSEL stem cells and/or an *in vitro* differentiated VSEL stem cell derivative thereof that is about 5-fold or greater, and in some embodiments an accumulation of the administered VSEL stem cells and/or an *in vitro* differentiated VSEL stem cell derivative thereof that is about 10-fold or greater than in an control tissue. The terms "selective targeting" and "selective homing" also refer to accumulation of a VSEL stem cells and/or an *in vitro* differentiated VSEL stem cell derivative thereof in a target tissue concomitant with an absence of accumulation in a control tissue, in some embodiments the absence of accumulation in all control tissues.

The term "absence of targeting" is used herein to describe substantially no binding or accumulation of a VSEL stem cells and/or an *in vitro* differentiated VSEL stem cell derivative thereof in one or more control tissues under conditions wherein accumulation would be detectable if present. The phrase also is intended to include minimal, background accumulation of a VSEL stem cells and/or an *in vitro* differentiated VSEL stem cell derivative thereof in one or more control tissues under such conditions.

The term "subject" as used herein refers to a member of any invertebrate or vertebrate species. Accordingly, the term "subject" is intended to encompass any member of the Kingdom *Animalia* including, but not limited to the phylum *Chordata* (*i.e*., members of Classes *Osteichythyes* (bony fish), *Amphibia* (amphibians), *Reptilia* (reptiles), *Aves* (birds), and *Mammalia* (mammals)), and all Orders and Families encompassed therein.

Similarly, all genes, gene names, and gene products disclosed herein are intended to correspond to homologs from any species for which the compositions and methods disclosed herein are applicable. Thus, the terms include, but are not limited to genes and gene products from humans and mice. It is understood that when a gene or gene product from a particular species is disclosed, this disclosure is intended to be exemplary only, and is not to be interpreted as a limitation unless the context in which it appears clearly indicates. Thus, for example, for the genes listed in Tables 1 and 2, which disclose GENBANK® Accession Nos. for the murine and human nucleic acid sequences, respectively, are intended to encompass homologous genes and gene products from other animals including, but not limited to other mammals, fish, amphibians, reptiles, and birds.

The methods of the presently disclosed subject matter are particularly useful for warm-blooded vertebrates. Thus, the presently disclosed subject matter concerns mammals and birds. More particularly contemplated is the isolation, manipulation, and use of VSEL stem cells from mammals such as humans and other primates, as well as those mammals of importance due to being endangered (such as Siberian tigers), of economic importance (animals raised on farms for consumption by humans) and/or social importance (animals kept as pets or in zoos) to humans, for instance, carnivores other than humans (such as cats and dogs), swine (pigs, hogs, and wild boars), ruminants (such as cattle, oxen, sheep, giraffes, deer, goats, bison, and camels), rodents (such as mice, rats, and rabbits), marsupials, and horses. Also provided is the use of the disclosed methods and compositions on birds, including those kinds of birds that are endangered, kept in zoos, as well as fowl, and more particularly domesticated fowl, *e.g.,* poultry, such as turkeys, chickens, ducks, geese, guinea fowl, and the like, as they are also of economic importance to humans. Thus, also contemplated is the isolation, manipulation, and use of VSEL stem cells from livestock, including but not limited to domesticated swine (pigs and hogs), ruminants, horses, poultry, and the like.

The term "about", as used herein when referring to a measurable value such as an amount of weight, time, dose, etc., is meant to encompass variations of in some embodiments ±20%, in some embodiments ±10%, in some embodiments ±5%, in some embodiments ±1%, and in some embodiments ±0.1% from the specified amount, as such variations are appropriate to perform the disclosed methods.

The term "isolated", as used in the context of a nucleic acid or polypeptide (including, for example, a peptide), indicates that the nucleic acid or polypeptide exists apart from its native environment. An isolated nucleic acid or polypeptide can exist in a purified form or can exist in a non-native environment.

The terms "nucleic acid molecule" and "nucleic acid" refer to deoxyribonucleotides, ribonucleotides, and polymers thereof, in single-stranded or double-stranded form. Unless specifically limited, the term encompasses nucleic acids containing known analogues of natural nucleotides that have similar properties as the reference natural nucleic acid. The terms "nucleic acid molecule" and "nucleic acid" can also be used in place of "gene", "cDNA", and "mRNA". Nucleic acids can be synthesized, or can be derived from any biological source, including any organism.

The term "isolated", as used in the context of a cell (including, for example, a VSEL stem cell), indicates that the cell exists apart from its native environment. An isolated cell can also exist in a purified form or can exist in a non-native environment.

As used herein, a cell exists in a "purified form" when it has been isolated away from all other cells that exist in its native environment, but also when the proportion of that cell in a mixture of cells is greater than would be found in its native environment. Stated another way, a cell is considered to be in "purified form" when the population of cells in question represents an enriched population of the cell of interest, even if other cells and cell types are also present in the enriched population. A cell can be considered in purified form when it comprises in some embodiments at least about 10% of a mixed population of cells, in some embodiments at least about 20% of a mixed population of cells, in some embodiments at least about 25% of a mixed population of cells, in some embodiments at least about 30% of a mixed population of cells, in some embodiments at least about 40% of a mixed population of cells, in some embodiments at least about 50% of a mixed population of cells, in some embodiments at least about 60% of a mixed population of cells, in some embodiments at least about 70% of a mixed population of cells, in some embodiments at least about 75% of a mixed population of cells, in some embodiments at least about 80% of a mixed population of cells, in some embodiments at least about 90% of a mixed population of cells, in some embodiments at least about 95% of a mixed population of cells, and in some embodiments about 100% of a mixed population of cells, with the proviso that the cell comprises a greater percentage of the total cell population in the "purified" population that it did in the population prior to the purification. In this respect, the terms "purified" and "enriched" can be considered synonymous.

### III. Isolation of Very Small Embryonic-like (VSEL) Stem Cells

### III.A. Generally

The presently disclosed subject matter provides methods of isolating a subpopulation of CD45⁻ stem cells from a population of cells. In some embodiments, the method comprises (a) providing a population of cells suspected of comprising CD45⁻ stem cells; (b) contacting the population of cells with a first antibody that is specific for CD45 and a second antibody that is specific for CD34 or Sca-1 under conditions sufficient to allow binding of each antibody to its target, if present, on each cell of the population of cells; (c) selecting a first subpopulation of cells that are CD34⁺ or Sca-1⁺, and are also CD45⁻; (d) contacting the first subpopulation of cells with one or more antibodies that are specific for one or more cell surface markers selected from the group including but not limited to CD45R/B220, Gr-1, TCRα*β*, TCRγδ, CD11b, and Ter-119 under conditions sufficient to allow binding of each antibody to its target, if present, on each cell of the population of cells; (e) removing from the first subpopulation of cells those cells that bind to at least one of the antibodies of step (d); and (f) collecting a second subpopulation of cells that are either CD34⁺/lin⁻/CD45⁻ or Sca-1⁺/lin⁻/CD45⁻, whereby a subpopulation of CD45⁻ stem cells is isolated.

As used herein, the term "CD45" refers to a tyrosine phosphatase, also known as the leukocyte common antigen (LCA), and having the gene symbol PTPRC. This gene corresponds to GENBANK® Accession Nos. NP_002829 (human), NP_035340 (mouse), NP_612516 (rat), XP_002829 (dog), XP_599431 (cow) and AAR16420 (pig). The amino acid sequences of additional CD45 homologs are also present in the GENBANK® database, including those from several fish species and several non-human primates.

As used herein, the term "CD34" refers to a cell surface marker found on certain hematopoietic and non-hematopoietic stem cells, and having the gene symbol CD34. The GENBANK® database discloses amino acid and nucleic acid sequences of CD34 from humans (e.g., AAB25223), mice (NP_598415), rats (XP_223083), cats (NP_001009318), pigs (MP_999251), cows (NP_776434), and others.

In mice, some stem cells also express the stem cell antigen Sca-1 (GENBANK® Accession No. NP_034868), also referred to as Lymphocyte antigen Ly-6A.2.

Thus, the subpopulation of CD45⁻ stem cells represents a subpopulation of all CD45⁻ cells that are present in the population of cells prior to the separating step. In some embodiments, the subpopulation of CD45⁻ stem cells are from a human, and are CD34⁺/CXCR4⁺/lin⁻/CD45⁻. In some embodiments, the subpopulation of CD45⁻ stem cells are from a mouse, and are Sca-1⁺/lin⁻/CD45⁻.

The isolation of the disclosed subpopulations can be performed using any methodology that can separate cells based on expression or lack of expression of the one or more of the CD45, CXCR4, CD34, AC133, Sca-1, CD45R/B220, Gr-1, TCRa*β*, TCRγδ, CD11b, and Ter-119 markers including, but not limited to fluorescence-activated cell sorting (FACS).

As used herein, lin⁻ refers to a cell that does not express any of the following markers: CD45R/B220, Gr-1, TCRa*β*, TCRγδ, CD11b, and Ter-119. These markers are found on cells of the B cell lineage from early Pro-B to mature B cells (CD45R/B220); cells of the myeloid lineage such as monocytes during development in the bone marrow, bone marrow granulocytes, and peripheral neutrophils (Gr-1); thymocytes, peripheral T cells, and intestinal intraepithelial lymphocytes (TCRa*β* and TCRγδ); myeloid cells, NK cells, some activated lymphocytes, macrophages, granulocytes, B1 cells, and a subset of dendritic cells (CD11b); and mature erythrocytes and erythroid precursor cells (Ter-119).

The separation step can be performed in a stepwise manner as a series of steps or concurrently. For example, the presence or absence of each marker can be assessed individually, producing two subpopulations at each step based on whether the individual marker is present. Thereafter, the subpopulation of interest can be selected and further divided based on the presence or absence of the next marker.

Alternatively, the subpopulation can be generated by separating out only those cells that have a particular marker profile, wherein the phrase "marker profile" refers to a summary of the presence or absence of two or more markers. For example, a mixed population of cells can contain both CD34⁺ and CD34⁻ cells. Similarly, the same mixed population of cells can contain both CD45⁺ and CD45⁻ cells. Thus, certain of these cells will be CD34⁺/CD45⁺, others will be CD34⁺/CD45⁻, others will be CD34⁻/CD45⁺, and others will be CD34⁻/CD45⁻. Each of these individual combinations of markers represents a different marker profile. As additional markers are added, the profiles can become more complex and correspond to a smaller and smaller percentage of the original mixed population of cells. In some embodiments, the cells of the presently disclosed subject matter have a marker profile of CD34⁺/CXCR4⁺/lin⁻/CD45⁻, and in some embodiments, the cells of the presently disclosed subject matter have a marker profile of Sca-1⁺/lin⁻/CD45⁻.

In some embodiments of the presently disclosed subject matter, antibodies specific for markers expressed by a cell type of interest (*e.g*., polypeptides expressed on the surface of a CD34⁺/CXCR4⁺/lin⁻/CD45⁻ or a Sca-1⁺/lin⁻/CD45⁻ cell) are employed for isolation and/or purification of subpopulations of BM cells that have marker profiles of interest. It is understood that based on the marker profile of interest, the antibodies can be used to positively or negatively select fractions of a population, which in some embodiments are then further fractionated.

In some embodiments, a plurality of antibodies, antibody derivatives, and/or antibody fragments with different specificities is employed. In some embodiments, each antibody, or fragment or derivative thereof, is specific for a marker selected from the group including but not limited to Ly-6A/E (Sca-1), CD34, CXCR4, AC133, CD45, CD45R, B220, Gr-1, TCRα*β*, TCRγδ, CD11b, Ter-119, c-met, LIF-R, SSEA-1, Oct-4, Rev-1, and Nanog. In some embodiments, cells that express one or more genes selected from the group including but not limited to SSEA-1, Oct-4, Rev-1, and Nanog are isolated and/or purified.

The presently disclosed subject matter relates to a population of cells that in some embodiments express the following antigens: CXCR4, AC133, CD34, SSEA-1 (mouse) or SSEA-4 (human), fetal alkaline phosphatase (AP), c-met, and the LIF-Receptor (LIF-R). In some embodiments, the cells of the presently disclosed subject matter do not express the following antigens: CD45, Lineage markers (*i.e*., the cells are lin⁻), HLA-DR, MHC class I, CD90, CD29, and CD105. Thus, in some embodiments the cells of the presently disclosed subject matter can be characterized as follows: CXCR4⁺/AC133⁺/CD34⁺/SSEA-1⁺ (mouse) or SSEA-4⁺ (human)/AP⁺/c-met⁺/LIF-R⁺/CD45⁻/lin⁻/HLA-DR⁻/MHC class I⁻/CD90⁻/CD29⁻/CD105⁻.

In some embodiments, each antibody, or fragment or derivative thereof, comprises a detectable label. Different antibodies, or fragments or derivatives thereof, which bind to different markers can comprise different detectable labels or can employ the same detectable label.

A variety of detectable labels are known to the skilled artisan, as are methods for conjugating the detectable labels to biomolecules such as antibodies and fragments and/or derivatives thereof. As used herein, the phrase "detectable label" refers to any moiety that can be added to an antibody, or a fragment or derivative thereof, that allows for the detection of the antibody. Representative detectable moieties include, but are not limited to, covalently attached chromophores, fluorescent moieties, enzymes, antigens, groups with specific reactivity, chemiluminescent moieties, and electrochemically detectable moieties, etc. In some embodiments, the antibodies are biotinylated. In some embodiments, the biotinylated antibodies are detected using a secondary antibody that comprises an avidin or streptavidin group and is also conjugated to a fluorescent label including, but not limited to Cy3, Cy5, and Cy7. In some embodiments, the antibody, fragment, or derivative thereof is directly labeled with a fluorescent label such as Cy3, Cy5, or Cy7. In some embodiments, the antibodies comprise biotin-conjugated rat anti-mouse Ly-6A/E (Sca-1; clone E13-161.7), streptavidin-PE-Cy5 conjugate, anti-CD45-APCCy7 (clone 30-F11), anti-CD45R/B220-PE (clone RA3-6B2), anti-Gr-1-PE (clone RB6-8C5), anti-TCRα*β* PE (clone H57-597), anti-TCRγδ PE (clone GL3), anti-CD11b PE (clone M1/70) and anti-Ter-119 PE (clone TER-119). In some embodiments, the antibody, fragment, or derivative thereof is directly labeled with a fluorescent label and cells that bind to the antibody are separated by fluorescence-activated cell sorting. Additional detection strategies are known to the skilled artisan.

While FACS scanning is a convenient method for purifying subpopulations of cells, it is understood that other methods can also be employed. An exemplary method that can be used is to employ antibodies that specifically bind to one or more of CD45, CXCR4, CD34, AC133, Sca-1, CD45R/B220, Gr-1, TCRa*β*, TCRγδ, CD11b, and Ter-119, with the antibodies comprising a moiety (*e.g*., biotin) for which a high affinity binding reagent is available (*e.g*., avidin or streptavidin). For example, a biotin moiety could be attached to antibodies for each marker for which the presence on the cell surface is desirable (*e.g*., CD34, Sca-1, CXCR4), and the cell population with bound antibodies could be contacted with an affinity reagent comprising an avidin or streptavidin moiety (*e.g*., a column comprising avidin or streptavidin). Those cells that bound to the column would be recovered and further fractionated as desired. Alternatively, the antibodies that bind to markers present on those cells in the population that are to be removed (*e.g*., CD45R/B220, Gr-1, TCRα*β*, TCRγδ, CD11b, and Ter-119) can be labeled with biotin, and the cells that do not bind to the affinity reagent can be recovered and purified further.

It is also understood that different separation techniques (*e.g*., affinity purification and FACS) can be employed together at one or more steps of the purification process.

A population of cells containing the CD34⁺/CXCR4⁺/lin⁻/CD45⁻ or Sca-1⁺/lin⁻/CD45⁻ cells of the presently disclosed subject matter can be isolated from any subject or from any source within a subject that contains them. In some embodiments, the population of cells comprises a bone marrow sample, a cord blood sample, or a peripheral blood sample. In some embodiments, the population of cells is isolated from peripheral blood of a subject subsequent to treating the subject with an amount of a mobilizing agent sufficient to mobilize the CD45⁻ stem cells from bone marrow into the peripheral blood of the subject. As used herein, the phrase "mobilizing agent" refers to a compound (*e.g*., a peptide, polypeptide, small molecule, or other agent) that when administered to a subject results in the mobilization of a VSEL stem cell or a derivative thereof from the bone marrow of the subject to the peripheral blood. Stated another way, administration of a mobilizing agent to a subject results in the presence in the subject's peripheral blood of an increased number of VSEL stem cells and/or VSEL stem cell derivatives than were present therein immediately prior to the administration of the mobilizing agent. It is understood, however, that the effect of the mobilizing agent need not be instantaneous, and typically involves a lag time during which the mobilizing agent acts on a tissue or cell type in the subject in order to produce its effect. In some embodiments, the mobilizing agent comprises at least one of granulocyte-colony stimulating factor (G-CSF) and a CXCR4 antagonist (*e.g*., a T140 peptide; Tamamura et al. (1998) 253 Biochem Biophys Res Comm 877-882).

In some embodiments, a VSEL stem cell or derivative thereof also expresses a marker selected from the group including but not limited to c-met, c-kit, LIF-R, and combinations thereof. In some embodiments, the disclosed isolation methods further comprise isolating those cells that are c-met⁺, c-kit⁺, and/or LIF-R⁺.

In some embodiments, the VSEL stem cell or derivative thereof also expresses SSEA-1, Oct-4, Rev-1, and Nanog, and in some embodiments, the disclosed isolation methods further comprise isolating those cells that express these genes.

The presently disclosed subject matter also provides a population of CD45⁻ stem cells isolated by the presently disclosed methods.

### III.B. Further Fractionation of the CD45⁻ Stem Cell Population

Disclosed herein is the isolation and/or purification of subpopulations of CD34⁺/CXCR4⁺/lin⁻/CD45⁻ or Sca-1⁺/lin⁻/CD45⁻ cells. These cells comprise a heterogeneous population of cells comprising pluripotent and tissue-committed stem cells. Also disclosed herein are strategies that can be employed for purifying the disclosed subpopulations.

In some embodiments, the heterogeneous subpopulation is further fractionated to enrich for VSEL stem cells of certain lineages. As disclosed herein, the CD34⁺/CXCR4⁺/lin⁻/CD45⁻ or Sca-1⁺/lin⁻/CD45⁻ subpopulations comprise VSEL stem cells for various tissues including, but not limited to neural cells, skeletal muscle cells, cardiac cells, liver cells, intestinal epithelium cells, pancreas cells, endothelium cells, epidermis cells, and melanocytes. These cells can be further fractionated by purifying from the CD34⁺/CXCR4⁺/lin⁻/CD45⁻ or Sca-1⁺/lin⁻/CD45⁻ subpopulations those cells that express one or more markers associated with these lineages. For example, VSEL stem cells for neural tissue can be fractionated using reagents that detect the expression of glial fibrillary acidic protein (GFAP), nestin, *β* III tubulin, oligodendrocyte transcription factor 1 (Olig1), and/or oligodendrocyte transcription factor 2 (Olig2). Similarly, VSEL stem cells for skeletal muscle can be fractionated using reagents that detect the expression of Myf5, MyoD, and/or myogenin. Additional VSEL stem cell types and markers that can be employed include, but are not limited to cardiomyocyte VSEL stem cells (Nsx2.5/Csx, GATA-4), liver cell VSEL stem cells (*α*-Fetoprotein, CK19), intestinal epithelium VSEL stem cells (Nkx 2-3, Tcf4), pancreas cell TCSCs (Nkx 6.1, Pdx 1, C-peptide), endothelial cell VSEL stem cells (VE-cadherin), epidermal cell VSEL stem cells (Krt 2-5, Krt 2-6a, BNC), and melanocyte VSEL stem cells (DCT, TYR, TRP).

### IV. Methods of Differentiating VSEL Stem Cells

### IV.A. Generation of Embryoid Body- (EB) like Spheres

The presently disclosed subject matter also provides a method of differentiating VSEL stem cells. In some embodiments, the methods comprise first generating an embryoid body-like sphere. As used herein, the phrases "embryoid body-like sphere" and "embryoid body-like" refer to an aggregate of cells that appears morphologically similar to an embryoid body formed by ES cells under appropriate *in vitro* culturing conditions. As used herein, the phrase is used interchangeably with "embryoid body" to refer to such aggregates when the cells that make up the embryoid body are CD34⁺/CXCR4⁺/lin⁻/CD45⁻ or Sca-1⁺/lin⁻/CD45⁻ cells isolated and/or purified using the presently disclosed techniques. Methods of generating EBs from ES cells are known in the art (see *e.g*., Martin & Evans (1975) in Teratomas and Differentiation (M. I. Sherman & D. Solter, Eds.), pp. 169-187, Academic Press, New York, New York, United States of America; Doetschman et al. (1985) 87 J Embryol Exp Morphol 27-45). Disclosed herein are methods to prepare EB-like spheres from other multipotent and pluripotent cells, including the CD34⁺/CXCR4⁺/lin⁻/CD45⁻ or Sca-1⁺/lin⁻/CD45⁻ cells.

In some embodiments, a method of forming an embryoid-like body from a population of very small embryonic-like (VSEL) stem cells or derivatives thereof comprises (a) providing a population of CD45⁻ cells comprising VSEL stem cells or derivatives thereof; and (b) culturing the VSEL stem cells or derivatives thereof *in vitro* in a medium comprising one or more factors that induce embryoid-like body formation of the VSEL stem cells or derivatives thereof for a time sufficient for an embryoid-like body to appear.

As used herein, the term "one or more factors that induce embryoid-like body formation of the VSEL stem cells or derivatives thereof" refers to a biomolecule or plurality of biomolecules that when in contact with a plurality of VSEL stem cells or derivatives thereof induces the VSEL stem cells or derivatives thereof to form one or more embryoid body (EB-like)-like spheres. In some embodiments, the one or more factors that induce embryoid body-like formation of the VSEL stem cells or derivatives thereof comprise epidermal growth factor (EGF), fibroblast growth factor-2, and combinations thereof. In some embodiments, the one or more factors are provided to the VSEL stem cells or derivatives thereof by co-culturing the VSEL stem cells or derivatives thereof with C2C12 cells.

### IV.B. Methods of Differentiating VSEL Stem Cells and Derivatives Thereof

Once EB-like spheres are generated, the cells therein can be differentiated *in vitro* by culturing the cells under appropriate conditions. In some embodiments, a method of differentiating a very small embryonic-like (VSEL) stem cell or derivative thereof into a cell type of interest *in vitro* comprises (a) providing an embryoid body-like comprising VSEL stem cells or derivatives thereof; and (b) culturing the embryoid body-like in a culture medium comprising a differentiation-inducing amount of one or more factors that induce differentiation of the VSEL stem cells or derivatives thereof into the cell type of interest until the cell type of interest appears in the *in vitro* culture.

As used herein, the phrase "differentiation-inducing amount" refers to an amount of a growth factor or other activator that when present within an *in vitro* differentiation medium, causes a VSEL stem cell or derivative thereof to differentiate into a cell type of interest. In some embodiments, the growth factor or other activator includes, but is not limited to epidermal growth factor (EGF), fibroblast growth factor-2 (FGF-2), nerve growth factor (NGF), basic fibroblast growth factor (bFGF), vascular endothelial growth factor (VEGF), transforming growth factor *β*1 (TGFβ1), and combinations thereof, and/or other supplements including, but not limited to N2 supplement-A, B27 supplement, and nicotinamide (available from Stem Cell Technologies Inc., Vancouver, British Columbia, Canada). *See also* Fraichard et al. (1995) 108 J Cell Sci 3181-3188.

The choice of growth factors and/or other supplements can depend on the cell type desired into which the EB-like spheres are to differentiate. In some embodiments, the EB-like spheres can be differentiated into neuronal derivatives including, but not limited to neurons, oligodendrocytes, astrocytes, and glial cells. As disclosed in EXAMPLE 22, EB-like spheres can be differentiated into neuronal derivatives by culturing them in medium comprising NEUROCULT® Basal Medium (Stem Cell Technologies Inc., Vancouver, British Columbia, Canada) supplemented with rhEGF, FGF-2, and NGF. EB-like spheres can be differentiated into endodermal derivatives by culturing them in medium comprising Activin A (see EXAMPLE 23). Also, EB-like spheres can be differentiated into cardiomyocytes by culturing them in medium comprising bFGF, VEGF, and TGFβ1 (*see* EXAMPLE 24).

Other cell types that can be generated *in vitro* from stem cells such as ES cells include, but are not limited to hepatocytes (Yamada et al. (2002) 20 Stem Cells 146-154), hematopoietic cells, and pancreatic cells. V. Methods and Compositions for Treatment using VSEL Stem Cells

### V.A. Subjects

The present invention provides a composition for use in a method for treating an injury to a tissue or organ in a subject, the method comprising administering to the subject a pharmaceutical composition, wherein the pharmaceutical composition comprises a plurality of isolated CD45⁻ stem cells comprising VSEL stem cells in a pharmaceutically acceptable carrier, in an amount and via a route sufficient to allow at least a fraction of the population of CD45⁻ stem cells comprising VSEL stem cells to engraft the tissue and differentiate therein, whereby the injury is treated, wherein the injury is a myocardial infarction. numbers counted by FACS analysis (FACSCAN™, Becton Dickinson) as described in Ratajczak et al. (2004a) 103 Blood 2071-2078 and Majka et al. (2001) 97 Blood 3075-3085.

### EXAMPLE 5

### Transmission Electron Microscopy (TEM) Analysis

For transmission electron microscopy, the Sca-1⁺/lin⁻/CD45⁻ and Sca-1⁺/lin⁻/CD45⁺ cells were fixed in 3% glutaraldehyde in 0.1M cacodylate buffer pH 7.4 for 10 hours at 4°C, post-fixed in osmium tetride, and dehydrated. Fixed cells were subsequently embedded in LX112 and sectioned at 800A, stained with uranyl acetate and lead citrate and viewed on a Philips CM10 electron microscope operating at 60 kV.

### EXAMPLE 6

### Reverse Transcription-Polymerase Chain Reaction (RT-PCR)

Total RNA was isolated using RNEASY® Mini Kit (Qiagen Inc., Valencia, California, United States of America). mRNA (10 ng) was reverse-transcribed with One Step RT-PCR (Qiagen Inc.) according to the instructions of the manufacturer. The resulting cDNA fragments were amplified using HOTSTARTAQ® DNA Polymerase (Qiagen Inc., Valencia, California, United States of America). The primers employed were for CXCR4 (GENBANK® Accession No. BC031665), forward primer 5'-GACGGACAAGTACCGGCTGC-3' (SEQ ID NO: 59) and reverse primer 5'- GACAGCTTAGAGATGATGAT-3' (SEQ ID NO: 60); for Met receptor (GENBANK® Accession No. NM_008591), forward primer 5'-CGCGTCGACTTATTCATGG-3' (SEQ ID NO: 61) and reverse primer 5'-CACACATTGATTGTGGCACC-3' (SEQ ID NO: 62); and for LIF-R (GENBANK® Accession No. NM_013584), forward primer 5'-GAGCATCCTTTGCTATCGGAAGC-3' (SEQ ID NO: 63) and reverse primer 5'-CGTTATTTCCTCCTCGATGATGG-3' (SEQ ID NO: 64). The correct sizes of the PCR products obtained were confirmed by separation on agarose gel.

### EXAMPLE 7

### Real Time Quantitative RT-PCR (RQ-PCR)

For analysis of Oct4, Nanog, Rex1, Dppa3, Rif1, Myf5, MyoD, Myogenin, GFAP, nestin, *β* III tubulin, Olig1, Olig2, α-fetoprotein, CK19, Nsx2.5/Csx, GATA-4, VE-cadherin, DCT, TYR, TRP, Nkx 2-3, Tcf4, Krt 2-5, Krt 2-6a, BNC, Nkx 6.1 and Pdx1 mRNA levels, total mRNA was isolated from cells with the RNEASY® Mini Kit (Qiagen Inc., Valencia, California, United States of America). mRNA was reverse-transcribed with TAQMAN® Reverse Transcription Reagents (Applied Biosystems, Foster City, California, United States of America). Detection of Oct4, Nanog, Rex1, Dppa3, Rif1, Myf5, MyoD, Myogenin, GFAP, nestin, *β* III tubulin, Olig1, Olig2, α-fetoprotein, CK19, Nsx2.5/Csx, GATA-4, VE-cadherin, DCT, TYR, TRP, Nkx 2-3, Tcf4, Krt 2-5, Krt 2-6a, BNC, Nkx 6.1 and Pdx1 and R2-microglobulin mRNA levels was performed by real-time RT-PCR using an ABI PRISM® 7000 Sequence Detection System (ABI, Foster City, California, United States of America) employing the primers disclosed in Table 1. A 25 µl reaction mixture contains 12.5 µl SYBR® Green PCR Master Mix, 10 ng of cDNA template, and forward and reverse primers. Primers were designed with PRIMER EXPRESS® software (Applied Biosystems, Foster City, California, United States of America)and are listed in Table 1.

The threshold cycle (Ct), *i.e*., the cycle number at which the amount of amplified gene of interest reached a fixed threshold, was determined subsequently. Relative quantitation of Oct4, Nanog, Rex1, Dppa3, Rif1, Myf5, MyoD, Myogenin, GFAP, nestin, *β* III tubulin, Olig1, Olig2, α-fetoprotein, CK19, Nsx2.5/Csx, GATA-4, VE-cadherin, DCT, TYR, TRP, Nkx 2-3, Tcf4, Krt 2-5, Krt 2-6a, BNC, Nkx 6.1 and Pdx1 mRNA expression was calculated with the comparative Ct method. The relative quantitation value of target, normalized to an endogenous control *β*2-microglobulin gene and relative to a calibrator, is expressed as 2^{-ΔΔCt} (fold difference), where ΔCt = Ct of target genes (Myf5 of Oct4, Nanog, Rex1, Dppa3, Rif1, Myf5, MyoD, Myogenin, GFAP, nestin, *β* III tubulin, Olig1, Olig2, α-fetoprotein, CK19, Nsx2.5/Csx, GATA-4, VE-cadherin, DCT, TYR, TRP, Nkx 2-3, Tcf4, Krt 2-5, Krt 2-6a, BNC, Nkx 6.1, Pdx1) - Ct of endogenous control gene (*β*2-microglobulin), and ΔΔCt = ΔCt of samples for target gene-ΔCt of calibrator for the target gene.

To avoid the possibility of amplifying contaminating DNA i) all the primers for real time RTR-PCR were designed with an intron sequence inside cDNA to be amplified, ii) reactions were performed with appropriate negative controls (template-free controls), iii) a uniform amplification of the products was rechecked by analyzing the melting curves of the amplified products (dissociation graphs), iv) the melting temperature (Tₘ) was 57-60°C, the probe Tₘ was at least 10°C higher than primer Tₘ, and finally, v) gel electrophoresis was performed to confirm the correct size of the amplification and the absence of unspecific bands.

The results of representative analyses are presented in Tables 3 and 4.

**Table 3**

| RQ-PCR Analysis of VSEL Stem Cell Markers* | | | |
|---|---|---|---|
| | VSEL Stem Cell Markers | Sca-1⁺/lin⁻/CD45⁺ | Sca-1⁺/lin⁻/CD45⁻ |
| ***neural*** | GFAP | 0.64 ± 0.03 | 243.41 ± 31.03* |
| | Nestin | 0.39 ± 0.06 | 128.31 ± 18.74* |
| | *β* III tubulin | 0.95 ± 0.09 | 201.36 ± 36.38* |
| | Olig1 | 1.02 ± 0.42 | 36.17 ± 7.14* |
| | Olig2 | 1.14 ± 0.47 | 15.20 ± 2.13* |
| ***skeletal muscle*** | Myf5 | 1.41 ± 0.29 | 179.76 ± 16.78* |
| | MyoD | 0.77 ± 0.50 | 151.78 ± 15.56* |
| | Myogenin | 0.76 ± 0.45 | 76.73 ± 3.21* |
| ***cardiac*** | Nsx2.5/Csx | 2.05 ± 0.12 | 98.63 ± 7.93* |
| | GATA-4 | 2.74 ± 0.41 | 268.63 ± 31.42* |
| ***liver*** | α-Fetoprotein | 0.57 ± 0.02 | 45.93 ± 3.83* |
| | CK19 | 1.12 ± 0.75 | 60.08 ± 9.01* |
| ***intestinal epithelium*** | Nkx 2-3 | 0.30 ± 0.01 | 0.26 ± 0.02 |
| | Tcf4 | 130.03 ± 10.27* | 33.74 ± 4.27* |
| ***pancreas*** | Nkx 6.1 | 0.68 ± 0.08 | 0.76 ± 0.06 |
| | Pdx 1 | 13.71 ± 2.65* | 8.41 ± 1.90* |
| ***endothelium*** | VE-cadherin | 1.05 ± 0.38 | 142.36 ± 12.49* |
| ***epidermis*** | Krt 2-5 | 1.25 ± 0.05 | 62.31 ± 6.81* |
| | Krt 2-6a | 0.69 ± 0.11 | 33.24 ± 3.24* |
| | BNC | 1.49 ± 0.41 | 57.53 ± 2.29* |
| ***melanocyte*** | DCT | 0.42 ± 0.02 | 6.49 ± 1.94* |
| | TYR | 0.38 ± 0.01 | 8.04 ± 1.08* |
| | TRP | 1.08 ± 0.20 | 13.95 ± 2.16* |

| | | | |
|---|---|---|---|
| • Data are expressed as fold increase in expression (mean +/- SD) as compared to expression in input BMMNC. (n = 3 independent sorts, BM from 12 donors pooled for each sort). *p < 0.00001. | | | |

**Table 4**

| RQ-PCR Analysis of PSC Markers* | | |
|---|---|---|
| PSC markers | Sca-1⁺/lin⁻/CD45⁺ | Sca-1⁺/lin⁻/CD45⁻ |
| Oct4 | 0.85 ± 0.01 | 174.49 ± 12.43* |
| Nanog | 0.51 ± 0.02 | 145.14 ± 29.36* |
| Rex1 | 0.96 ± 0.07 | 140.91 ± 16.68* |
| Dppa3 | 0.24 ± 0.03 | 39.25 ± 4.49* |
| Rif1 | 15.17 ± 0.45 | 66.04 ± 7.83* |

| | | |
|---|---|---|
| • Data are expressed as fold increase in expression (means +/- SD) as compared to expression in input BMMNC. (n= 3 independent sorts, BM from 12 donors pooled for each sort). *p < 0.00001 • | | |

### EXAMPLE 8

### Fluorescence Staining of the Sorted Cells

The expression of each antigen was examined in cells from four independent sorts. The Sca-1⁺/lin⁻/CD45⁻ cells were fixed in 3.5% paraformaldehyde for 20 min, permeabilized by 0.1% Triton X100, washed in PBS, pre-blocked with 2% BSA and subsequently stained with antibodies to CXCR4 (1:100, rabbit polyclonal IgG; Santa Cruz Biotechnology, Santa Cruz, California, United States of America), Met (1:100, rabbit polyclonal IgG; Santa Cruz Biotechnology, Santa Cruz, California, United States of America), LIF Receptor gp190 (1:200, mouse monoclonal IgG; BD Biosciences), Oct4 (1:200, mouse monoclonal IgG; Chemicon Int., Temecula, California, United States of America), SSEA-1 (1:200, mouse monoclonal IgM; Chemicon Intl., Temecula, California, United States of America), and Nanog (1:200, goat polyclonal IgG; Santa Cruz Biotechnology, Santa Cruz, California, United States of America). Appropriate secondary Alexa Fluor 488 goat anti-rabbit IgG, Alexa Fluor 594 goat anti-mouse IgG, Alexa Fluor 594 goat anti-mouse IgG, Alexa Fluor 488 goat anti-mouse IgM and Alexa Fluor 594 rabbit anti-goat IgG were used (1:400; Molecular Probes, Eugene, Oregon, United States of America).

In control experiments, cells were stained with secondary antibodies only. The nuclei were labeled with DAPI (Molecular Probes, Eugene, Oregon, United States of America). The fluorescence images were collected with the TE-FM Epi-Fluorescence system attached to a Nikon Inverted Microscope Eclipse TE300 and captured by a COOLSNAP™ HQ digital B/W CCD (Roper Scientific, Tucson, Arizona, United States of America) camera.

### EXAMPLE 9

### Hematopoietic Assays

For cell proliferation assays, murine or human sorted BMMNCs were plated in serum-free methylcellulose cultures in the presence of granulocyte macrophage colony stimulating factor (GM-CSF) + interleukin (IL)-3 for colony-forming unit-granulocyte macrophage (CFU-GM) colonies, erythropoietin (EPO) + stem cell factor (SCF) for burst forming unit-erythroid (BFU-E) colonies, and thrombopoietin (TPO) for CFU-megakaryocytic colonies as described in Ratajczak et al. (2004a) 103 Blood 2071-2078 and Majka et al. (2001) 97 Blood 3075-3085. Using an inverted microscope, murine hematopoietic colonies were scored on day 7 and human hematopoietic colonies on day 12.

For the CFU-S assays, female Balb/C mice (4-6 weeks old) were irradiated with a lethal dose of *γ*-irradiation (900 cGy). After 24 hours, the mice were transplanted with 1 x 10⁴ sorted BMMNCs obtained from syngeneic mice via tail-vein injection. On day 12, spleens were removed and fixed in Tellysyniczky's fixative and CFU-Spleen colonies were counted on the surface of the spleen using a magnifying glass as described in Ratajczak et al. (2004a) 103 Blood 2071-2078.

For long term repopulation assays, C57BL/6 (Ly 5.2) mice were irradiated with a lethal dose of γ-irradiation (900 cGy). After 24 hours, the mice were transplanted (by tail vein injection) with 10⁶ of BMMNC isolated from C57BL/6 (Ly5.2) along with 2x104 of Sca-1⁺/lin⁻/CD45⁻ cells or 2x10⁴ of Sca-1⁺/lin⁻/CD45⁺ from C57BL/6 (Ly5.1) mice. Transplanted mice were bled at various intervals from the retro-orbital plexus to obtain samples for Ly5 phenotyping. Final analysis of donor-derived chimerism was evaluated 8 months after transplantation.

### EXAMPLE 10

### Chimerism Assay

Samples of PBMNC and BMMNC were analyzed on a FACSVANTAGE™ (Becton Dickinson, Mountain View, California, United States of America). Cells were stained with FITC-conjugated anti-CD45.2 (clone 104) and PE-conjugated anti-CD45.1 (clone A20). The percentage of donor engraftment was calculated from two separate measurements (Ly5.1-positive and Ly5.2-negative) after subtraction of background.

### EXAMPLE 11

### In vitro Migration Assay to "MATRIGEL® drop"

To investigate cell migration, briefly the SDF-1 at the concentration of 200 ng/ml or HGF/SF (10 ng/ml) or LIF (100 ng/ml) were dissolved in a Growth Factor Reduced MATRIGEL® Matrix (BD Bioscience, Bedford, Massachusetts, United States of America) at 4°C. As a control the Growth Factor Reduced MATRIGEL® Matrix without chemoattractant was used. The drop of MATRIGEL® was transferred onto a glass bottom well (Willco Wells BV, Amsterdam, The Netherlands) and incubated at 37°C for 30 minutes to polymerize. Subsequently, the Sca-1⁺/lin⁻/CD45⁻ cells were resuspended in DMEM with 0.5% BSA were added at a density of 2 x 10³ per well.

The plates were incubated at 37°C, 95% humidity, 5% CO₂ for 12 hours. Then Sca-1⁺/lin⁻/CD45⁻ cells were stained with a Hoechst 33342 (Sigma Aldrich, St. Louis, Missouri, United States of America) and the number of cells migrating to an SDF-1 gradient was quantified by counting the number of cells visible/100 µm of MATRIGEL® drop circumference using a TE-FM Epi-Fluorescence system attached to a Nikon Inverted Microscope Eclipse TE300 and captured by a cool snap HQ digital B/W CCD (Roper Scientific, Tucson, Arizona, United States of America) camera.

### EXAMPLE 12

### Statistical Analysis

Arithmetic means and standard deviations of our FACS data were calculated on a Macintosh computer PowerBase 180, using Instat 1.14 (GraphPad, San Diego, California, United States of America) software. Data were analyzed using the Student t-test for unpaired samples or ANOVA for multiple comparisons. Statistical significance was defined as p < 0.05.

### EXAMPLE 13

### Bone Marrow-derived Sca-1⁺/lin⁻/CD45⁻ cells Resemble Undifferentiated Embryonic Stem Cells

The instant co-inventors demonstrated previously that BM contains a population of hematopoietic Sca-1⁺/lin⁻/CD45⁺ and a population of non-hematopoietic Sca-1⁺/lin⁻/CD45⁻ stem cells (Kucia et al. (2005b) 19 Leukemia 1118-1127), and that the latter cells are highly enriched in mRNA for markers of early VSEL stem cells. *See* Kucia et al. (2005b) 33 Exp Hematol 613-623 and Kucia et al. (2004b) Circ Res 1191-1199. Disclosed herein is an evaluation of the morphology of these rare cells by employing transmission electron microscopy (TEM).

As shown in Figures 1A and 1B, Sca-1⁺/lin⁻/CD45⁻ (Figure 1A) cells as compared to Sca-1⁺/lin⁻/CD45⁺ (Figure 1B) cells are smaller in size (3-4 µm vs. 8-10 µm), contain relatively large nuclei, and have a narrow rim of cytoplasm. Additionally, DNA in the nuclei of these small Sca-1⁺/lin⁻/CD45⁻ cells contain open-type euchromatin that is characteristic of pluripotent embryonic stem cells (see Figure 1A). Thus, disclosed herein for the first time is morphological evidence for the presence of embryonic-like cells in adult BM.

### EXAMPLE 14

### Bone Marrow-derived Sca-1⁺/lin⁻/CD45⁻ Cells Express Several Pluripotent Stem Cell (PSC) Markers

Sca-1⁺/lin⁻/CD45⁻ cells express mRNA typical for VSEL stem cells. As disclosed herein, an expanded panel of genes for several markers of VSEL stem cells for neural tissue, skeletal and heart muscle, liver, pancreas, epidermis, melanocytes and intestinal epithelium (*see* Table 3) was evaluated.

Interestingly it was determined that these cells also express mRNA typically associated with PSC, including Oct-4, Nanog, Rex1, and Dppa3, and are enriched in mRNA for telomerase protein Rif1 (see Table 4). Additionally, the instant disclosure provides evidence that purified Sca-1⁺/lin⁻/CD45⁻ cells express several embryonic stem cell markers, including SSEA-1, Oct-4, and Nanog, at the protein level (*see* Figure 2). As depicted therein, SSEA-1, Oct-4, and Nanog were detectable on 57 ± 7%, 43 ± 6%, and 28 ± 4% of Sca-1⁺/lin⁻/CD45⁻ cells, respectively, demonstrating that PSC proteins are expressed in a freshly isolated defined population of cells from the BM.

### EXAMPLE 15

### Bone Marrow-derived Sca-1⁺/lin⁻/CD45⁻ Express CXCR4, c-met, and LIF-R

Previous studies have demonstrated that BM-derived cells that express markers of VSEL stem cells could be isolated from a suspension of BMMNC by employing chemotaxis to stromal derived factor-1 (SDF-1), hepatocyte growth factor/scatter factor, (HGF/SF) or leukemia inhibitory factor (LIF) gradients (Ratajczak et al. (2004b) 18 Leukemia 29-40). As disclosed herein, the corresponding population of Sca-1⁺/lin⁻/CD45⁻ cells purified by FACS were examined for expression of the corresponding receptors (CXCR4, c-met, and LIF-R). Figure 3A shows that Sca-1⁺/lin⁻/CD45⁻ cells sorted by FACS express mRNA for all of these receptors. Additionally, as shown in Figure 3B, expression of these receptors was also confirmed by immunostaining. These receptors were present on 82 ± 6% (CXCR4), 61 ± 8% (c-met), and 43 ± 5% (LIF-R) of purified Sca-1⁺/lin⁻/CD45⁻ cells. Furthermore, in direct chemotactic studies, it was determined that these highly purified cells respond robustly to SDF-1 (*see* Figure 3C), HGF/SF, and LIF gradients.

### EXAMPLE 16

### Sca-1⁺/lin⁻/CD45⁻ Cells are Enriched in BM from Young Mice.

Previous RQ-PCR data generated by the co-inventors suggested that BM from young mice contains more PSC and/or VSEL stem cells than does BM from older mice (Kucia et al. (2005b) Leukemia 1118-1127). As shown in Figure 4, by employing FACS analysis of BMMNC derived from 1 month old and 1 year old mice, it has been determined that the number of Sca-1⁺/lin⁻/CD45⁻ cells is reduced by about 6-10 times in BM from older animals (Figure 4A, lower panel). Furthermore, the FACS analysis disclosed herein corresponded with a significant decrease of mRNA expression for PSC and VSEL stem cell markers in BMMNC isolated from older animals as evaluated by RQ-PCR (Figure 4B).

### EXAMPLE 17

### Sca-1⁺/lin⁻/CD45⁻ Cells are Decreased in Short Living DBA/2J Mice

Also disclosed herein is the discovery that the number of Sca-1⁺/lin⁻/CD45⁻ cells varies with murine strain. In particular, it is shown that the number of these cells is reduced in short living DBA/2J mice as compared to long living C57BL/6 mice. The data presented in Figure 5 demonstrated that in fact mRNA for several PSC/VSEL stem cells is significantly lower in mRNA isolated from BMMNC from 3 weeks old DBA/2J mice.

### EXAMPLE 18

### Sca-1⁺/lin⁻/CD45⁻ Cells are Present in the Side Population of BM Cells

It is known that the side population (SP) of BMMNC is highly enriched in stem cells (*see e.g*., Goodell et al. (1996) 183 J Exp Med 1797-1806; Jackson et al. (2001) 107 J Clin Invest 1395-1402; Macpherson et al. 118 J Cell Sci 2441-2450). To address whether the embryonic-like stem cells identified by the techniques disclosed herein are present in SP of BMMNC, a side population of BMMNC was isolated from BM (see Figure 6A). For comparison, Sca-1⁺/lin⁻/CD45⁻ cells were isolated from the same marrow samples (see Figure 4A).

Subsequently, both Sca-1⁺/lin⁻/CD45⁺ (SP Sca-1⁺/lin⁻/CD45⁺) and Sca-1⁺/lin⁻/CD45⁻ (SP Sca-1⁺/lin⁻/CD45⁻) cells were isolated from SP BMMNC. All of these populations of cells were compared along with unpurified BMMNC for expression of mRNA for early PSC/VSEL stem cells. As shown in Figure 6B, SP is highly enriched in mRNA for markers of PSC/VSEL stem cells. However, calculations of the total yield of Sca-1⁺/lin⁻/CD45⁻ cells isolated from the same number of BMMNC revealed that the number of Sca-1⁺/lin⁻/CD45⁻ cells resorted from SP was about two orders of magnitude lower when compared to direct sorting of these cells from the lymph gate of BMMNC. Additionally, SP cells depleted from a population of Sca-1⁺/lin⁻/CD45⁻ did not express mRNA for PSC/VSEL stem cells, which suggests that the SP Sca-1⁺/lin⁻/CD45⁻ cells probably account for the pluripotency of SP cells.

### EXAMPLE 19

### Sca-1⁺/lin⁻/CD45⁻ Cells in Contrast to Sca-1⁺/lin⁻/CD45⁺ Cells are Not Hematopoietic

Several assays were employed to evaluate if embryonic like-cells isolated from BM possess hematopoietic potential. First, it was determined if these cells are able to grow *in vitro* hematopoietic colonies, but no clonogenic activity of these cells was detected. Next, Sca-1⁺/lin⁻/CD45⁻ in contrast to Sca-1⁺/lin⁻/CD45⁺ cells did not radioprotect lethally irradiated mice or form CFU-S colonies in lethally irradiated syngeneic recipients.

To address if these cells were be enriched for some long term hematopoietic repopulating stem cells, the contribution of these cells to long-term repopulation of the hematopoietic system after transplantation to lethally irradiated mice was studied by employing donor/recipient animals congenic at the Ly.5 locus. Transplantation of 10⁴ Sca-1⁺/lin⁻/CD45⁺ cells from Ly5.1 mice along with 10⁶ BMMNC of Ly5.2 cells into Ly5.2 recipient mice resulted in about 17 ± 3% chimerism of mice (n = 6) as evaluated 8 months after transplantation. No contribution of donor cells to hematopoiesis was observed in similar experiments after transplantation of 2x10⁴ Sca-1⁺/lin⁻/CD45⁻ cells co-transplanted with 10⁶ BMMNC (see Figure 7). Similar results were obtained in similar experiments after transplantation of green immunofluorescence positive (GFP+) Sca-1⁺/lin⁻/CD45⁻ and Sca-1⁺/lin⁻/CD45⁺ cells into lethally irradiated syngeneic recipients.

### Discussion of EXAMPLES 1-19

Contribution of BM-derived cells to organ regeneration has been explained by some investigators to involve the phenomenon of trans-dedifferentiation of HSC. However, the co-inventors have determined that BM contains a population of rare Sca-1⁺/lin⁻/CD45⁻ cells that express several markers of non-hematopoietic stem cells and are able to differentiate *in vitro* cultures into mesoderm-derived cardiomyocytes and ectoderm-derived neural cells. These cells have been referred to as very small embryonic-like (VSEL) stem cells. It is possible that VSEL stem cells circulate during organogenesis and rapid body growth/expansion. Since VSEL stem cells respond to an SDF-1 gradient, the SDF-1-CXCR4 axis alone or in combination with other chemoattractants might play a crucial role in accumulation of these cells in young BM.

Disclosed herein is that these highly purified BM-derived Sca-1⁺/lin⁻/CD45⁻ cells (∼0.02% of BMMNC) express both at the mRNA and protein level several embryonic stem cell markers, such as surface marker SSEA-1 and transcription factors Oct-4, Nanog, and Rex-1. In direct TEM studies it was observed that these cells are very small (3-4 µm) and show a very immature morphology (*e*.*g*., they posses relatively large nuclei and contain immature open-type euchromatin). The open type of chromatin in these cells correlates with the presence of mRNA not only for embryonic stem cells but also mRNA for several VSEL stem cells, such as those that are competent to differentiate into skeletal muscle, heart muscle, neural, liver, intestinal epithelium, skin epidermis, melanocytes, and endocrine pancreas. Thus, disclosed herein for the first time is the identification at the morphological level a population of embryonic-like cells in adult BM.

Additionally, some of these cells express early developmental markers for neural, cardiac, or skeletal muscles at the protein level, suggesting that despite their similar homogenous morphology these cells show some degree of tissue commitment and are heterogeneous. It is interesting to note that the expression of several potential chemoattractants of stem cells (*e.g*., SDF-1, HGF/SF, and LIF) are upregulated in damaged organs, and hypoxia regulated/induced transcription factor (HIF-1) plays an important role in their expression (Ceradini et al. (2004) 10 Nat Med 858-864; Pennacchietti et al. (2003) 3 Cancer Cell 347-361). To support this notion, promoters of SDF-1, HGF/SF, and LIF, contain several functional HIF-1 binding sites. Thus the SDF-1 - CXCR4, HGF/SF - c-met, and LIF - LIF-R axes might direct trafficking of stem cells.

To support this notion, disclosed herein is the demonstration that highly purified Sca-1⁺/lin⁻/CD45⁻ cells express CXCR4, c-met, and LIF-R at the protein level, and respond robustly by chemotaxis to SDF-1, LIF, and HGF/SF gradients, respectively. This observation is in agreement with the fact that murine embryonic stem cells also express functional CXCR4, c-met, and LIF-R on their surfaces, and SDF-1, HGF/SF, and LIF affect the motility of these cells (Kucia et al. (2005c) 23 Stem Cells 879-894; Guo et al. (2005) 23 Stem Cells 1324-1332).

One might also expect that if a population of Sca-1⁺/lin⁻/CD45⁻ BMMNC is enriched in embryonic-like PSC, these cells should be also able to differentiate along the hematopoietic lineage. However, neither protected lethally irradiated mice nor contributed to long-term hematopoiesis after transplantation into lethally irradiated recipients. Thus, the population of CD45⁻ cells appears to be restricted to heterogeneous non-hematopoietic VSEL stem cell only. However, it is also possible that in the standard assays disclosed herein the potential pluripotency of Sca-1⁺/lin⁻/CD45⁻ BMMNC was not detected and the final answer on their hematopoietic potential is obtained after injection into a developing blastocyst.

The number of embryonic-like stem cells identified is higher in BM in young animals, and their number decreases with age. Furthermore, Sca-1⁺/lin⁻/CD45⁻ cells are barely detectable in 1 year old mice which corresponds to a 50 year old human. This age dependent content of VSEL stem cells in BM might explain why regeneration processes are more efficient in younger individuals. Differences were also noticed in the content of Sca-1⁺/lin⁻/CD45⁻ cells among BMMNC between long and short living mouse strains. The concentration of these cells is much higher in BM of long living (*e*.*g*., C57BL/6) as compared to relatively short living (DBA/2J) mice.

Finally, VSEL stem cells were highly mobile and responded to an SDF-1 gradient and adhered to BM-derived fibroblasts. Time-lapse studies revealed that these cells attach rapidly to, migrate beneath, and/or undergo emperipolesis in these cells. Interaction of VSEL stem cells with BM-derived fibroblasts was efficiently inhibited after their preincubation with CXCR4 antagonist, T140. Since fibroblasts secrete SDF-1 and other chemottractants, they might create a homing environment for these cells. Their robust interaction with BM-derived fibroblasts has an important implication - suggesting that isolated BM stromal cells might be "contaminated" by these small embryonic-like PSC/VSEL stem cells.

It appears that the Sca-1⁺/lin⁻/CD45⁻ cells disclosed herein represent a new subpopulation of BM-derived stem cells. For example, mesenchymal stem cells (MSC) have a morphology similar to that of fibroblastic cells. Hematopoietic cells are CD45⁺. MSC are also CXCR4⁻ and CD34⁻, and have never been identified at the single cell level. Similarly, putative multipotent adult progenitor cells (MAPC) have not been definitively identified at the single cells level, nor have USSC cells or MIAMI cells. The existences of these cells have only been postulated based on observed *in vitro* differentiation of cord blood or marrow cells to different tissues.

Furthermore, the fact that Sca-1⁺/lin⁻/CD45⁻ PSC/VSEL stem cells are very small should be considered, especially in protocols based on gradient or velocity centrifugation employed to isolate stem cells from BM, mPB, and CB. It is very likely that the majority of PSC/VSEL stem cells could be lost during those isolation procedures because of their very small size.

### EXAMPLE 20

### Formation of Embryoid Body-like Spheres

GFP⁺ Sca-1⁺/lin⁻/CD45⁻ (5x10⁴/35 mm glass bottom plate) isolated from BMMNC of C57BL/6-Tg(ACTB-EGFP)1 Osb/J mice (available from The Jackson Laboratory, Bar Harbor, Maine, United States of America) were cultured along with C2C12 cells (1.5x10⁶/35 mm glass bottom plate), which is a subclone of the mouse myoblast cell line commercially available from the American Type Culture Collection (ATCC; Manassas, Virginia, United States of America) in Dulbecco's Modified Eagle's Medium with 4 mM L-glutamine, 4.5 g/l glucose, 5% heat-inactivated FBS, 10 ng/ml rhEGF, 10 ng/ml FGF-2. The growth factors were added to the cultures daily. The medium was exchanged every 72 hours. The embryoid body-like spheres started appearing about 5-7 days after starting the co-cultures.

Cells from VSEL stem cell-derived spheres (VSEL-DS) were stained with propidium iodide and subjected to FACS analysis to assess ploidy of the cells. Three independent examples are shown in Figure 12.

The embryonic bodies were fixed in 3.5% paraformaldehyde for 20 minutes, permeabilized by 0.1% Triton X100, washed in PBS, pre-blocked with 2% BSA and subsequently stained with antibodies to SSEA-1 (1:200, mouse monoclonal IgM; Chemicon Intl., Temecula, California, United States of America; see Figure 8A), or Oct-4 (1:200, mouse monoclonal IgG; Chemicon Intl.; see Figure 8B). Appropriate secondary Alexa Fluor 594 anti-mouse IgM and Alexa Fluor 594 goat anti-mouse IgG were used (1:400; Molecular Probes, Eugene, Oregon, United States of America). In control experiments, cells were stained with secondary antibodies only. The nuclei were labeled with DAPI (Molecular Probes, Eugene, Oregon, United States of America). The Green Fluorescent Protein was visualized by anti-green fluorescent protein Alexa Fluor 488 conjugate (1:400; Molecular Probes, Eugene, Oregon, United States of America; see Figures 9A and 9B). The fluorescence images were collected with the TE-FM Epi-Fluorescence system attached to a Nikon Inverted Microscope Eclipse TE300 and captured by a cool snap HQ digital B/W CCD (Roper Scientific, Tucson, Arizona, United States of America) camera.

### EXAMPLE 21

### Plating of VSEL Stem Cells on C2C12 Cells

Murine C2C12 cells are a primitive myoblastic cells line is employed as a model for myogeneic differentiation. In order to differentiate/expand VSEL stem cells into myogenic lineage, purified by FACS BM-derived Sca-1⁺/lin⁻/CD45⁻ VSEL stem cells were plated over C2C12 cells. 5-10% of plated VSEL stem cells began proliferate and form slightly attached/floating embryoid body-like spheres containing round cells.

In order to rule out the possibility that these embryoid body-like spheres were formed from the C2C12 cells, VSEL stem cells were isolated from GFP⁺ mice and embryoid body-like spheres were formed as in EXAMPLE 20. It was determined that the embryoid body-like spheres were formed by the GFP⁺ VSEL stem cells.

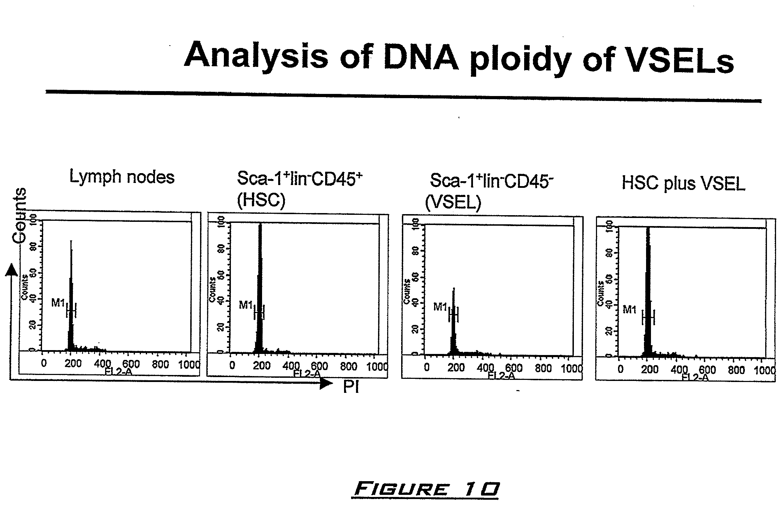

The possibility of fusion between C2C12 cells and VSEL stem cells was excluded by DNA ploidy analysis. Briefly, cells isolated from murine lymph nodes, HSCs (hematopoietic stem cells; Sca-1⁺/lin⁻/CD45⁺) or VSEL stem cells (Sca-1⁺/lin⁻/CD45⁻) were stained with propidium iodide and subjected to FACS analysis. DNA contents per cell were determined by staining cells with propidium iodide and subsequent FACS analysis (*see* Figure 10).

Interestingly, the embryoid body-like spheres expressed embryonic stem cell-specific alkaline phosphatase (*see* Figure 13).

Further characterization of the embryoid body-like spheres revealed that they expressed early embryonic developmental markers such as SSEA-1, GATA-6, GATA-4, FOXD1, and Nanog (*see* Figure 14). Transmission electron microscopy revealed that the cells that were present in the VSEL stem cell-derived embryoid body-like spheres were larger in size than the original VSEL stem cells from which they were derived (Figure 15, upper panel), but still possessed very primitive nuclei containing euchromatin.

Developmental migration of VSEL stem cells can be orchestrated by SDF-1, HGF/SF, and LIF. It was further determined that cells isolated from VSEL stem cell-derived embryoid body-like spheres responded to stimulation by these factors by robust phosphorylation of MAPKp42/44, which suggested that these factors might play a role in their development and migration. It was further determined that the corresponding receptors (CXCR4, c-met, and LIF-R, respectively) were expressed on the surface of the VSEL stem cell-derived embryoid body-like spheres (Figure 15, middle panel).

Furthermore, cells from VSEL stem cell-derived embryoid body-like spheres (VSEL-DS), after replating over C2C12 cells, can again grow new embryoid body-like spheres (up to at least 5-7 additional passages). However, the number and size of these embryoid body-like spheres became smaller with each passage. RT-PCR analysis of cells isolated from the embryoid body-like spheres from consecutive passages revealed an increase in expression of mRNA for genes regulating gastrulation of embryonic bodies, such as GATA-6, Cdx2, Sox2, HNF3, AFP (Figure 15, lower panel).

### EXAMPLE 22

### Neuronal Differentiation of Embryoid Body-like Spheres

To generate neuronal derivatives (neurons, oligodendrocytes, glial cells), 10-50 embryoid body-like spheres/35 mm glass bottom plate were plated in NeuroCult Basal Medium (Stem Cell Technologies, Vancouver, British Columbia, Canada) supplemented with 10 ng/ml rhEGF, 20 ng/ml FGF-2, and 20 ng/ml NGF. Cells were cultured for 10-15 days. Growth factors were added every 24 hours and medium was replaced every 2-3 days.

At day 15 of differentiation, the cells were fixed in 3.5% paraformaldehyde for 20 minutes, permeabilized by 0.1% Triton X100, washed in PBS, pre-blocked with 2% BSA, and subsequently stained with antibodies to *β* III tubulin (1:100, rabbit polyclonal IgG; Santa Cruz Biotechnology, Santa Cruz, California, United States of America), nestin (1:200, mouse monoclonal IgG1; Chemicon Intl., Temecula, California, United States of America), or O4 (1:200, oligodendrocyte marker 4, mouse monoclonal IgM; Chemicon Intl.). Appropriate secondary Alexa Fluor 594 goat anti-rabbit IgG, Alexa Fluor 594 goat anti-mouse IgG, and Alexa Fluor 594 goat anti-mouse IgM were used (1:400, Molecular Probes, Eugene, Oregon, United States of America). In control experiments, cells were stained with secondary antibodies only.

Figures 16A-16C and 17A-17D summarize the staining of oligodendrocytes (Figures 16A-16C) and neurons (Figures 17A-17D) derived from VSEL stem cells. In Figures 16 and 17, the blue color is indicative of DAPI staining of nuclei (Molecular Probes; blue color), nestin staining appears red, and Green Fluorescent Protein (GFP) was visualized by anti-green fluorescent protein Alexa Fluor 488 conjugate (1:400; Molecular Probes, Eugene, Oregon, United States of America). The GFP is present in the isolated cells, which were isolated from GFP⁺ mice (C57BL/6-Tg(ACTbEGFP)1Osb/J mice purchased from The Jackson Laboratory, Bar Harbor, Maine, United States of America). The fluorescence images were collected with the TE-FM Epi-Fluorescence system attached to a Nikon Inverted Microscope Eclipse TE300 and captured by a cool snap HQ digital B/W CCD (Roper Scientific, Tucson, Arizona, United States of America) camera.

### EXAMPLE 23

### Endodermal Differentiation of Embryoid Body-like Spheres

Before initiating differentiation, embryoid body-like spheres were given a brief wash in PBS. 10-50 embryoid body-like spheres per 35 mm glass bottom plate were plated in DMEM/F12 Medium with 4 mM L-glutamine, 4.5 g/l glucose, 1 % heat-inactivated FBS, and 50 ng/ml of recombinant human Activin A. After 48 hours, medium was exchanged and differentiation was carried out in DMEM/F12 Medium with 4 mM L-glutamine, 4.5 g/l glucose, and 5% heat-inactivated FBS in the presence of N2 supplement-A, B27 supplement, and 10 mM nicotinamide (purchased from Stem Cell Technologies Inc., Vancouver, British Columbia, United States of America). Medium was changed every second day. Islet-like clusters appeared after 12-17 days of culture.

After 17 days of differentiation, cells were fixed in 3.5% paraformaldehyde for 20 minutes, permeabilized by 0.1 % Triton X100, washed in PBS, pre-blocked with 2% BSA, and subsequently stained with antibodies to pancreatic C-peptide (1:100; guinea pig IgG, Linco Research, Inc., St. Charles, Missouri, United States of America). Appropriate secondary, Alexa Fluor 594 anti-guinea pig IgG were used (1:400; Molecular Probes, Eugene, Oregon, United States of America). In control experiments, cells were stained with secondary antibodies only.

Figures 18A-18C summarize the staining of endodermal cells derived from VSEL stem cells. In Figures 18A-18C, the blue color is indicative of DAPI staining of nuclei (Molecular Probes, Eugene, Oregon, United States of America; blue color), C-peptide staining appears red, and Green Fluorescent Protein (GFP) was visualized by anti-green fluorescent protein Alexa Fluor 488 conjugate (1:400; Molecular Probes, Eugene, Oregon, United States of America). The GFP is present in the isolated cells, which were isolated from GFP⁺ mice (C57BL/6-Tg(ACTB-EGFP)1Osb/J mice purchased from The Jackson Laboratory, Bar Harbor, Maine, United States of America). The fluorescence images were collected with the TE-FM Epi-Fluorescence system attached to a Nikon Inverted Microscope Eclipse TE300 and captured by a cool snap HQ digital B/W CCD (Roper Scientific, Tucson, Arizona, United States of America) camera.

### EXAMPLE 24

### Cardiomyocyte Differentiation of Embryoid Body-like Spheres

10-50 embryoid body-like spheres/35 mm glass bottom plate were plated in DMEM with 4 mM L-glutamine, 4.5 g/l glucose, 10% heat-inactivated FBS, and 10 ng/ml bFGF, 10 ng/ml VEGF, and 10 ng/ml TGFβ1. Growth factors were added every 24 hours and medium was replaced every 2-3 days. Cardiomyocytes differentiated after about 15-17 days of differentiation.

At day 17 of differentiation, cells were fixed in 3.5% paraformaldehyde for 20 minutes, permeabilized by 0.1% Triton X100, washed in PBS, pre-blocked with 2% BSA, and subsequently stained with antibodies to Troponin I (1:200; mouse monoclonal IgG2b, Chemicon Intl., Temecula, California, United States of America), and α-sarcomeric actinin (1:100; mouse monoclonal IgM, Abcam, Inc., Cambridge, Massachusetts, United States of America). Appropriate secondary Alexa Fluor 594 goat anti-mouse IgG, and Alexa Fluor 594 anti-mouse IgM were used (1:400; Molecular Probes, Eugene, Oregon, United States of America). In control experiments, cells were stained with secondary antibodies only.

Figures 19A-19C and 20A-20D summarize the staining of cardiomyocytes derived from VSEL stem cells. In Figures 19A-19C, the blue color is indicative of DAPI staining of nuclei (Molecular Probes, Eugene, Oregon, United States of America; blue color), troponin I staining appears red, and Green Fluorescent Protein (GFP) was visualized by anti-green fluorescent protein Alexa Fluor 488 conjugate (1:400; Molecular Probes, Eugene, Oregon, United States of America). In Figures 20A-20D, the red color corresponds to staining of *α* sarcomeric actinin. The GFP is present in the isolated cells, which were isolated from GFP⁺ mice (C57BL/6-Tg(ACTB-EGFP)1Osb/J mice purchased from The Jackson Laboratory, Bar Harbor, Maine, United States of America). The fluorescence images were collected with the TE-FM Epi-Fluorescence system attached to a Nikon Inverted Microscope Eclipse TE300 and captured by a cool snap HQ digital B/W CCD (Roper Scientific, Tucson, Arizona, United States of America) camera.

### Discussion of EXAMPLES 21-24

To support the hypothesis that cells present in embryoid body-like spheres growing from single VSEL stem cells are able to differentiate into all three germ layers, cells from single VSEL-DS were plated in differentiating media that support grow of cardiac myocytes, neuronal cells, and pancreatic cells. Both histochemical staining as well as RT-PCR analysis (Figures 21A-21C) revealed that cells from single VSEL stem cell-derived spheres differentiate into cardiomyocytes (mesoderm), neural cells and olgodendrocytes (ectoderm), and pancreatic β-islets (endoderm) insulin-producing cells. These changes in cell morphology and expression of lineage-specific proteins were paralleled by upregulation of tissue-specific genes (*see* Figure 21).

### EXAMPLE 25

### Studies of Myocardial Infarction

Two groups (n = 24/group) of wild-type mice (C57BL/6,129 strain, body wt. 25-35 g, age 12-16 weeks) purchased from Jackson Laboratory were used. The experimental preparation has been described in Guo et al. (1998) 275 Am J Physiol H1375-1387 and Guo et al. (1999) 96 Proc Natl Acad Sci. U S A 11507-11512. Mice were anesthetized with pentobarbital sodium (50 mg/kg i.p.), intubated, and ventilated using a small rodent ventilator. Body temperature, heart rate, and arterial pH were carefully maintained within the physiological range throughout the experiments. Using a sterile technique, the chest was opened through a midline sternotomy. An 8-0 nylon suture was passed with a tapered needle under the left anterior descending coronary artery 2-3 mm from the tip of the left auricle, and a non-traumatic balloon occluder was applied on the artery. Coronary occlusion was induced by inflating the balloon occluder. Mice in group I underwent a 30 minute coronary occlusion followed by reperfusion while mice in group II underwent a sham operation (1 hour open-chest state). See Guo et al. (1998) 275 Am J Physiol H1375-1387 and Guo et al. (1999) 96 Proc Natl Acad Sci. U S A 11507-11512. Mice (n = 6 mice in each group at each time-point) were sacrificed at 6 hours, 24 hours, 48 hours, or 96 hours after the onset of reperfusion.

Following euthanasia, blood samples (1.0-1.5 ml from each mouse) were collected in heparin-rinsed syringes for the isolation of peripheral blood mononuclear cells (PBMNCs). Myocardial tissue samples were harvested from the ischemic and non-ischemic regions and frozen immediately in liquid nitrogen for mRNA extraction.

### EXAMPLE 26

### In vitro Expression of Cardiac Markers

The ability of the bone marrow-derived Sca-1⁺/lin⁻/CD45⁻ MNCs to differentiate into a cardiomyocyte phenotype in culture was tested. Due to the inability of the Sca-1⁺/lin⁻/CD45⁻ cells to survive when cultured alone, Sca-1⁺/lin⁻/CD45⁻ and Sca-1⁺/lin⁻/CD45⁺ BMMNCs were cultured in separate plates along with unpurified bone marrow cells that provide a conducive milieu for cell survival. Twenty-one days later, these cells were immunostained to examine the expression of cardiac-specific myosin heavy chain and cardiac troponin I. Cultured cells in plates to which the Sca-1⁺/lin⁻/CD45⁻ BMMNCs were added (Figures 22A-22C and 22D-22F) exhibited a different phenotype compared with the plates to which the Sca-1⁺/lin⁻/CD45⁺ cells were added. Numerous cells in plates with Sca-1⁺/lin⁻/CD45⁺ cells were positive for cardiac-specific myosin heavy chain (Figures 22B, 22C, 22E, and 22F; green fluorescence). Many of these cardiac-specific myosin heavy chain-positive cells were also positive for cardiac troponin I (Figures 22D and 22F [arrowheads]; red fluorescence).

In contrast, cultured cells in plates to which the Sca-1⁺/lin⁻/CD45⁺ cells were added (Figures 22G-22I) were largely negative for the expression of these cardiac-specific antigens (Figure 22H). In Figures 22A-22I, the nuclei are identified by DAPI (blue fluorescence). These results indicate that Sca-1⁺/lin⁻/CD45⁺ cells are capable of differentiating into a cardiomyocyte phenotype in culture.

### EXAMPLE 27

### Immunohistochemistry

The expression of cardiac-specific markers (GATA-4 and Nkx2.5/Csx) in PSC/VSEL stem cells was verified by immunocytochemistry. Murine control (unpurified) BMMNCs and BMMNCs chemoattracted to SDF-1, HGF, and LIF, or Sca-1⁺/lin⁻/CD45⁺ and Sca-1⁺/lin⁻/CD45⁺ BM-derived cells sorted by FACS were fixed in 1% paraformaldehyde for 30 minutes, permeabilized with 0.5% Triton X-100, and incubated overnight at 4°C with rabbit polyclonal anti-GATA-4 (Santa Cruz) and rabbit polyclonal anti-Nkx2.5/Csx (Santa Cruz Biotechnology, Santa Cruz, California, United States of America) primary antibodies. FITC- and TRITC-labeled secondary antibodies were used for the detection of GATA-4 and Nkx2.5/Csx, respectively. Cells positive for cardiac markers were counted using a confocal microscope (Zeiss LSM 510, Carl Zeiss, Thornwood, New York, United States of America) and expressed as a percentage of total MNCs.

### EXAMPLE 28

### Functional Pluripotent VSEL Stem Cell Numbers Decrease with Age

The number of VSEL stem cells in young versus old mice was also investigated. The yield of Sca-1⁺/lin⁻/CD45⁺ cells that could be sorted by FACS was observed to decrease with age (Figure 23 and 24). It was further determined that VSEL-DS could be formed in co-cultures with C2C12 cells only by VSEL stem cells that were isolated from young mice (Figure 25). Interestingly, VSEL stem cells from 2.5-year old animals formed cells clusters of round cells when co-cultured with C2C12. These round cells expressed the CD45 antigen and were able to grow hematopoietic colonies in secondary cultures in methyllocelulose (Figure 26).

### EXAMPLE 29

### Isolation of VSEL Stem Cells from Cord Blood

*Staining and isolation of Cord Blood (CB) derived VSEL stem cells.* Whole human umbilical CB was lysed in BD lysing buffer (BD Biosciences, San Jose, California, United States of America) for 15 minutes at room temperature and washed twice in PBS. A single cell suspension was stained for various lineage markers (CD2 clone RPA-2.10; CD3 clone UCHT1; CD14 clone M5E2; CD66b clone G10F5; CD24 clone ML5; CD56 clone NCAM16.2; CD16 clone 3G8; CD19 clone HIB19; and CD235a clone GA-R2) conjugated with FITC, CD45 (clone HI30) conjugated with PE, and combination of CXCR4 (clone 12G5), CD34 (clone 581) or CD133 (CD133/1) conjugated with APC, for 30 minutes on ice. After washing, cells were analyzed by FACS (BD Biosciences, San Jose, California, United States of America). At least 10⁶ events were acquired and analyzed by using Cell Quest software.

CXCR4⁺/lin⁻/CD45⁻, CD34⁺/lin⁻/CD45⁻, or CD133⁺/lin⁻/CD45⁻, cells were sorted from a suspension of CB MNC by multiparameter, live sterile cell sorting (MOFLO™, Dako A/S, Fort Collins, Colorado, United States of America, or BD FACSARIA™ Cell-Sorting System, BD Biosciences, San Jose, California, United States of America).

*Transmission electron microscopy (TEM) analysis.* For transmission electron microscopy, the CXCR4⁺/lin⁻/CD45⁻, cells were fixed in 3% glutaraldehyde in 0.1M cacodylate buffer pH 7.4 for 10 hours at 4°C, post-fixed in osmium tetride, and dehydrated. Fixed cells were subsequently embedded in LX112 resin (Ladd Research Industries, Inc., Burlington, Vermont, United States of America) and sectioned at 800A, stained with uranyl acetate and lead citrate, and viewed on a Philips CM10 electron microscope (Philips, Eindhoven, the Netherlands) operating at 60kV.

*RT-PCR.* Total RNA was isolated using the RNEASY® Mini Kit (Qiagen Inc., Valencia, California, United States of America). mRNA (10 ng) was reverse-transcribed with One Step RT-PCR (Qiagen Inc., Valencia, California, United States of America) according to the instructions of the manufacturer. The resulting cDNA fragments were amplified using HOTSTARTAQ® DNA Polymerase (Qiagen Inc., Valencia, California, United States of America). Primer sequences for Oct4 were forward primer: 5'-TTG CCA AGC TCC TGA AGC A -3' (SEQ ID NO: 65) and reverse primer: 5'- CGT TTG GCT GAA TAC CTT CCC-3' (SEQ ID NO: 66), for Nanog were forward primer: 5'-CCC AAA GCT TGC CTT GCT TT -3' (SEQ ID NO: 67) and reverse primer: 5'-AGA CAG ICT CCG TGT GAG GCA T-3' (SEQ ID NO: 68) . The correct size of PCR products was confirmed by separation on agarose gel.

*Real time RT-PCR (RQ-PCR).* For analysis of Oct4, Nanog, Nkx2.5/Csx, VE-cadherin, and GFAP mRNA levels, total mRNA was isolated from cells with the RNEASY® Mini Kit (Qiagen Inc., Valencia, California, United States of America). mRNA was reverse-transcribed with TAQMAN® Reverse Transcription Reagents (Applied Biosystems, Foster City, California, United States of America). Detection of Oct4, Nanog, Nkx2.5/Csx, VE-cadherin, GFAP, and *β*2-microglobulin mRNA levels was performed by real-time RT-PCR using an ABI PRISM® 7000 Sequence Detection System (Applied Biosystems, Foster City, California, United States of America). A 25 µl reaction mixture contains 12.5 µl SYBR Green PCR Master Mix, 10 ng of cDNA template, 5'-GAT GTG GTC CGA GTG TGG TTC T -3' (SEQ ID NO: 69) forward and 5'-TGT GCA TAG TCG CTG CTT GAT -3' (SEQ ID NO: 70) reverse primers for Oct4; 5'- GCA GAA GGC CTC AGC ACC TA -3' (SEQ ID NO: 71) forward and 5'- AGG TTC CCA GTC GGG TTC A -3' (SEQ ID NO: 72) reverse primers for Nanog; 5'- CCC CTG GAT TTT GCA TTC AC -3' (SEQ ID NO: 73) forward and 5'- CGT GCG CAA GAA CAA ACG -3' (SEQ ID NO: 74) reverse primers for Nkx2.5/Csx; 5'- CCG ACA GTT GTA GGC CCT GTT -3' (SEQ ID NO: 75) forward and 5'- GGC ATC TTC GGG TTG ATC CT-3' (SEQ ID NO: 76) reverse primers for VE-cadherin; 5'- GTG GGC AGG TGG GAG CTT GAT TCT -3' (SEQ ID NO: 77) forward and 5'- CTG GGG CGG CCT GGT ATG ACA -3' (SEQ ID NO: 78) reverse primers for GFAP; 5'- AAT GCG GCA TCT TCA AAC CT -3' (SEQ ID NO: 79) forward and 5'- TGA CTT TGT CAC AGC CCA AGA TA -3' (SEQ ID NO: 80) reverse primers for 2 microglobulin. Primers were designed with PRIMER EXPRESS® software (Applied Biosystems, Foster City, California, United States of America).

The threshold cycle (Ct; *i.e*., the cycle number at which the amount of amplified gene of interest reached a fixed threshold) was determined subsequently. Relative quantitation of Oct4 and Nanog mRNA expression was calculated with the comparative Ct method. The relative quantization value of target, normalized to an endogenous control *β*2-microglobulin gene and relative to a calibrator, is expressed as 2^{-ΔΔCt} (fold difference), where ΔCt = Ct of target genes (Oct4, Nanog, Nkx2.5/Csx, VE-cadherin, GFAP) - Ct of endogenous control gene (*β*2-microglobulin), and ΔΔCt = ΔCt of samples for target gene-ΔCt of calibrator for the target gene.

To avoid the possibility of amplifying contaminating DNA, all the primers for real time RT-PCR were designed with an intron sequence inside the cDNA to be amplified, reactions were performed with appropriate negative controls (template-free controls), a uniform amplification of the products was rechecked by analyzing the melting curves of the amplified products (dissociation graphs), the melting temperature (Tₘ) was 57-60°C, the product Tₘ was at least 10°C higher than primer Tₘ, and gel electrophoresis was performed to confirm the correct size of the amplification product and the absence of unspecific bands.

*Fluorescent staining of CB-derived VSEL stem cells.* The expression of each antigen was examined in cells from four independent experiments. CXCR4⁺/lin⁻/CD45⁻ cells were fixed in 3.5% paraformaldehyde for 20 minutes, permeabilized by 0.1% Triton X100, washed in PBS, pre-blocked with 2% BSA, and subsequently stained with antibodies to SSEA-4 (clone MC-813-70; 1:100; mouse monoclonal IgG, Chemicon Intl., Temecula, California, United States of America), Oct-4 (clone 9E3; 1:100; mouse monoclonal IgG, Chemicon Intl., Temecula, California, United States of America), and Nanog (1:200; goat polyclonal IgG, Santa Cruz Biotechnology, Inc., Santa Cruz, California, United States of America). Appropriate secondary Alexa Fluor 488 goat anti-mouse IgG, Alexa Fluor 594 goat anti-mouse IgG, and Alexa Fluor 594 rabbit anti-goat were used (1:400; Molecular Probes, Eugene, Oregon, United States of America).

In control experiments, cells were stained with secondary antibodies only. The nuclei were labeled with DAPI (Molecular Probes, Eugene, Oregon, United States of America). The fluorescence images were collected with the TE-FM Epi-Fluorescence system attached to a Nikon Inverted Microscope Eclipse TE300 and captured by a cool snap HQ digital B/W CCD (Roper Scientific, Tucson, Arizona, United States of America) camera.

*Statistical Analysis.* Arithmetic means and standard deviations of FACS data were calculated on a Macintosh computer PowerBase 180, using Instat 1.14 (GraphPad, San Diego, California, United States of America) software. Data were analyzed using the Student t-test for unpaired samples or ANOVA for multiple comparisons. Statistical significance was defined as p < 0.05.

VSEL stem cells were also isolated from human cord blood using the general FACS procedure outlined in EXAMPLE 2. For human cells, antibodies directed against CXCR4 (labeled with allophycocyanin (APC)), CD45 (labeled with phycoerythrin (PE)), CD19, CD16, CD2, CD14, CD3, CD24, CD56, CD66b, and CD235a were employed. Antibodies against the lineage markers were labeled with fluorescein isothiocyanate (FITC). The isolated VSEL stem cells were CXCR4⁺/lin⁻/CD45⁻ under TEM looked like murine VSEL stem cells (*i*.*e*., were about 3-4 µm in diameter, posses large nuclei surrounded by a narrow rim of cytoplasm, and contain open-type chromatin (euchromatin)), and were enriched in markers of pluripotent stem cells by real time RT-PCR (*see* EXAMPLE 7).

### Discussion of EXAMPLE 29

### A population of CD34⁺CD133⁺CXCR4⁺lin⁻/CD45⁻ cells is present in CB

Multiparameter analysis (outlined in Figure 32) was performed to determine if human CB mononuclear cells (CB MNC) contained a population of cells that resemble VSEL stem cells. In order to separate MNC from CB, Ficoll-Paque centrifugation was not employed, and erythrocytes were removed by hypotonic lysis. Additionally, it was hypothesized that CB-VSEL stem cells, like their counterparts in adult murine BM, would be small and lin⁻/CD45⁻.

Thus, a population of small (< 5µm) lin⁻/CD45⁻ CB MNC was investigated. That these cells might express CXCR4 as do their murine BM-derived counterparts was also investigated. In addition, the cells were tested for expression of other human stem cell antigens such as CD133 and CD34.

Figure 27A shows that human CB contained a population of lin⁻/CD45⁻ MNC that express CXCR4 (0.037 ± 0.02%, n = 9), CD34 (0.118 ± 0.028%, n = 5), and CD133 (0.018 ± 0.008%, n = 5). These CXCR4⁺/CD133⁺/CD34⁺/lin⁻/CD45⁻ cells were sorted by FACS in a manner similar to VSEL stem cells, did not grow hematopoietic colonies *in vitro,* and also similar to murine VSEL stem cells are very small (about 3-5 µm; Figure 27B, upper panel). In contrast, CB-derived lin⁻/CD45⁻ hematopoietic cells are larger (> 6 µm; Figure 27B, lower panel). Furthermore, a significant overlap in co-expression of CXCR4, CD34, and CD133 antigens was observed among CB-derived small lin⁻/CD45⁻ cells, and it was determined that 0.015 ± 0.005% of lin⁻/CD45⁻ cells were CXCR4⁺/CD133⁺/CD34⁺.

CB-derived CXCR4⁺/CD133⁺/CD34⁺/lin⁻/CD45⁻ cells sorted by FACS, as well as CXCR4⁺/lin⁻/CD45⁻ CD34⁺/lin⁻/CD45⁻ and CD133⁺/lin⁻/CD45⁻ cells are highly enriched for mRNA for transcriptions factors expressed by pluripotent embryonic cells such as Oct-4 and Nanog (Figures 28A and 28B). Expression of these markers was subsequently confirmed by regular RT-PCR (Figure 28C). Furthermore, these cells, as is disclosed herein for BM-derived VSEL stem cells, are also enriched in mRNA for several developmental genes for different organs/tissues such as Nkx2.5/Csx, VE-cadherin, and GFAP, which are markers for cardiac-, endothelial- and neural tissue committed stem cells (TCSC), respectively.

*CB-derived CXCR4⁺lin⁻*/*CD45⁻ cells express SSEA-4, Oct-4, and Nanog at the protein level.* Murine BM-derived VSEL stem cells express SSEA-1, Oct-4, and Nanog at the protein level. Thus, immunofluorescence staining was performed to evaluate if CB-VSEL stem cells also expressed similar embryonic stem cell markers. Figure 29 shows an example of staining showing that highly purified CB-derived CXCR4⁺/lin⁻/CD45⁻ cells expressed SSEA-4 on their surface and Oct-4 and Nanog transcription factors in nuclei.

*Transmission electron-microscopy analysis of CB-derived CXCR4⁺*//*in*⁻/*CD45⁻*/ *cells.* CXCR4⁺/CD34⁺/CD133⁺/lin⁻/CD45⁻ cells were analyzed by transmission electron microscopy (TEM). Figure 30 shows that CB-VSEL stem cells were very small ∼3-5 µm and contained relatively large nuclei and a narrow rim of cytoplasm with numerous mitochondria. DNA in the nuclei of these cells contained open-type euchromatin that is characteristic for pluripotent embryonic stem cells. Thus, the presently disclosed subject matter provides for the first time morphological evidence for the presence of a distinct population of very small embryonic-like (VSEL) stem cells in adult CB.

### EXAMPLE 30

### VSEL-DS Can Differentiate into Hematopoietic Cells

It was observed that cells isolated from VSEL-DS derived from GFP⁺ mice formed small secondary spheres if plated in methylcellulose cultures supplemented with IL-3 + GM-CSF (Figures 31A and 31B). The single cell suspension prepared from these secondary spheres recovered by methylcellulose solubilization from the primary methylcellulose cultures, if plated again in methylcellulose cultures (middle panel) or plasma clot (right panel) and stimulated by IL-3 and GM-CSF formed hematopoietic colonies. Evidence that these were hematopoietic colonies was obtained by FACS analysis of CD45 expression of cells derived from solubilized colonies growing in methylcellulose or by immunofluorescence staining cells from colonies growing in plasma clot cultures for CD45 (Figure 31C).

In parallel, cells isolated from the colonies growing in methylcellulose were analyzed for expression of hematopoietic genes by employing real time RT-PCR. Upregulation of mRNA for hematopoietic transcription factors such as c-myb, PU-1, and SCL was observed by normal RT-PCR and by RQ-PCR during expansion in the presence of IL-3 + GM-CSF. Thus, VSEL stem cells might be a source of the most primitive HSC in BM.

### EXAMPLE 31

### Ex Vivo Differentiation of VSEL-DS into Hematopoietic Cells

VSEL-DS are trypsynized and plated in methylcellulose-based medium (StemCell Technologies Inc., Vancouver, British Columbia, Canada). At day 5 of culture in methylcellulose medium, cells proliferate and form small spheres. These spheres are recovered from methylcellulose cultures by aspiration, are washed and trypsinized to obtain a single cell suspension, and re-plated in methylcellulose-based medium that contains a selected combination of cytokines and growth factors for hematopoietic colony formation.

For CFU-GM colony growth, murine interleukin-3 (mIL-3) + murine granulocyte-macrophage colony stimulating factor m(GM-CSF) is added. At the same time, an. aliquot of these cells is plated in plasma clot cultures. The reason for this is that these cultures are suitable for analysis by immunofluorescence and immunohistochemical staining. At day 4-6 hematopoietic colonies are formed both in methylcellulose and plasma clot conditions. Cells from the colonies growing in methylcellulose are recovered for mRNA isolation or FACS analysis.

### REFERENCES

The references listed below as well as all references cited in the specification, including patents, patent applications, journal articles, and all database entries (*e*.*g*., GENBANK® Accession Nos., including any annotations presented in the GENBANK® database that are associated with the disclosed sequences), are
Amit et al. (2000) 227 Dev Biol 271-278.
Bradley et al. (1984) 309 Nature 255-258.
Caplan et al. (2001) 7 Trends Mol Med 259-64.
Castro et al. (2002) 297 Science 1299.
Ceradini et al. (2004) 10 Nat Med 858-864.
Corti et al. (2002) 277 Exp Cell Res 74-85.
Doetschman et al. (1985) 87 J Embryol Exp Morphol 27-45.
Fraichard et al. (1995) 108 J Cell Sci 3181-3188.
Geiger et al. 100 Blood 721-723.
GENBANK® Accession Nos. AAB25223; AAR16420; AF091351; AF240635 ; AY278951; BC031665; DQ486513; M28382; M28698; NM_002055; NM_004048; NM_004387; NM_007423; NM_007562; NM_008476; NM_008591; NM_008656; NM_008699; NM_008814; NM_009735; NM_010024; NM_010612; NM_010866; NM_011661; NM_011708; NM_013584; NM_013685; NM_016701; NM_016967; NM_016968; NM_023279; NM_024865; NM_025282; NM_027011; NM_031202; NM_139218; NM_144955; NM_175238; NP_001009318; NP_002829; NP_034868; NP_035340; NP_598415; NP_612516; NP_776434; NP_999251; U85046; X02801; X15784; X52437; X83930; XP_002829; XP_223083; XP_599431.
Goodell et al. (1996) 183 J Exp Med 1797-1806.
Goodell et al. (2005) Methods Mol Biol 343-352.
Guo et al. (1998) 275 Am J Physiol H1375-1387.
Guo et al. (1999) 96 Proc Natl Acad Sci. U S A 11507-11512.
Guo et al. (2005) 23 Stem Cells 1324-1332.
Hao et al. (2003) 12 J Hematother Stem Cell Res 23-32.
Haynesworth et al. (1992) 13 Bone 81-88.
Holden & Vogel (2002) 296 Science 2126-2129.
Ianus et al. (2003) 111 J Clin Invest 843-850.
Jackson et al. (2001) 107 J Clin Invest 1395-1402.
Jaenisch (1988) 240 Science 1468-1474
Kawada & Ogawa (2001) 98 Blood 2008-2013.
Kogler et al. (2004) 200 J Exp Med 123-135.
Korbling et al. (2002) 346 N Engl J Med 738-746.
Kucia et al. (2004a) 32 Blood Cells Mol Dis 52-57.
Kucia et al. (2004b) Circ Res 1191-1199.
Kucia et al. (2005a) 33 Exp Hematol 613-623.
Kucia et al. (2005b) 19 Leukemia 1118-1127.
Kucia et al. (2005c) 23 Stem Cells 879-894.
Labarge & Blau (2002) 111 Cell 589-601.
Lee & Stoffel (2003) 111 J Clin Invest 799-801
Lemischka (2002) 30 Exp Hematol 848-852.
Mackay et al. (1998) 4 Tissue Eng 415-28.
Macpherson et al. 118 J Cell Sci 2441-2450.
Majka et al. (2001) 97 Blood 3075-3085.
Makino et al. (1999) 103 J Clin Invest 697-705.
Martin & Evans (1975) in Teratomas and Differentiation (M. I. Sherman & D. Solter, Eds.), pp. 169-187, Academic Press, New York, New York, United States of America.
McKinney-Freeman et al. (2002) 99 Proc Natl Acad Sci U S A 1341-1346.
Nagy et al. (2003) Manipulating the Mouse Embryo. A Laboratory Manual. Third Edition, Cold Spring Harbor Laboratory Press, Cold Spring Harbor, New York, United States of America.
Orlic et al. (2003) 7 Pediatr Transplant 86-88.
Pennacchietti et al. (2003) 3 Cancer Cell 347-361.
Petersen et al. (1999) 284 Science 1168-1170.
Pittenger et al. (2000) 251 Curr Top Microbiol Immunol 3-11.
Ratajczak et al. (2004a) 103 Blood 2071-2078.
Ratajczak et al. (2004b) 18 Leukemia 29-40.
Reyes & Verfaillie (2001) 938 Ann NY Acad Sci 231-235.
Reyes et al. (2001) 98 Blood 2615-2625.
Robertson (1991) 44 Biol Reprod 238-45.
Robertson et al. (1986) 323 Nature 445-447.
Sanchez-Ramos (2002) 69 Neurosci Res 880-893.
Schuldiner et al. (2000) 97 Proc Natl Acad Sci U S A 11307-11312.
Schwartz et al. (2000) 109 J Clin Invest 1291-1302.
Shamblott et al. (1998) 95 Proc Natl Acad Sci U S A 13726-13731.
Stamm et al. (2003) 361 Lancet 45-46.
Tamamura et al. (1998) 253 Biochem Biophys Res Comm 877-882.
Thomson et al. (1995) 92 Proc Natl Acad Sci U S A 7844-7848.
Thomson et al. (1998) 282 Science 1145-1147
U.S. Patent Nos. 5,650,550; 5,736,396; 5,750,397; 5,777,195; 5,843,780; and 6,090,622.
Wagers et al. (2002) 297 Science 2256-2259.
Yamada et al. (2002) 20 Stem Cells 146-154.
Yoo et al. (1998) 80 J Bone Joint Surg Am 1745-57.
Young et al. (1998) 16 J Orthop Res 406-13.

It will be understood that various details of the described subject matter can be changed without departing from the scope of the described subject matter. Furthermore, the foregoing description is for the purpose of illustration only, and not for the purpose of limitation.

## Claims

1. A composition comprising isolated CD45⁻ stem cells comprising VSEL stem cells for use in treating a myocardial infarction in a subject, wherein the VSEL stem cells comprise Sca-1⁺/lin⁻/CD45⁻ and express at least one of SSEA-1, Oct-4, Rex-1, and Nanog, or comprise CD34⁺/lin⁻/CD45⁻ and express at least one of SSEA-4, Oct-4, Rex-1, and Nanog.

2. The composition for use according to claim 1, wherein the VSEL stem cells comprise CXCR4⁺ and/or CD133⁺.

3. The composition for use according to claims 1 or 2, wherein the subject is a mammal.

4. The composition for use according to claim 3, wherein the mammal is a mouse or a human.

5. The composition for use according to any one of claims 1 to 4, wherein the isolated CD45⁻ stem cells comprising VSEL stem cells were isolated from a source selected from the group consisting of bone marrow, peripheral blood, spleen, cord blood, and combinations thereof.

6. The composition for use according to any one of claims 1 to 5, wherein the isolated CD45⁻ stem cells are differentiated to produce a pre-determined cell type prior to administering the composition to the subject.

7. The composition for use according to claim 6, wherein the pre-determined cell type is a cardiomyocyte.

8. The composition for use according to claim 6, wherein the pre-determined cell type expresses one or more markers selected from the group consisting of Nsx2.5/Csx and GATA-4.

9. The composition for use according to any one of claims 1 to 5, wherein the VSEL stem cells express one or more markers for a cell type of interest, wherein the markers are selected from the group consisting of GFAP, Nestin, β III tubulin, Olig1, Olig2, Myf5, MyoD, myogenin, Nsx2.5/Csx, GATA-4, MEF2C, α-fetoprotein, CK19, Nkx 2-3, Tcf4, Nkx 6.1, Pdx 1, VEGFR2, VE-cadherin, von Willebrand factor, TIE2, Krt 2-5, Krt 2-6a, BNC, DCT, TYR, and TRP.

10. The composition for use according to any one of claims 1 to 5, wherein the VSEL stem cells comprise a stem cell for a cell type of interest selected from a skeletal muscle cell, an intestinal epithelium cell, a pancreas cell, an endothelial cell, an epidermis cell, a melanocyte, a neuronal cell, a myocardial cell, a chondrocyte, an adipocyte, a liver cell, a pancreas cell, an endothelial cell, an epithelial cell, a retinal pigment cell, and an endodermal cell.

## Patentansprüche

1. Zusammensetzung, die isolierte CD45⁻-Stammzellen umfasst, die VSEL-Stammzellen zur Verwendung bei der Behandlung eines Herzinfarkts in einem Subjekt umfassen, wobei die VSEL-Stammzellen Sca-1⁺/lin⁻/CD45⁻ umfassen und mindestens eines von SSEA-1, Oct-4, Rex-1 und Nanog exprimieren, oder CD34⁺/lin⁻/CD45⁻ umfassen und mindestens eines von SSEA -4, Oct-4, Rex-1 und Nanog exprimieren.

2. Zusammensetzung zur Verwendung nach Anspruch 1, wobei die VSEL-Stammzellen CXCR4⁺ und/oder CD133⁺ umfassen.

3. Zusammensetzung zur Verwendung nach den Ansprüchen 1 oder 2, wobei das Subjekt ein Säuger ist.

4. Zusammensetzung zur Verwendung nach Anspruch 3, wobei der Säuger eine Maus oder ein Mensch ist.

5. Zusammensetzung zur Verwendung nach einem der Ansprüche 1 bis 4, wobei die isolierten CD45⁻-Stammzellen, die VSEL-Stammzellen umfassen, aus einer Quelle isoliert wurden, die aus der Gruppe ausgewählt ist, die aus Knochenmark, peripherem Blut, Milz, Nabelschnurblut und Kombinationen davon besteht.

6. Zusammensetzung zur Verwendung nach einem der Ansprüche 1 bis 5, wobei die isolierten CD45⁻-Stammzellen differenziert werden, um vor der Verabreichung der Zusammensetzung an das Subjekt einen vorbestimmten Zelltyp zu erzeugen.

7. Zusammensetzung zur Verwendung nach Anspruch 6, wobei der vorbestimmte Zelltyp ein Kardiomyozyt ist.

8. Zusammensetzung zur Verwendung nach Anspruch 6, wobei der vorbestimmte Zelltyp einen oder mehrere Marker exprimiert, die aus der Gruppe ausgewählt sind, die aus Nsx2.5/Csx und GATA-4 besteht.

9. Zusammensetzung zur Verwendung nach einem der Ansprüche 1 bis 5, wobei die VSEL-Stammzellen einen oder mehrere Marker für einen Zelltyp von Interesse exprimieren, wobei die Marker aus der Gruppe ausgewählt sind, die aus GFAP, Nestin, β-III-Tubulin, Olig1, Olig2, Myf5, MyoD, Myogenin, Nsx2.5/Csx, GATA-4, MEF2C, α-Fetoprotein, CK19,
Nkx 2-3, Tcf4, Nkx6.1, Pdx 1, VEGFR2, VE-Cadherin, von-Willebrand-Faktor, TIE2, Krt 2-5, Krt 2-6a, BNC, DCT, TYR und TRP besteht.

10. Zusammensetzung zur Verwendung nach einem der Ansprüche 1 bis 5, wobei die VSEL-Stammzellen eine Stammzelle für einen Zelltyp von Interesse umfassen, der aus einer Skelettmuskelzelle, einer Darmepithelzelle, einer Pankreaszelle, einer Endothelzelle, einer Epidermiszelle, einem Melanozyten, einer neuronalen Zelle, einer Myokardzelle, einem Chondrozyten, einem Adipozyten, einer Leberzelle, einer Pankreaszelle, einer Endothelzelle, einer Epithelzelle, einer Netzhautpigmentzelle und einer endodermalen Zelle ausgewählt ist.

## Revendications

1. Composition comprenant des cellules souches isolées CD45⁻ comprenant des cellules souches VSEL pour une utilisation dans le traitement d'un infarctus du myocarde chez un sujet, dans laquelle les cellules souches VSEL comprennent Sca-1⁺/lin⁻/CD45⁻ et expriment au moins l'un de SSEA-1, Oct-4, Rex-1, et Nanog, ou comprennent CD34⁺/lin⁻/CD45⁻ et expriment au moins l'un de SSEA-4, Oct-4, Rex-1, et Nanog.

2. Composition pour une utilisation selon la revendication 1, dans laquelle les cellules souches VSEL comprennent CXCR4+ et/ou CD133+.

3. Composition pour une utilisation selon les revendications 1 ou 2, dans laquelle le sujet est un mammifère.

4. Composition pour une utilisation selon la revendication 3, dans laquelle le mammifère est une souris ou un être humain.

5. Composition pour une utilisation selon l'une quelconque des revendications 1 à 4, dans laquelle les cellules souches isolées CD45⁻ comprenant des cellules souches VSEL ont été isolées à partir d'une source choisie parmi le groupe constitué par la moelle osseuse, le sang périphérique, la rate, le sang de cordon, et des combinaisons de ceux-ci.

6. Composition pour une utilisation selon l'une quelconque des revendications 1 à 5, dans laquelle les cellules souches isolées CD45⁻ sont différenciées pour produire un type cellulaire prédéterminé avant l'administration de la composition au sujet.

7. Composition pour une utilisation selon la revendication 6, dans laquelle le type cellulaire prédéterminé est un cardiomyocyte.

8. Composition pour une utilisation selon la revendication 6, dans laquelle le type cellulaire prédéterminé exprime un ou plusieurs marqueurs choisis parmi le groupe constitué par Nsx2.5/Csx et GATA-4.

9. Composition pour une utilisation selon l'une quelconque des revendications 1 à 5, dans laquelle les cellules souches VSEL expriment un ou plusieurs marqueurs pour un type cellulaire d'intérêt, dans laquelle les marqueurs sont choisis parmi le groupe constitué par GFAP, nestine, β III-tubuline, Olig1, Olig2, Myf5, MyoD, myogénine, Nsx2.5/Csx, GATA-4, MEF2C, α-foetoprotéine, CK19, Nkx 2-3, Tcf4, Nkx 6.1, Pdx 1, VEGFR2, VE-cadhérine, facteur de von Willebrand, TIE2, Krt 2-5, Krt 2-6a, BNC, DCT, TYR, et TRP.

10. Composition pour une utilisation selon l'une quelconque des revendications 1 à 5, dans laquelle les cellules souches VSEL comprennent une cellule souche pour un type cellulaire d'intérêt choisi parmi une cellule de muscle squelettique, une cellule épithéliale intestinale, une cellule pancréatique, une cellule endothéliale, une cellule épidermique, un mélanocyte, une cellule neuronale, une cellule myocardique, un chondrocyte, un adipocyte, une cellule hépatique, une cellule pancréatique, une cellule endothéliale, une cellule épithéliale, une cellule de pigment rétinien, et une cellule endodermique.


### SEQUENCE LISTING

<110> UNIVERSITY OF LOUISEVILLE RESEARCH FOUNDATION, INC.
<120> VERY SMALL EMBRYONIC-LIKE (VSEL) STEM CELLS AND METHODS OF ISOLATING AND USING THE SAME
<130> ULR13781PCTEPD1
<150 EP 06 827 358.0
   <151> 2006-11-02
<150> US 60/748,685
   <151> 2005-12-08
<160> 80
<170> PatentIn version 3.3
<210> 1
   <211> 22
   <212> DNA
   <213> Mus musculus
<400> 1
   catacgcctg cagagttaag ca 22
<210> 2
   <211> 24
   <212> DNA
   <213> Mus musculus
<400> 2
   gatcacatgt ctcgatccca gtag 24
<210> 3
   <211> 23
   <212> DNA
   <213> Mus musculus
<400> 3
   accttcagga gatatgcaaa tcg 23
<210> 4
   <211> 25
   <212> DNA
   <213> Mus musculus
<400> 4
   ttctcaatgc tagttcgctt tctct 25
<210> 5
   <211> 21
   <212> DNA
   <213> Mus musculus
<400> 5
   cgttcccaga attcgatgct t 21
<210> 6
   <211> 22
   <212> DNA
   <213> Mus musculus
<400> 6
   ttttcagaaa tcccttccct cg 22
<210> 7
   <211> 21
   <212> DNA
   <213> Mus musculus
<400> 7
   agatggcttc cctgacggat a 21
<210> 8
   <211> 22
   <212> DNA
   <213> Mus musculus
<400> 8
   cctccaagct ttcgaaggat tt 22
<210> 9
   <211> 20
   <212> DNA
   <213> Mus musculus
<400> 9
   gcagtctacg gaaccgcatt 20
<210> 10
   <211> 21
   <212> DNA
   <213> Mus musculus
<400> 10
   ttgaacttcc ctccggattt t 21
<210> 11
   <211> 25
   <212> DNA
   <213> Mus musculus
<400> 11
   gagctggatt cttttggatc agtaa 25
<210> 12
   <211> 20
   <212> DNA
   <213> Mus musculus
<400> 12
   gccaaaggtg accagacaca 20
<210> 13
   <211> 20
   <212> DNA
   <213> Mus musculus
<400> 13
   ggagctcaat gaccgctttg 20
<210> 14
   <211> 20
   <212> DNA
   <213> Mus musculus
<400> 14
   tccaggaagc gaaccttctc 20
<210> 15
   <211> 21
   <212> DNA
   <213> Mus musculus
<400> 15
   ccctgatgat ccatcctcct t 21
<210> 16
   <211> 30
   <212> DNA
   <213> Mus musculus
<400> 16
   ctggaatatg ctagaaactc tagactcact 30
<210> 17
   <211> 19
   <212> DNA
   <213> Mus musculus
<400> 17
   tccgttcgct caggtcctt 19
<210> 18
   <211> 20
   <212> DNA
   <213> Mus musculus
<400> 18
   cccagactga ccgaaaacga 20
<210> 19
   <211> 20
   <212> DNA
   <213> Mus musculus
<400> 19
   acgtcgtagc gcaggcttat 20
<210> 20
   <211> 19
   <212> DNA
   <213> Mus musculus
<400> 20
   cgcccaactc cgcttactt 19
<210> 21
   <211> 18
   <212> DNA
   <213> Mus musculus
<400> 21
   gggaggcgcc attgtaca 18
<210> 22
   <211> 19
   <212> DNA
   <213> Mus musculus
<400> 22
   gtgcaggcag gaagttcca 19
<210> 23
   <211> 20
   <212> DNA
   <213> Mus musculus
<400> 23
   ctaggagggc gtccttcatg 20
<210> 24
   <211> 21
   <212> DNA
   <213> Mus musculus
<400> 24
   cacgtattct gcccagcttt t 21
<210> 25
   <211> 18
   <212> DNA
   <213> Mus musculus
<400> 25
   ggacagccgg tgtgcatt 18
<210> 26
   <211> 19
   <212> DNA
   <213> Mus musculus
<400> 26
   cactccggaa ccccaacag 19
<210> 27
   <211> 19
   <212> DNA
   <213> Mus musculus
<400> 27
   ggagaagcgc aggctcaag 19
<210> 28
   <211> 20
   <212> DNA
   <213> Mus musculus
<400> 28
   ttgagcaggg tgctcctctt 20
<210> 29
   <211> 18
   <212> DNA
   <213> Mus musculus
<400> 29
   cggatgtggc tcgtttgc 18
<210> 30
   <211> 18
   <212> DNA
   <213> Mus musculus
<400> 30
   ttgggaccct cccgagat 18
<210> 31
   <211> 22
   <212> DNA
   <213> Mus musculus
<400> 31
   tccagtgctg tctgctctaa gc 22
<210> 32
   <211> 19
   <212> DNA
   <213> Mus musculus
<400> 32
   tggcctgcga tgtctgagt 19
<210> 33
   <211> 18
   <212> DNA
   <213> Mus musculus
<400> 33
   acccgcttcc ctcatcct 18
<210> 34
   <211> 22
   <212> DNA
   <213> Mus musculus
<400> 34
   aaactcattt cgtgcaatgc tt 22
<210> 35
   <211> 21
   <212> DNA
   <213> Mus musculus
<400> 35
   catgcgaagc caatatgagg t 21
<210> 36
   <211> 20
   <212> DNA
   <213> Mus musculus
<400> 36
   tcagcatcct tccggttctg 20
<210> 37
   <211> 22
   <212> DNA
   <213> Mus musculus
<400> 37
   ggagccaaaa aagctgtcag tt 22
<210> 38
   <211> 19
   <212> DNA
   <213> Mus musculus
<400> 38
   cgtcctcgct cgtcctaca 19
<210> 39
   <211> 21
   <212> DNA
   <213> Mus musculus
<400> 39
   acccttgcac tcactgcaaa g 21
<210> 40
   <211> 21
   <212> DNA
   <213> Mus musculus
<400> 40
   ggagaacatg aatcgcatcg t 21
<210> 41
   <211> 19
   <212> DNA
   <213> Mus musculus
<400> 41
   gcctgtaccc cccatcaag 19
<210> 42
   <211> 21
   <212> DNA
   <213> Mus musculus
<400> 42
   acgtgggtct ggtgtgtttt c 21
<210> 43
   <211> 20
   <212> DNA
   <213> Mus musculus
<400> 43
   cggctgagca agctaaggtt 20
<210> 44
   <211> 22
   <212> DNA
   <213> Mus musculus
<400> 44
   ggaagaagcg ctctctttga aa 22
<210> 45
   <211> 20
   <212> DNA
   <213> Mus musculus
<400> 45
   ttcaagctgc cagaaaacca 20
<210> 46
   <211> 21
   <212> DNA
   <213> Mus musculus
<400> 46
   gagccttgtc agggtctttg g 21
<210> 47
   <211> 20
   <212> DNA
   <213> Mus musculus
<400> 47
   ccctctgaac ctgcaaatcg 20
<210> 48
   <211> 22
   <212> DNA
   <213> Mus musculus
<400> 48
   tgatctgctc cctctcctca gt 22
<210> 49
   <211> 23
   <212> DNA
   <213> Mus musculus
<400> 49
   aggaaccatg tctaccaaaa cca 23
<210> 50
   <211> 19
   <212> DNA
   <213> Mus musculus
<400> 50
   ctggctgagc tggcactgt 19
<210> 51
   <211> 21
   <212> DNA
   <213> Mus musculus
<400> 51
   catgcacccc tttgagaacc t 21
<210> 52
   <211> 22
   <212> DNA
   <213> Mus musculus
<400> 52
   atgtactgtt caggcagcga cc 22
<210> 53
   <211> 20
   <212> DNA
   <213> Mus musculus
<400> 53
   cagtttcccc gagcttgcat 20
<210> 54
   <211> 17
   <212> DNA
   <213> Mus musculus
<400> 54
   agaggcgggc agcattc 17
<210> 55
   <211> 20
   <212> DNA
   <213> Mus musculus
<400> 55
   cgagcctgtg cctcctctaa 20
<210> 56
   <211> 20
   <212> DNA
   <213> Mus musculus
<400> 56
   gactcccatc acccatccat 20
<210> 57
   <211> 24
   <212> DNA
   <213> Mus musculus
<400> 57
   cctagctcag ttctctggac atga 24
<210> 58
   <211> 24
   <212> DNA
   <213> Mus musculus
<400> 58
   gcaggcctct aagatacgag aatt 24
<210> 59
   <211> 20
   <212> DNA
   <213> Mus musculus
<400> 59
   gacggacaag taccggctgc 20
<210> 60
   <211> 20
   <212> DNA
   <213> Mus musculus
<400> 60
   gacagcttag agatgatgat 20
<210> 61
   <211> 19
   <212> DNA
   <213> Mus musculus
<400> 61
   cgcgtcgact tattcatgg 19
<210> 62
   <211> 20
   <212> DNA
   <213> Mus Musculus
<400> 62
   cacacattga ttgtggcacc 20
<210> 63
   <211> 23
   <212> DNA
   <213> Mus musculus
<400> 63
   gagcatcctt tgctatcgga agc 23
<210> 64
   <211> 23
   <212> DNA
   <213> Mus musculus
<400> 64
   cgttatttcc tcctcgatga tgg 23
<210> 65
   <211> 19
   <212> DNA
   <213> Homo sapiens
<400> 65
   ttgccaagct cctgaagca 19
<210> 66
   <211> 21
   <212> DNA
   <213> Homo sapiens
<400> 66
   cgtttggctg aataccttcc c 21
<210> 67
   <211> 20
   <212> DNA
   <213> Homo sapiens
<400> 67
   cccaaagctt gccttgcttt 20
<210> 68
   <211> 22
   <212> DNA
   <213> Homo sapiens
<400> 68
   agacagtctc cgtgtgaggc at 22
<210> 69
   <211> 22
   <212> DNA
   <213> Homo sapiens
<400> 69
   gatgtggtcc gagtgtggtt ct 22
<210> 70
   <211> 21
   <212> DNA
   <213> Homo sapiens
<400> 70
   tgtgcatagt cgctgcttga t 21
<210> 71
   <211> 20
   <212> DNA
   <213> Homo sapiens
<400> 71
   gcagaaggcc tcagcaccta 20
<210> 72
   <211> 19
   <212> DNA
   <213> Homo sapiens
<400> 72
   aggttcccag tcgggttca 19
<210> 73
   <211> 20
   <212> DNA
   <213> Homo sapiens
<400> 73
   cccctggatt ttgcattcac 20
<210> 74
   <211> 18
   <212> DNA
   <213> Homo sapiens
<400> 74
   cgtgcgcaag aacaaacg 18
<210> 75
   <211> 21
   <212> DNA
   <213> Homo sapiens
<400> 75
   ccgacagttg taggccctgt t 21
<210> 76
   <211> 20
   <212> DNA
   <213> Homo sapiens
<400> 76
   ggcatcttcg ggttgatcct 20
<210> 77
   <211> 24
   <212> DNA
   <213> Homo sapiens
<400> 77
   gtgggcaggt gggagcttga ttct 24
<210> 78
   <211> 21
   <212> DNA
   <213> Homo sapiens
<400> 78
   ctggggcggc ctggtatgac a 21
<210> 79
   <211> 20
   <212> DNA
   <213> Homo sapiens
<400> 79
   aatgcggcat cttcaaacct 20
<210> 80
   <211> 23
   <212> DNA
   <213> Homo sapiens
<400> 80
   tgactttgtc acagcccaag ata 23